# EUROPEAN PATENT SPECIFICATION

(11) **EP 4 419 206 B1**
(45) Date of publication and mention of the grant of the patent: **17.09.2025**
(21) Application number: 22808742.5
(22) Date of filing: 21.10.2022
(51) Int. Cl.: A61P 33/06, C07D 213/38, C07D 401/14, A61K 31/4427

(54) **COMPOUNDS FOR THE TREATMENT OF MALARIA**
VERBINDUNGEN ZUR BEHANDLUNG VON MALARIA
COMPOSÉS POUR LE TRAITEMENT DU PALUDISME

(30) Priority: 21.10.2021 EP 21382949
(43) Date of publication of application: 28.08.2024
(73) Proprietor: Fundacio Institut de Bioenginyeria de Catalunya, 08028 Barcelona (ES); Fundación Privada Instituto De Salud Global Barcelona, 08036 Barcelona (ES); Universitat de Barcelona, 08028 Barcelona (ES)
(72) Inventor: FERNÀNDEZ BUSQUETS, Xavier, 08028 Barcelona (ES); BIOSCA ROMANILLOS, Arnau, 08028 Barcelona (ES); BOUZÓN ARNÁIZ, Inés, 08028 Barcelona (ES); MUÑOZ-TORRERO LÓPEZ-IBARRA, Diego, 08028 Barclona (ES); MARTÍNEZ ARCE, Elsa, 08028 Barcelona (ES); REOLID COLL, Pau, 08028 Barcelona (ES)
(74) Representative: ZBM Patents - Zea, Barlocci & Markvardsen
(86) International application number: PCT/EP2022/079438
(87) International publication number: WO 2023/067170

(56) References cited:
- EP-A2- 2 053 094
- WO-A2-2009/109457
- WO-A2-2011/065980
- GB-A- 2 011 457
- KOVALSKA V B ET AL: "Studies on the Spectral-Luminescent Properties of the Novel Homodimer Styryl Dyes in Complexes with DNA", JOURNAL OF FLUORESCENCE, KLUWER ACADEMIC PUBLISHERS-PLENUM PUBLISHERS, NE, vol. 15, no. 3, 1 May 2005 (2005-05-01), pages 215 - 219, XP019281685, ISSN: 1573-4994

## Description

### FIELD OF THE INVENTION

The present invention relates to the fields of medicine and parasitology and particularly, to compounds and compositions to benefit human health, useful as medicaments for the treatment of diseases, particularly malaria.

### BACKGROUND ART

WO2011065980A2 describes dyes, compositions and methods useful for detection of protein aggregates. One of said dyes is compound YAT2150 which is included in the world-wide known commercial product Proteostat^{®}. This product has been commercialized for over a decade only for the purpose of detecting protein aggregation for qualitative and quantitative assays in the field of biomedicine. To the best of inventors' knowledge, no disclosure on any medical or pharmaceutical effect of YAT2150 or any other compound disclosed in WO2011065980A2 has been made. YAT2150 corresponds to the following formula:

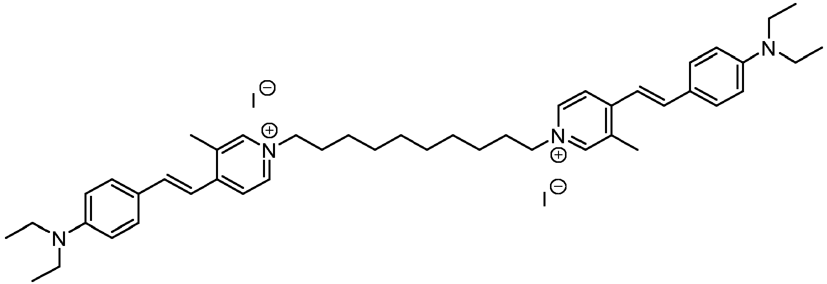

Malaria is a disease caused by protozoan parasites of the genus *Plasmodium,* which is transmitted by the bite of female *Anopheles* mosquitoes. Five species cause disease in humans (P. *falciparum, P. vivax, P. malariae, P. ovale and P. knowlesi*), where they live and multiply first in hepatocytes (namely, liver stage) and later in red blood cells (namely, blood stage). P. *falciparum* is the deadliest and most virulent species, especially when the infection occurs in young children and pregnant women. Each cycle of blood stage parasite multiplication lasts 48-72 hours and leads to the destruction of the host erythrocyte. It is manifested by mild symptoms such as fever and headache, but if left untreated, it may quickly develop into life-threatening complications such as cerebral malaria.

There are several drugs able to kill plasmodial parasites and are used to treat malaria. Artemisinin-based combination therapy (ACT) is currently the first-line treatment for falciparum malaria, along with a non-ACT combination of amodiaquine-sulfadoxine-pyrimethamine. However, the efficacy of these treatments is declining due to the rampant evolution of resistance by *Plasmodium.* In fact, previous first-line anti-malarial treatments such as chloroquine and sulfadoxine-pyrimethamine were already phased out due to drug resistance and consequently treatment failure. Therefore, it appears clear that the currently available arsenal of antimalarial drugs is insufficient, and the deployment of novel compounds is an urgent need to progress towards eradication of the disease. In those lines, the fact that there is only a small collection of antimalarial drugs belonging to a handful of chemical families significantly contributes to the parasite resistance to every drug deployed against it. For those molecules belonging to chemical classes where currently used antimalarials belong, resistance might be achieved with relative rapidity through the adaptation of already existing resistance mechanisms. However, resistance would be slowed down significantly for those molecules belonging to chemical families where no antimalarials have been described so far.

In these lines, an undesirable characteristic is resistance that emerges rapidly through single gene modification. As an example of this, resistance to the aminoquinolines chloroquine and piperaquine has been associated with distinct sets of point mutations in the gene encoding for P. *falciparum* chloroquine resistance transporter PfCRT. PfCRT is an efflux pump evolved by the parasite to expel chloroquine and which rendered this drug virtually useless as antimalarial in many areas of the world.

Further, desirable characteristics of yet to be discovered antimalarial compounds would include targeting several parasite stages (both in the human and in the mosquito), an easy and inexpensive synthesis, low *in vivo* toxicity and potential for oral administration formulations.

In view of the above, it is of great interest to find new therapies and compounds for the treatment of subjects suffering from malaria, which can avoid a rapid emerge of resistance towards them while targeting several stages of the pathogen and therefore ensure long-term therapy effectiveness.

### SUMMARY OF THE INVENTION

The problem to be solved by the present invention is to provide compounds useful for the treatment of diseases, particularly malaria, in a subject in need thereof.

Surprisingly, the inventors have found that a compound, herein referred as AID-X-2020, shows an outstanding antimalarial activity by inhibiting the growth of *Plasmodium falciparum* in critical stages of the parasite. These antimalarial effects of AID-X-2020 consequently lead to the arrest of the pathogen's life cycle at trophozoite stage and a significant reduction of parasite's growth in new invaded red blood cells (RBCs). Furthermore, the mechanism underlying such antimalarial activity has been found to be the inhibition of protein aggregates, which appears to be a highly desirable mechanism of antimalarial drugs because protein aggregation in malaria parasites is prominent in most stages of the pathogen in both the human and mosquito hosts. Additionally, impairing generalized protein aggregation targets many *Plasmodium* gene products, thus rapid evolution of resistance by the parasite to inhibitors of protein aggregation is unlikely to occur.

AID-X-2020 has the same organic moiety as YAT2150 previously described in WO2011065980A2. Nevertheless, the compound synthesized in the present invention differs from YAT2150 in that it includes two bromide anions, whereas YAT2150 comprises two iodide anions instead.

YAT2150 is the compound of the commercial product Proteostat^{®}, which has been commercialized only for the purpose of performing protein aggregation monitoring assays for over a decade. In those lines, YAT2150 has been used as a dye to detect protein aggregation, therefore the skilled person would not expect this compound to have any effect on protein aggregates nor on *Plasmodium*'s growth. Indeed, an antiaggregating effect of YAT2150 would not be expected considering that any compound used as a dye to detect protein aggregation should be neutral in terms of the activity measured by the dye. Therefore, from teachings of WO2011065980A2, the skilled person would not derive to the inhibition effect of YAT2150 on protein aggregates and the herein observed anti-malarial activity.

Working examples herein provide detailed experimental data demonstrating the antimalarial effect of AID-X-2020 through the inhibition of P. *falciparum* growth, which compromises the parasite's viability in early stages of the infection. Further, inventors have been able to describe the mechanism underlying such antimalarial activity to be the inhibition of protein aggregation. AID-X-2020 has also been proved herein to be safe for administration *in vivo* at therapeutic doses. Therefore, the compound described herein shows a clear potential to become an effective first-line treatment for malaria.

EXAMPLE 1 shows that AID-X-2020 has an outstanding antimalarial activity, which is comparable or even superior to that of other antimalarial treatments currently in use and known in the art. Other dyes for protein aggregation such as Thioflavin T (ThT) and Congo Red herein used as controls do not exhibit any antimalarial activity, therefore suggesting that this is not a common characteristic shared by all dyes detecting protein aggregation, but rather a specific and unexpected effect of AID-X-2020.

EXAMPLE 2 shows that AID-X-2020 is capable to arrest the life cycle of the parasite at trophozoite stage when added to ring stages. Thus, results evidence the potential of AID-X-2020 to exhibit its high antimalarial activity in the earliest stages of infection, which is critical for an antimalarial treatment to be highly effective. Moreover, in late *Plasmodium* forms there is a significant reduction of parasite's growth in new invaded RBCs. Furthermore, EXAMPLE 6 shows that AID-X-2020 is also capable of inhibiting the transition of the gametocyte to ookinete, unlike DONE3TCI, a protein antiaggregating compound which had previously shown to have high antimalarial activity.

EXAMPLES 3, 4 and 5 show that AID-X-2020 is a strong inhibitor of Aβ40 protein aggregation and consistently able to rapidly deregulate cell proteostasis. Hence, the potential mechanism underlying the antimalarial activity of AID-X-2020 relies on its protein antiaggregating capacity.

In EXAMPLE 8, eighteen compounds were synthesized, which are representative of compounds included in formula (I) other than AID-X-2020. Seventeen of these compounds (EMA357, EMA359, EMA366, EMA368, EMA377, PRC-5, PRC-8, PRC-14, PRC-15, PRC-20, PRC-25, PRC-28, PRC-29, PRC-31, PRC-39, PRC-42 and PRC-50) were tested and all show a potent antimalarial activity, comparable to the antimalarial activity of AID-X-2020, and even superior in four cases (Table 4). Therefore, evidence in EXAMPLE 8 confirms that structural analogs of AID-X-2020, featuring the core bis(aminostyrylpyridinium) scaffold, exhibit similar biological properties, particularly antimalarial activity.

EXAMPLE 9 shows that AID-X-2020 is active against chloroquine-, artemisinin- and multi-resistant *P. falciparum* strains, confirming that the compound belongs to a chemical family where no antimalarials have been described so far, thus being a potential new antimalarial drug with no potential resistance in comparison to the existing antimalarials.

Of note -in line with EXAMPLES 3, 4 and 5- EXAMPLE 10 shows that AID-X-2020 is capable of disassembling preformed Aβ40 fibrils, confirming not only its activity as an inhibitor of the aggregation of a model of amyloidogenic peptides, but also its disaggregating activity.

EXAMPLE 11 shows that in parasitized red blood cells (pRBCs), AID-X-2020 is mainly located in the cytosol in association with the endoplasmic reticulum regions. These findings are consistent with the role of AID-X-2020 as a protein aggregation inhibitor capable of disrupting parasite's aggresome.

EXAMPLE 12 shows that AID-X-2020 treatment causes a decrease in aggregated protein load in live parasites, which is consistent with the mechanism of action of this drug as described in previous examples.

EXAMPLE 13 shows the capacity of AID-X-2020 to block the development of gametocytes *in vitro* considerably more efficiently than DONE3TCI, in line with the results shown in EXAMPLE 6.

EXAMPLE 14 shows the presence of circulating ring forms of the parasite through the microscopic observation of AID-X-2020-stained clinical blood samples of a malaria-infected person.

Altogether, inventors have found different compounds to have a high antimalarial activity through the inhibition of protein aggregation in *Plasmodium,* which significantly reduce the parasite's viability *in vitro* and efficiently block the development of *Plasmodium* gametocytes in blood stages. These compounds belong to a novel structural class of antimalarials with mechanism of action that potentially targets many genes, therefore rapid resistance appearance to such compounds is unlikely to occur, making them good candidates to significantly contribute to malaria eradication.

Overall, the compounds of the present invention encompass the most desirable properties of future antimalarials in order to minimize the risk of resistance evolution: (i) a high therapeutic index to allow for the administration of high doses; (ii) classification into a chemical group where no antimalarials have been described so far to minimize the risk of adaptation of existing resistance mechanisms; and (iii) a target present in several stages of the pathogen and which is not a single-gene product to reduce the chances that resistance can emerge rapidly.

Accordingly, a first aspect of the invention relates to a compound of formula (I) 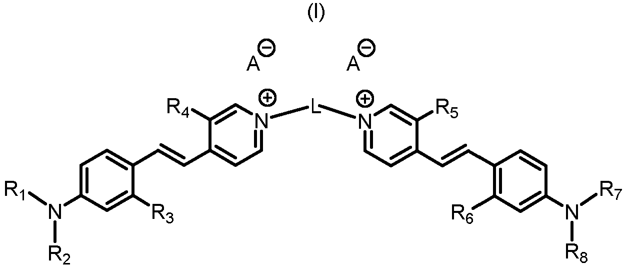 or a pharmaceutically acceptable salt thereof for use as a medicament, wherein:
A is an anion;
L is a diradical selected from the group consisting of 1,2-phenylene*bis*(methylene), 1,3-phenylene*bis*(methylene), 1,4-phenylenebis(methylene) and a (C₂-C₁₂)-oligomethylene chain, wherein one to three methylene groups of the (C₂-C₁₂)-oligomethylene chain can be substituted by oxygen atoms;
R₁, R₂, R₇, and R₈ are radicals independently selected from the group consisting of hydrogen, (C₁-C₄)-alkyl, hydroxy-(C₁-C₄)-alkyl, cyano-(C₁-C₄)-alkyl, (C₁-C₄)-alkanoyloxy-(C₁-C₄)-alkyl, phenyl, (C₁-C₄)-alkanoyl, (C₁-C₄)-alkanesulfonyl, benzenesulfonyl, naphthalenesulfonyl, and *p-*toluenesulfonyl, or alternatively, when taken in combination, R₁-N-R₂ and R₇-N-R₈ are independently selected from the group consisting of azetidine, pyrrolidine, piperidine, azepine, piperazine, 4-(C₁-C₂)-alkylpiperazine, and morpholine ring;
R₃ and R₆ are radicals independently selected from the group consisting of hydrogen, (C₁-C₄)-alkyl, nitro, amino, (C₁-C₄)-alkylamino, (C₁-C₄)-alkanamido, (C₁-C₄)-alkanesulfonamido, benzene-sulfonamido, naphthalenesulfonamido, and *p*-toluenesulfonamido; and
R₄ and R₅ are radicals independently selected from the group consisting of hydrogen, (C₁-C₄)-alkyl, halogen, amino, (C₁-C₄)-alkylamino, (C₁-C₄)-alkanamido, (C₁-C₄)-alkanesulfonamido, benzenesulfonamido, naphthalenesulfonamido, *p*-toluenesulfonamido, amino-(C₁-C₄)-alkyl, (C₁-C₂)-alkylamino-(C₁-C₄)-alkyl, (C₁-C₄)-alkanamido-(C₁-C₄)-alkyl, (C₁-C₄)-alkanesulfonamido-(C₁-C₄)-alkyl, benzenesulfonamido-(C₁-C₄)-alkyl, naphthalenesulfonamido-(C₁-C₄)-alkyl, and *p-*toluenesulfonamido-(C₁-C₄)-alkyl.

The first aspect can alternatively be formulated as related to the compound as defined above for use as an active pharmaceutical ingredient or for treating a disease. Alternatively, the invention also relates to the use of the compounds defined above for the manufacture of a medicament or a pharmaceutical composition. Alternatively, the invention also relates to a method to prevent and/or treat a disease in a subject in need thereof, comprising administering to the subject a therapeutically effective amount of a compound as defined in the first aspect of the invention.

A second aspect of the invention relates to a compound as defined in the first aspect of the invention, for use in the treatment, prevention or amelioration of malaria, or symptoms, complications and/or sequela thereof. Alternatively, the invention relates to the use of a compound as defined in the first aspect for the manufacture of a medicament for the treatment, prevention or amelioration of malaria, or symptoms, complications and/or sequela thereof. Alternatively, the invention relates to a method to treat, prevent, or ameliorate malaria, or symptoms, complications and/or sequela thereof, in a subject in need thereof, comprising administering to the subject a therapeutically effective amount of a compound as defined in the first aspect of the invention.

A third aspect of the invention relates to a pharmaceutical composition comprising an effective amount of a compound as defined in the first aspect of the invention, together with appropriate amounts of pharmaceutically acceptable excipients or carriers.

A fourth aspect of the invention relates to a compound of formula (I) 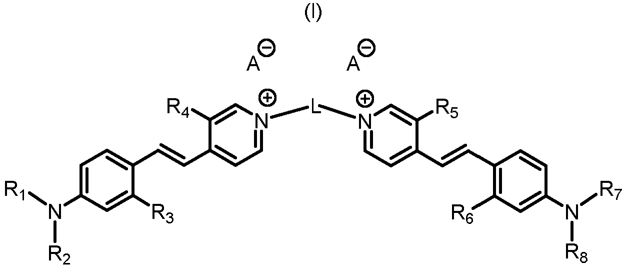 or a pharmaceutically acceptable salt thereof, wherein:
A is an anion;
L is a diradical selected from the group consisting of ethylene, trimethylene, tetramethylene, pentamethylene, hexamethylene, heptamethylene, octamethylene, nonamethylene, decamethylene, undecamethylene, dodecamethylene, 3-oxapentamethylene, 3,6-dioxaoctamethylene, 3,6,9-trioxaundecamethylene, 1,2-phenylene*bis*(methylene), 1,3-phenylene*bis*(methylene), and 1,4-phenylene*bis*(methylene);
R₁, R₂, R₇, and R₈ are radicals independently selected from the group consisting of hydrogen, (C₁-C₄)-alkyl, hydroxy-(C₁-C₄)-alkyl, cyano-(C₁-C₄)-alkyl, (C₁-C₄)-alkanoyloxy-(C₁-C₄)-alkyl, phenyl, (C₁-C₄)-alkanoyl, (C₁-C₄)-alkanesulfonyl, benzenesulfonyl, naphthalenesulfonyl, and *p-*toluenesulfonyl or alternatively, when taken in combination, R₁-N-R₂ and R₇-N-R₈ are independently selected from the group consisting of azetidine, pyrrolidine, piperidine, azepine, piperazine, 4-(C₁-C₂)-alkylpiperazine, and morpholine ring;
R₃ and R₆ are radicals independently selected from the group consisting of hydrogen, (C₁-C₄)-alkyl, nitro, amino, (C₁-C₄)-alkylamino, (C₁-C₄)-alkanamido, (C₁-C₄)-alkanesulfonamido, benzenesulfonamido, naphthalenesulfonamido, and *p*-toluenesulfonamido; and
R₄ and R₅ are radicals independently selected from the group consisting of hydrogen, (C₁-C₄)-alkyl, halogen, amino, (C₁-C₄)-alkylamino, (C₁-C₄)-alkanamido, (C₁-C₄)-alkanesulfonamido, benzenesulfonamido, naphthalenesulfonamido, *p*-toluenesulfonamido, amino-(C₁-C₄)-alkyl, (C₁-C₂)-alkylamino-(C₁-C₄)-alkyl, (C₁-C₄)-alkanamido-(C₁-C₄)-alkyl, (C₁-C₄)-alkanesulfonamido-(C₁-C₄)-alkyl, benzenesulfonamido-(C₁-C₄)-alkyl, naphthalenesulfonamido-(C₁-C₄)-alkyl, and *p-*toluenesulfonamido-(C₁-C₄)-alkyl;
with the proviso that when R₁, R₂, R₃, R₄, R₅, R₆, R₇ and R₈ are hydrogen, L is not 1,4-phenylene*bis*(methylene);
with the proviso that when R₁, R₂, R₇ and R₈ are methyl and R₃, R₄, R₅ and R₆ are hydrogen, L is not ethylene, trimethylene, tetramethylene, pentamethylene, hexamethylene, decamethylene, dodecamethylene, 3,6-dioxaoctamethylene, 1,2-phenylene*bis*(methylene), 1,3-phenylene*bis*(methylene) or 1,4-phenylene*bis*(methylene);
with the proviso that when R₁, R₂, R₇ and R₈ are ethyl and R₃, R₄, R₅ and R₆ are hydrogen, L is not trimethylene, tetramethylene, 3-oxapentamethylene, 1,2-phenylene*bis*(methylene) or 1,4-phenylene*bis*(methylene);
with the proviso that when R₁, R₂, R₇ and R₈ are phenyl and R₃, R₄, R₅ and R₆ are hydrogen, L is not trimethylene, tetramethylene, pentamethylene, hexamethylene, octamethylene, decamethylene, dodecamethylene, 1,2-phenylene*bis*(methylene), 1,3-phenylene*bis*(methylene) or 1,4-phenylene*bis*(methylene);
with the proviso that when R₁, R₂, R₇ and R₈ are HO-CH₂-CH₂- and R₃, R₄, R₅ and R₆ are hydrogen, L is not tetramethylene;
with the proviso that when R₁ and R₇ are ethyl, R₂ and R₈ are HO-CH₂-CH₂- and R₃, R₄, R₅ and R₆ are hydrogen, L is not ethylene, trimethylene, tetramethylene, pentamethylene, hexamethylene or heptamethylene;
with the proviso that when R₁ and R₇ are methyl, R₂ and R₈ are NC-CH₂- and R₃, R₄, R₅ and R₆ are hydrogen, L is not 1,2-phenylene*bis*(methylene) or 1,4-phenylene*bis*(methylene),
with the proviso that when R₁ and R₇ are acetyl and R₂, R₃, R₄, R₅ R₆ and R₈ are hydrogen, L is not 1,4-phenylene*bis*(methylene);
with the proviso that when R₁, R₂, R₃, R₆, R₇ and R₈ are methyl and R₄ and R₅ are hydrogen, L is not trimethylene or 1,4-phenylene*bis*(methylene);
with the proviso that when R₁, R₂, R₄, R₅, R₇ and R₈ are methyl and R₃ and R₆ are hydrogen, L is not trimethylene, tetramethylene or decamethylene;
with the proviso that when R₁, R₂, R₇ and R₈ are ethyl, R₃ and R₆ are hydrogen and R₄ and R₅ are methyl, L is not decamethylene;
with the proviso that when R₁-N-R₂ and R₇-N-R₈ are pyrrolidino and R₃, R₄, R₅ and R₆ are hydrogen, L is not trimethylene or 1,4-phenylenebis(methylene);
with the proviso that when R₁-N-R₂ and R₇-N-R₈ are piperidino and R₃, R₄, R₅ and R₆ are hydrogen, L is not trimethylene, 1,2-phenylene*bis*(methylene), 1,3-phenylene*bis*(methylene) or 1,4-phenylene*bis*(methylene); and
with the proviso that when R₁-N-R₂ and R₇-N-R₈ are 4-methylpiperazino and R₃, R₄, R₅ and R₆ are hydrogen, L is not trimethylene.

A fifth aspect of the invention relates to a process for the preparation of the compound of formula (I) according to the fourth aspect, which comprises the following steps:
A) reacting 4-methylpyridines of formula (II) with alkylating reagents of formula (III), wherein X is a common leaving group, such as halide, acyloxy, sulfonate or sulfate; and
B) reacting the resulting compound of formula (IV) with a 4-aminobenzaldehyde of formula (V), and, if necessary, by standard transformation of functional groups that are present in radicals R₁-R₈ according to the fourth aspect.

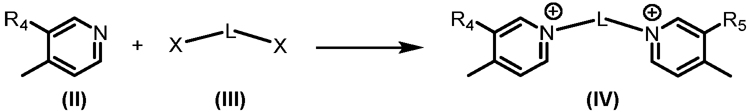

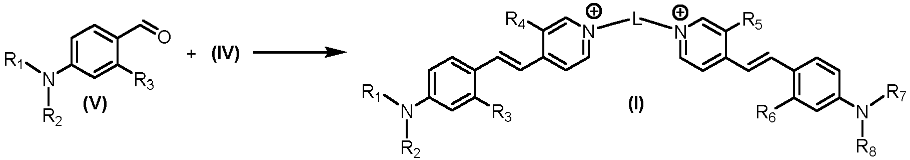

Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein. The following examples and drawings are provided herein for illustrative purposes, and without intending to be limiting to the present invention.

### DESCRIPTION OF DRAWINGS

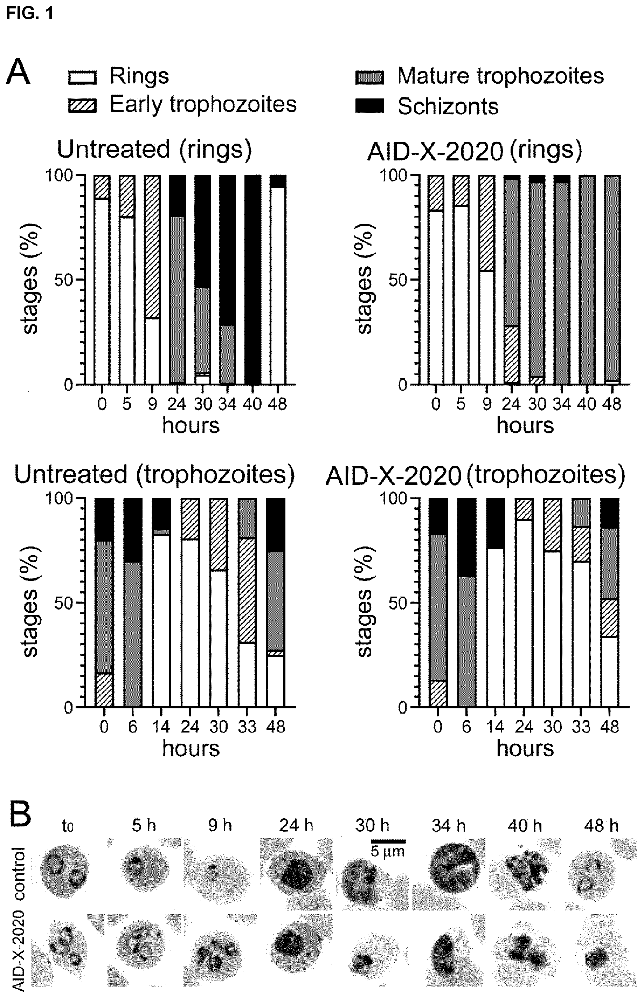

**FIG. 1** shows the stage of growth inhibition of *P*. *falciparum* during 48 h of 200 nM AID-X-2020 treatment. The drug was added to synchronized parasite cultures at the ring or trophozoite stages. Giemsa-stained blood smears were prepared at the indicated time points between 0 and 48 h of incubation, and the numbers of ring stages, early trophozoites, late trophozoites and schizonts were counted in samples of at least 100 pRBCs for each time point. (A) Bars indicate the percentages of developmental stages present in the respective blood smears. (B) Representative images of pRBCs in the assay where AID-X-2020 was added at ring stage.

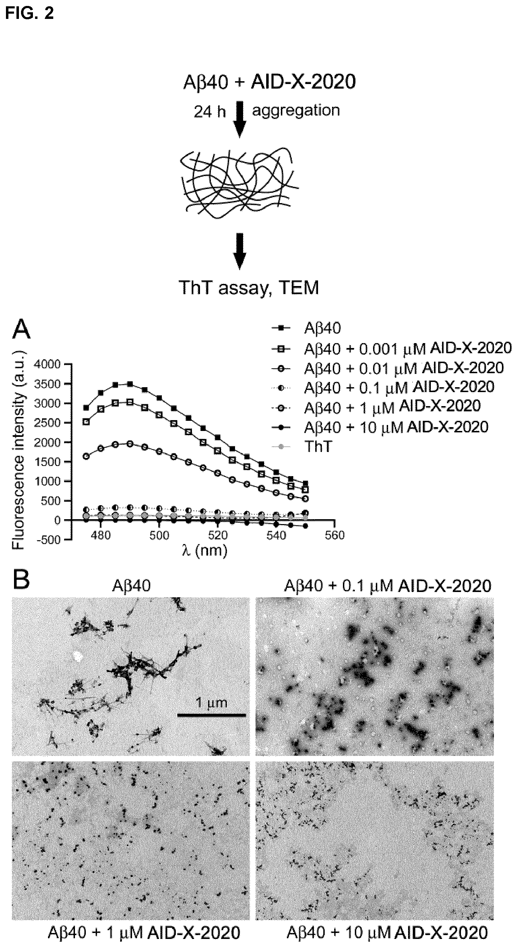

**FIG. 2** shows the effect of AID-X-2020 at different concentrations on the *in vitro* aggregation of Aβ40. (A) ThT fluorescence assay, which shows the effect of AID-X-2020 on inhibiting aggregation of Aβ40. a.u.: arbitrary units. λ: wavelength (nm). (B) TEM analysis shows amyloid fibril aggregates of AID-X-2020-containing samples and control untreated Aβ40 samples. In the presence of AID-X-2020, aggregates are smaller and more fragmented than those untreated.

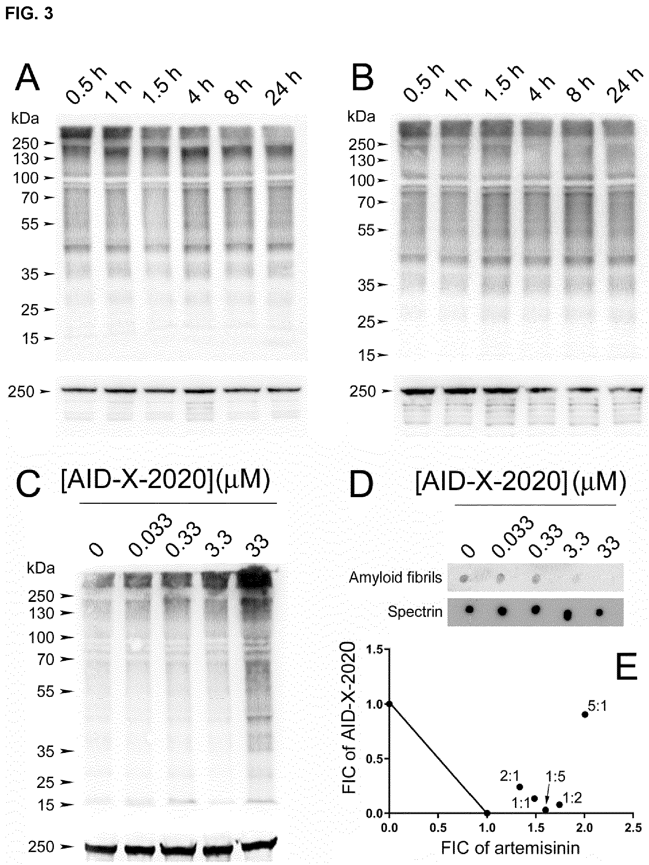

**FIG. 3** shows the effect of AID-X-2020 on markers of protein aggregation in live *P. falciparum* cultures. (A-C) Western blot assays for the detection of ubiquitinated proteins in cultures treated (A) for 24 h with 90 nM AID-X-2020, or (C) for 90 min with different concentrations of the compound. (B) corresponds to an untreated control. Anti-spectrin antibody is used as loading control shown at the bottom of each gel. (D) Dot blot assay of the same samples from panel C using an antibody against amyloid fibrils. (E) Isobologram of the interaction between AID-X-2020 (Y) and artemisinin (A) at different Y:A ratios.

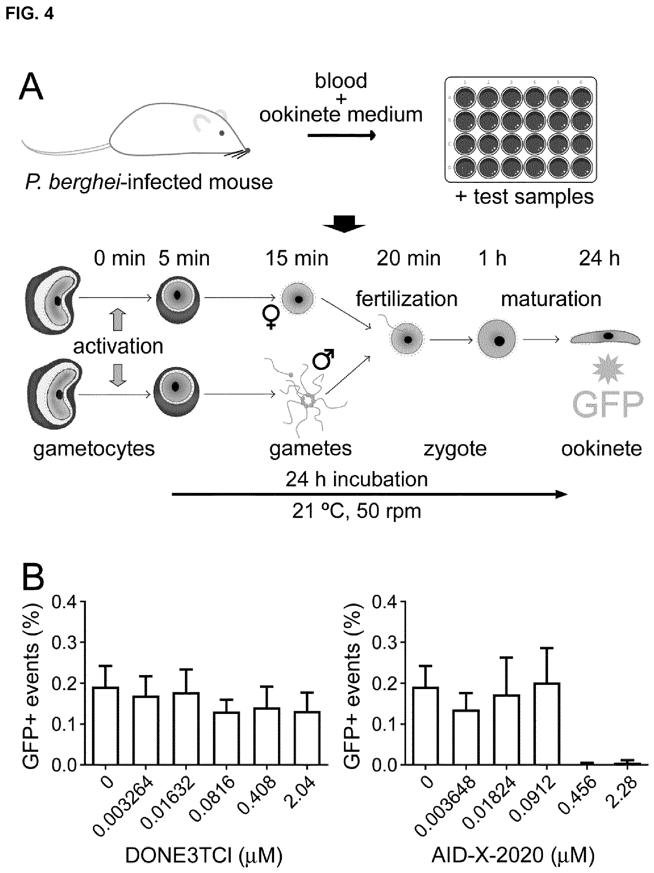

**FIG. 4** shows the results of an *in vitro* inhibition assay of the gametocyte to ookinete transition in *P. berghei.* (A) Scheme of the experimental protocol. For ookinete production, blood carrying gametocytes were collected from infected mouse with *Plasmodium berghei* CTRP-GFP, which expresses GFP when reaching ookinete stage. The effect of AID-X-2020 and DONE3TCI on gametocyte to ookinete conversion was assessed in 96-well plates, by plating each culture with growing concentrations of the compounds for 24 h of incubation. 24 h later, samples were analyzed in a flow cytometer to select GFP positive cell population. (B) Effect of DONE3TCI and AID-X-2020 on ookinete production. Because the CTRP protein is only expressed in ookinetes, the presence of a fluorescence signal is representative of *P*. *berghei* CTRP-GFP reaching the ookinete stage (GFP+ events, %), whereas a lack of fluorescence is representative of *P*. *berghei* CTRP-GFP not reaching the ookinete stage. Administration of AID-X-2020 at 0.5 µM abolishes ookinete production by the parasite, whereas administration of DONE3TCI at up to 2 µM does not show any observable effect on ookinete development.

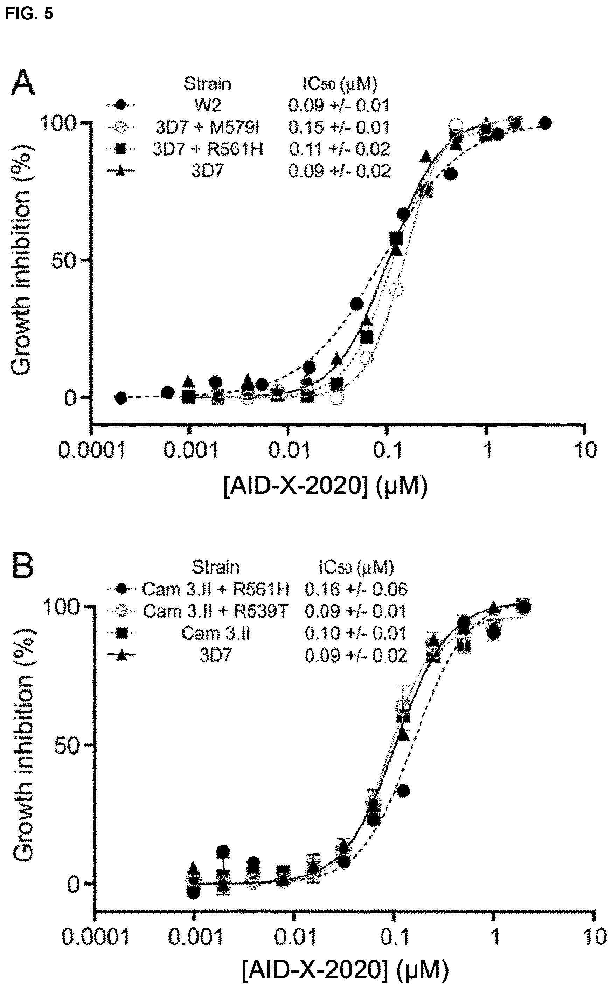

**FIG. 5** shows AID-X-2020 *in vitro* growth inhibition assays in chloroquine- and artemisinin-resistant *P. falciparum* strains. (A) Chloroquine-resistant W2 strain and artemisinin-resistant M579I and R561H strains compared to parental 3D7 and (B) Cam 3.II artemisinin-resistant R561H and R539T strains compared to parental Cam 3.II (chloroquine and sulfadoxine/pyrimethamine-resistant strain) and to 3D7 (chloroquine-sensitive strain). In both panels means ± SD are shown.

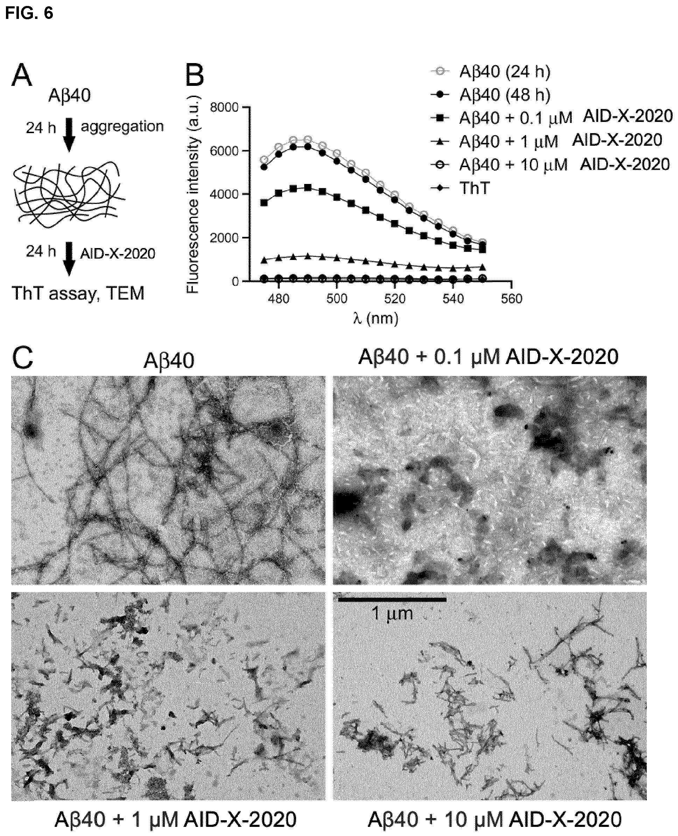

**FIG. 6** shows the effect of AID-X-2020 on pre-aggregated Aβ40. (A) Scheme of the assay. (B) ThT fluorescence assay. a.u.: arbitrary units. The mean fluorescence intensity value of each sample in each wavelength is represented. (C) TEM analysis of the samples after 48 h of incubation.

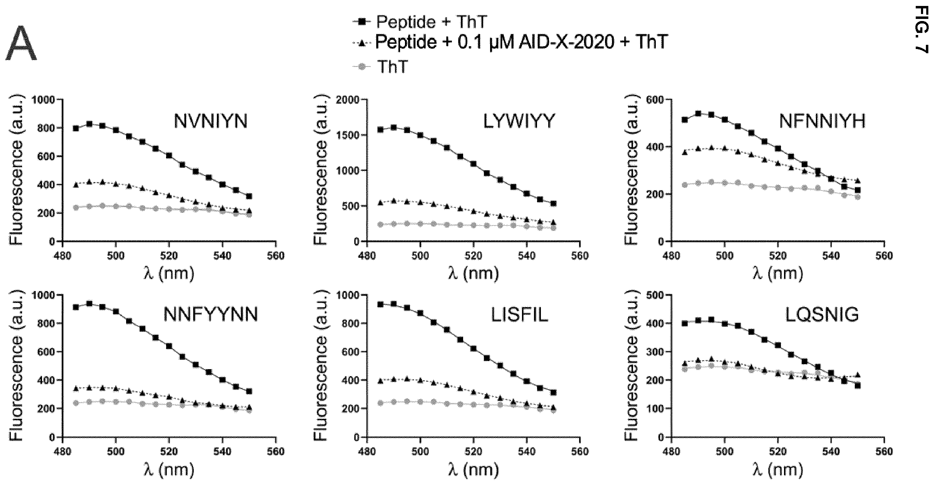

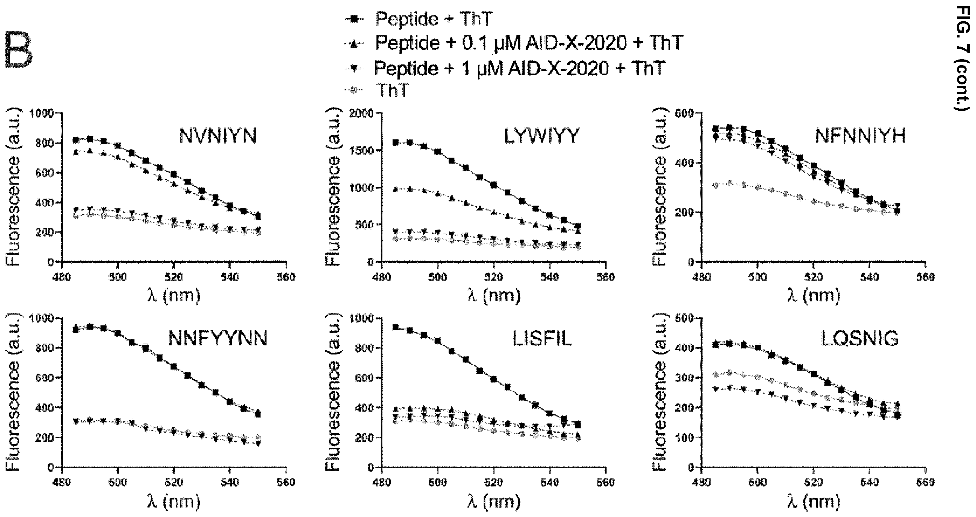

**FIG. 7** shows the ThT analysis of the effect of AID-X-2020 on the *in vitro* aggregation of aggregative peptides present in *P. falciparum* proteins. (A) Inhibition of aggregation assay. (B) Disaggregation assay. In both panels the mean fluorescence intensity value of each sample in each wavelength is represented.

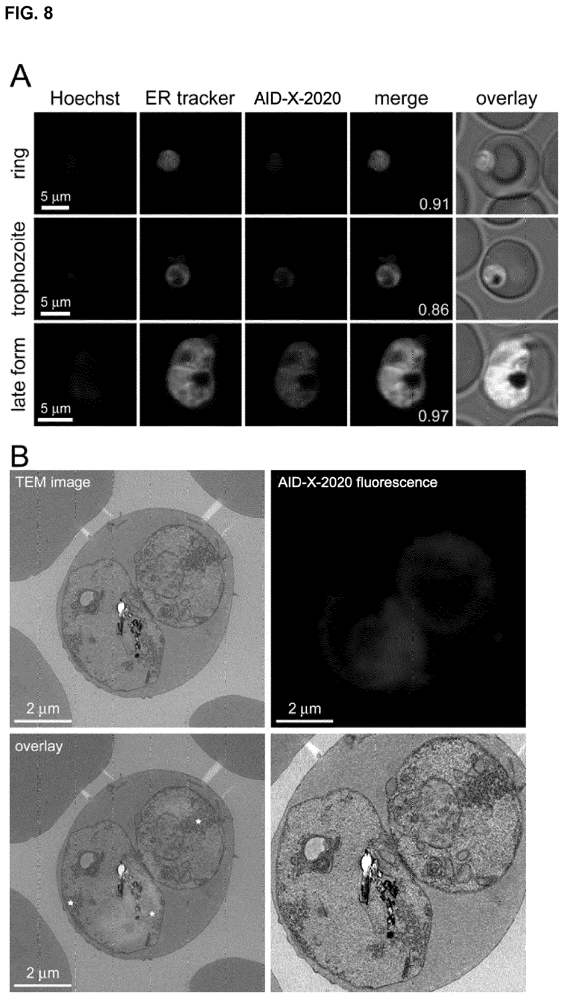

**FIG.** 8 shows the subcellular localization of AID-X-2020 in pRBCs. (A) Confocal fluorescence microscopy colocalization analysis in different *P*. *falciparum* blood stages of AID-X-2020 with the cytoplasmic marker ER-Tracker^{™} Green. The merge image corresponds to red (AID-X-2020) and green (ER tracker) channels only. Manders' overlap correlation coefficients are indicated in yellow digits. (B) Correlative light and electron microscopy analysis. The stars in the overlay image indicate three ER regions. The blown-up micrograph in the lower right panel is included for a better identification of subcellular structures.

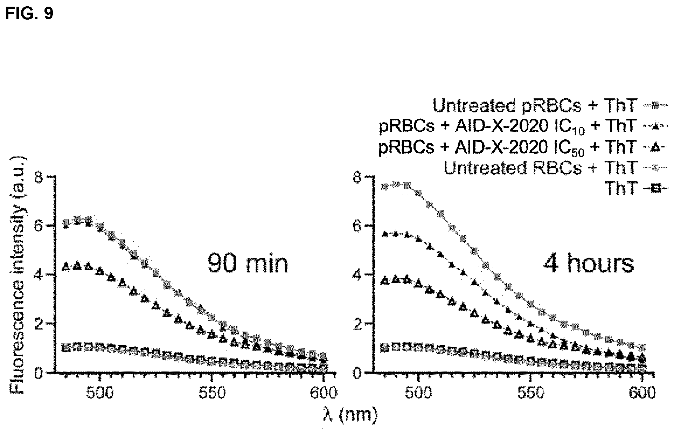

**FIG. 9** shows ThT fluorescence of *P. falciparum* culture extracts normalized to have equal protein content, either non-treated or treated with AID-X-2020 at its *in vitro* IC₁₀ (27 nM) and IC₅₀ (90 nM), for 90 min and 4 h. A non-parasitized RBC protein extract is shown as reference. The mean fluorescence intensity value of each sample in each wavelength is represented.

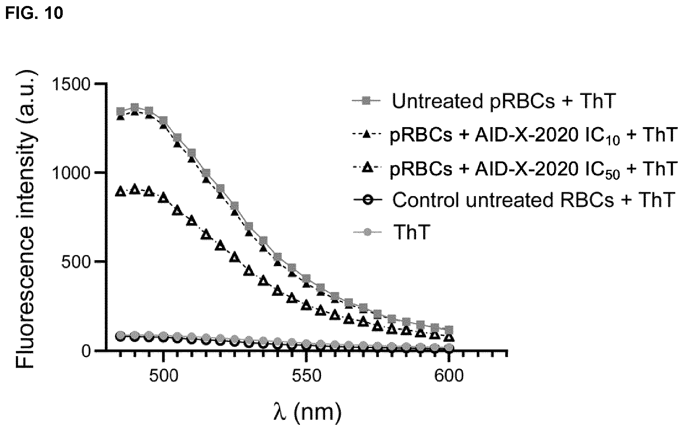

**FIG. 10** shows protein aggregation in live *P. falciparum* cultures. ThT fluorescence of *P. falciparum* culture extracts were normalized to have equal protein content, either non-treated or treated with AID-X-2020 at its *in vitro* IC₁₀ (27 nM) and IC₅₀ (90 nM), for 30 h. A non-parasitized RBC protein extract is shown as reference. The mean fluorescence intensity value of each sample in each wavelength is represented.

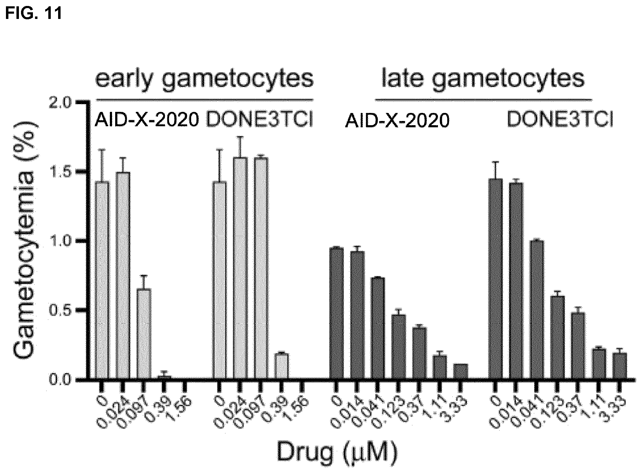

**FIG. 11** shows *in vitro* activity of AID-X-2020 and DONE3TCI on *Plasmodium* early and late gametocytes. Bars represent mean ± SD of two independent experiments.

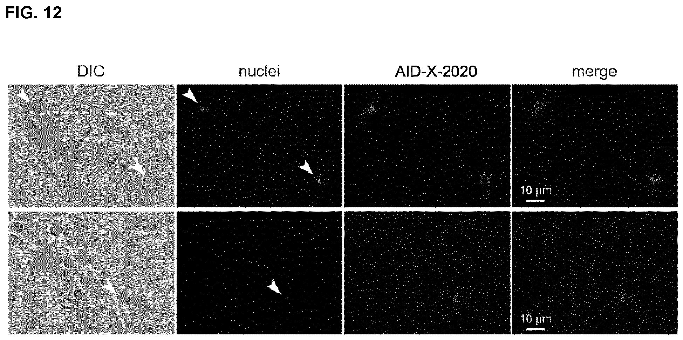

**FIG. 12** shows AID-X-2020 staining of a clinical sample of a *P. falciparum* infection. DIC: differential interference contrast image. The merge panel refers to fluorescence images only. Arrowheads indicate the three *P. falciparum-infected* red blood cells present in the two microscope fields shown.

### DETAILED DESCRIPTION OF THE INVENTION

### Definitions

Excipient: The terms "excipient" and "carrier" are used interchangeably and refer to an inert substance added to a e.g., pharmaceutical composition, to further facilitate administration of a compound of the present disclosure.

Prevent: The terms "prevent," "preventing," "prophylaxis" and variants thereof as used herein, refer, e.g., to
(i) partially or completely delaying onset of a disease, disorder and/or condition disclosed herein;
(ii) partially or completely delaying onset of one or more symptoms, features, or clinical manifestations, complications, or sequelae of a particular disease, disorder, and/or condition disclosed herein;
(iii) partially or completely delaying onset of one or more symptoms, features, or manifestations, complications, or sequelae of a particular disease, disorder, and/or condition disclosed herein;
(iv) partially or completely delaying progression from a particular disease, disorder and/or condition disclosed herein; and/or
(v) decreasing the risk of developing pathology associated with the disease, disorder, and/or condition disclosed herein.

Subject: The terms "subject", "patient", "individual", "host" and variants thereof are used interchangeably herein and refer to any mammalian subject, particularly humans, but also including without limitation, humans, domestic animals (e.g., dogs, cats and the like), farm animals (e.g., cows, sheep, pigs, horses and the like), and laboratory animals (e.g., monkey, rats, mice, rabbits, guinea pigs and the like) for whom diagnosis, treatment, or therapy is desired. The compositions and methods described herein are applicable to both human therapy and veterinary applications.

Subject in need thereof: As used herein, "subject in need thereof" includes subjects, such as mammalian subjects, that would benefit from administration of the compositions of the disclosure.

Therapeutically effective amount: As used herein the term "therapeutically effective amount" is the amount of a compound/composition of the present disclosure that is sufficient to produce a desired therapeutic effect, pharmacological and/or physiological effect on a subject in need thereof.

Treatment: The terms "treat", "treatment", "therapy" as used herein refer to, e.g., the reduction in severity of disease or condition disclosed herein; the amelioration or elimination of one or more symptoms, complications, or sequelae associated with a disease disclosed herein (e.g., malaria); the provision of beneficial effects to a subject with a condition/disease disclosed herein, without necessarily curing the disease or condition. The term also includes prophylaxis or prevention of a disease or condition or symptoms, complications, or sequelae thereof.

The term refers to a clinical or nutritional intervention to prevent the disease or condition; cure the disease or condition; delay onset of the disease or condition; delay onset of a symptom, complication or sequela; reduce the seriousness of the disease or condition; reduce the seriousness of a symptom, complication, or sequela; improve one or more symptoms; improve one or more complications; improve one or more sequelae; prevent one or more symptoms; prevent one or more complications; prevent one or more sequelae; delay one or more symptoms; delay one or more symptoms; delay one or more complications; delay one or more sequelae; ameliorate one or more symptoms; ameliorate one or more complications; ameliorate one or more sequelae; shorten the duration of one or more symptoms; shorten the duration of one or more complications; shorten the duration of one or more sequelae; reduce the frequency of one or more symptoms; reduce the frequency of one or more complications; reduce the frequency of one or more sequelae; reduce the severity of one or more symptoms; reduce the severity of one or more complications; reduce the severity of one or more sequelae; improve the quality of life; increase survival; prevent a recurrence of the disease or condition; delay a recurrence of the disease or condition; or any combination thereof, e.g., with respect to what is expected in the absence of the treatment with the compound/composition of the present disclosure.

Symptom: As used herein, the term "symptom" refers to subjective or physical sign, indication, or evidence of disease or physical disturbance observed by the subject. In general, the term refers to any morbid phenomenon or departure from the normal in structure, function, or sensation, experienced by the patient and indicative of disease. Symptoms are felt or noticed by the individual experiencing the symptom but may not easily be noticed by others. In some aspects, a symptom can be a mild symptom, a moderate symptom, or a severe symptom. As used herein, the term "mild symptom" refers to a symptom that is not life threatening and does not require, e.g., intensive care treatment (e.g., at a hospital ICU). As used herein, the term "moderate symptom" refers to a symptom that requires monitoring because it may become life threatening and may require, e.g., hospitalization. As used herein, the term "severe symptom" refers to a symptom that is life threatening and requires, e.g., intensive care treatment (e.g., at a hospital ICU).

Complication: As used herein, the term "complication" refers to a pathological process or event occurring during a disease or condition that is not an essential part of the disease or condition; where it may result from the disease/condition or from independent causes. Accordingly, the term complication refers to medical/clinical problems that are observed in subjects diagnosed with a disease or condition disclosed herein, e.g., malaria. In some aspects, a complication can be temporary. In some aspects, a complication can be chronic or permanent.

Sequela: As used herein, the term "sequela" refers to a long term, chronic, or permanent complication.

Pharmaceutically acceptable salt: As used herein, the term "pharmaceutically acceptable salt" refers to that the salt derived from the corresponding compound is suitable for administration to a subject to achieve the treatments described herein, without unduly deleterious side effects in light of the severity of the disease and necessity of the treatment.

### Compounds of formula (I) or pharmaceutically acceptable salts thereof

In some embodiments of the first aspect, the invention provides a family of bis(aminostyrylpyridinium) compounds containing a tether chain to connect both aminostyrylpyridinium moieties, differently substituted by pyridinium and aminophenyl rings. In a particular embodiment, the invention provides a family of homodimeric bis(aminostyrylpyridinium) compounds, i.e., R₁ = R₇, R₂ = R₈, R₃ = R₆, and R₄ = R₃ or alternatively, when used in combination, R₁-N-R₂ = R₇-N-R₈, R₃ = R₆, and R₄ = R₅. In another particular embodiment, the invention provides a family of heterodimeric bis(aminostyrylpyridinium) compounds.

In some embodiments, the invention relates to salts of compounds of formula (I). Examples of salts can be but are not limited to hydrochloride, hydrobromide, sulfate, nitrate, phosphates, acetate, propionate, benzoate, maleate, hemimaleate, fumarate, lactate, tartrate, citrate, succinate, hemisuccinate, glycollate, gluconate, tosylate, mesylate, esylate, napsylate, isethionate, besylate, hexanoate, octanoate, decanoate, oleate, or stearate.

It is understood that since the compounds described in the present invention comprise cationic groups, there will also be anionic counterions present. Any anion may serve this purpose if it does not interfere with the use of the compounds. Examples of anions that may serve as counterions can include but are not limited to halide anions, such as bromide (Br⁻), iodide (I⁻), or chloride (Cl⁻), sulfonate anions, such as mesylate, tosylate, esylate, isethionate, napsylate or besylate, or any other pharmaceutically acceptable anions, such as those derived from carboxylic acids (acetate, propionate, maleate, benzoate, fumarate), hydroxy acids (citrate, lactate, succinate, tartrate) or fatty acids (hexanoate, octanoate, decanoate, oleate, stearate). Particularly, anions are selected from the group consisting of iodide, bromide and chloride.

It should also be appreciated by those skilled in the art that the stoichiometric number of counterion or counterions which balance the charge or charges on the compound can be the same or they can be different provided that the counterions balance the charge(s) on the compound. The combination of counterions can be selected from any of the above-mentioned anions.

In some embodiments, the diradical, also referred to as diradical linker (L), that connects the two aminostyrylpyridinium moieties is an oligomethylene chain from 2 to 12 carbon atoms, in which one to three methylene groups can be optionally replaced by oxygen atoms.

In some embodiments, the diradical can be an ortho-, meta, or para-phenylene group (1,2-phenylene*bis*(methylene), 1,3-phenylene*bis*(methylene) or 1,4-phenylene*bis*(methylene)).

In some embodiments, the aminostyrylpyridinium moieties can be unsubstituted or optionally substituted at position 3 (R₄ and R₅) of the pyridinium ring with a (C₁-C₄)-alkyl group, a halogen atom, an amino group, an amino-(C₁-C₄)-alkyl group, a (C₁-C₂)-alkylamino-(C₁-C₄)-alkyl group, a (C₁-C₄)-alkanamido group, a (C₁-C₄)-alkanesulfonamido group, a benzenesulfonamido group, a naphthalenesulfonamido group, a toluenesulfonamido group. More particularly, the pyridinium ring contains hydrogen at position 3 or is substituted at position 3 with chlorine, bromine, an amino group, or a methyl group.

In some embodiments, the aminostyrylpyridinium moieties can be substituted at the amino group (R₁, R₂, R₇, and R₈) with a (C₁-C₄)-alkyl group, a hydroxy-(C₁-C₄)-alkyl group, a cyano-(C₁-C₄)-alkyl group, a (C₁-C₄)-alkanoyloxy-(C₁-C₄)-alkyl group, a phenyl group, a (C₁-C₄)-alkanesulfonyl group, a benzenesulfonyl group, a naphthalenesulfonyl group or a toluenesulfonyl group. More particularly, the amino group is substituted with two methyl, ethyl, phenyl, or 2-acetyloxyethyl groups or it is substituted with a methyl and a 2-cyanoethyl group.

In some embodiments, the nitrogen atom of the amino group of the aminostyrylpyridinium moiety can be part of a heterocycle such as azetidine, pyrrolidine, piperidine, azepine, piperazine or morpholine. More particularly, the nitrogen atom of the amino group is part of a pyrrolidine, a piperidine, a piperazine or a morpholine ring.

In some embodiments, the aminostyrylpyridinium moieties can be substituted at position 2 of the phenyl ring (R₃ and R₆) with a (C₁-C₄)-alkyl group, a nitro group, an amino group, a (C₁-C₄)-alkylamino group, a (C₁-C₄)-alkanamido group, a (C₁-C₄)-alkanesulfonamido group, a benzenesulfonamido group, a naphthalenesulfonamido group or a toluenesulfonamido group. Alternatively, the aminostyrylpyridinium moieties can be substituted at position 2 of the phenyl ring (R₃ and R₆) with a hydroxy group, a (C₁-C₄)-alkoxy group or a (C₁-C₄)-alkanoyloxy group. In a particular embodiment, the aminostyrylpyridinium moieties are substituted at position 2 of the phenyl ring with a hydroxy group. More particularly, the compound is 1,1'-(decane-1,10-diyl)*bis*{4-[(*E*)-4-(diethylamino)-2-hydroxystyryl]-3-methylpyridin-1-ium} dibromide (PRC-69).

In some embodiments, the compound for use is according to the first aspect, wherein:
L is a diradical selected from the group consisting of 1,2-phenylene*bis*(methylene), 1,3-phenylene*bis*(methylene), 1,4-phenylene*bis*(methylene) and a (C₄-C₁₂)-oligomethylene chain, wherein one to three methylene groups of the (C₄-C₁₂)-oligomethylene chain can be substituted by oxygen atoms;
R₁, R₂, R₇, and R₈ are radicals independently selected from the group consisting of (C₁-C₂)-alkyl, cyano-(C₁-C₂)-alkyl, and phenyl, or alternatively, when taken in combination, R₁-N-R₂ and R₇-N-R₈ are independently selected from the group consisting of pyrrolidine, piperidine, azepine, piperazine, 4-methylpiperazine, and morpholine;

R₃ and R₆ are hydrogen; and
R₄ and R₅ are independently selected from the group consisting of hydrogen, methyl, chloro, bromo, fluoro and iodo.

In a particular embodiment, the compound for use is according to the first aspect, wherein:
L is a diradical selected from the group consisting of 1,3-phenylene*bis*(methylene), 1,4-phenylene*bis*(methylene), a C₄-oligomethylene chain, and a (C₈-C₁₂)-oligomethylene chain, wherein two or three methylene groups of the oligomethylene chains can be substituted by two or three oxygen atoms;
R₁, R₂, R₇, and R₈ are radicals independently selected from the group consisting of (C₁-C₂)-alkyl, cyano-C₂-alkyl and phenyl, or alternatively, when taken in combination, R₁-N-R₂ and R₇-N-R₈ are independently selected from pyrrolidine and morpholine;
R₃ and R₆ are hydrogen; and
R₄ and R₅ are independently selected from the group consisting of hydrogen, methyl, chloro, and bromo.

In some embodiments, the compound for use according to the first aspect of the invention is selected from the group consisting of:
1,1'-(decane-1,10-diyl)*bis*{3-methyl-4-[(*E*)-4-(pyrrolidin-1-yl)styryl]pyridin-1-ium} dibromide (EMA368);
1,1'-(decane-1,10-diyl)*bis*{3-methyl-4-[(*E*)-4-(piperidin-1-yl)styryl]pyridin-1-ium} dibromide;
1,1'-(decane-1,10-diyl)*bis*{3-methyl-4-[(*E*)-4-morpholinostyryl]pyridin-1-ium} dibromide (EMA377);
1,1'-(decane-1,10-diyl)*bis*{3-methyl-4-[(*E*)-4-(piperazin-1-yl)styryl]pyridin-1-ium} dibromide;
1,1'-(decane-1,10-diyl)*bis*{4-[(*E*)-4-(diphenylamino)styryl]-3-methylpyridin-1-ium} dibromide (EMA366);
1,1'-(decane-1,10-diyl)*bis*{4-[(*E*)-4-(dimethylamino)styryl]-3-methylpyridin-1-ium} dibromide (EMA357);
1,1'-(decane-1,10-diyl)*bis*{4-[(*E*)-4-(dimethylamino)-2-nitrostyryl]-3-methylpyridin-1-ium} dibromide;
1,1'-(decane-1,10-diyl)*bis*{4-{(*E*)-4-[(2-cyanoethyl)methylamino]styryl}-3-methylpyridin-1-ium} dibromide (EMA359);
1,1'-(decane-1,10-diyl)*bis*{4-{(*E*)-4-[bis(2-acetoxyethyl)amino]styryl}-3-methylpyridin-1-ium} dibromide;
1,1'-(decane-1,10-diyl)*bis*{4-{(*E*)-4-[bis(2-hydroxyethyl)amino]styryl}-3-methylpyridin-1-ium} dibromide;
1,1'-(ethane-1,2-diyl)*bis*{4-[(*E*)-4-(diethylamino)styryl]-3-methylpyridin-1-ium} dibromide;
1,1'-(propane-1,3-diyl)*bis*{4-[(*E*)-4-(diethylamino)styryl]-3-methylpyridin-1-ium} dibromide;
1,1'-(butane-1,4-diyl)*bis*{4-[(*E*)-4-(diethylamino)styryl]-3-methylpyridin-1-ium} dibromide (PRC-5);
1,1'-(pentane-1,5-diyl)*bis*{4-[(*E*)-4-(diethylamino)styryl]-3-methylpyridin-1-ium} dibromide;
1,1'-(hexane-1,6-diyl)*bis*{4-[(*E*)-4-(diethylamino)styryl]-3-methylpyridin-1-ium} dibromide;
1,1'-(heptane-1,7-diyl)*bis*{4-[(*E*)-4-(diethylamino)styryl]-3-methylpyridin-1-ium} dibromide;
1,1'-(octane-1,8-diyl)*bis*{4-[(*E*)-4-(diethylamino)styryl]-3-methylpyridin-1-ium} dibromide (PRC-8);
1,1'-(nonane-1,9-diyl)*bis*{4-[(*E*)-4-(diethylamino)styryl]-3-methylpyridin-1-ium} dibromide (PRC-25);
1,1'-(decane-1,10-diyl)*bis*{4-[(*E*)-4-(diethylamino)styryl]-3-methylpyridin-1-ium} dibromide (AID-X-2020);
1,1'-(undecane-1,11-diyl)*bis*{4-[(*E*)-4-(diethylamino)styryl]-3-methylpyridin-1-ium} dibromide (PRC-14);
1,1'-(dodecane-1,12-diyl)*bis*{4-[(*E*)-4-(diethylamino)styryl]-3-methylpyridin-1-ium} dibromide (PRC-15);
1,1'-(3-oxapentane-1,5-diyl)*bis*{4-[(*E*)-4-(diethylamino)styryl]-3-methylpyridin-1-ium} dichloride;
1,1'-(3,6-dioxaoctane-1,8-diyl)*bis*{4-[(*E*)-4-(diethylamino)styryl]-3-methylpyridin-1-ium} diiodide (PRC-20);
1,1'-(3,6,9-trioxaundecane-1,11-diyl)*bis*{4-[(*E*)-4-(diethylamino)styryl]-3-methylpyridin-1-ium} dichloride (PRC-28);
1,1'-[1,2-phenylenebis(methylene)]*bis*{4-[(*E*)-4-(diethylamino)styryl]-3-methylpyridin-1-ium} dibromide;
1,1'-[1,3-phenylenebis(methylene)]*bis*{4-[(*E*)-4-(diethylamino)styryl]-3-methylpyridin-1-ium} dibromide (PRC-31);
1,1'-[1,4-phenylenebis(methylene)]*bis*{4-[(*E*)-4-(diethylamino)styryl]-3-methylpyridin-1-ium} dibromide (PRC-29);
1,1'-(decane-1,10-diyl)*bis*{4-[(*E*)-4-(diethylamino)styryl]pyridin-1-ium} dibromide (PRC-39);
1,1'-(decane-1,10-diyl)*bis*{3-bromo-4-[(*E*)-4-(diethylamino)styryl]pyridin-1-ium} dibromide (PRC-42); 1,1'-(decane-1,10-diyl)*bis*{3-chloro-4-[(*E*)-4-(diethylamino)styryl]pyridin-1-ium} dibromide (PRC-50); and 1,1'-(decane-1,10-diyl)*bis*{3-amino-4-[(*E*)-4-(diethylamino)styryl]pyridin-1-ium} dibromide.

In a particular embodiment, the compound for use according to the first aspect of the invention is selected from the group consisting of:
1,1'-(decane-1,10-diyl)*bis*{4-[(*E*)-4-(diethylamino)styryl]-3-methylpyridin-1-ium} dibromide (AID-X-2020);
1,1'-(decane-1,10-diyl)*bis*{4-[(*E*)-4-(dimethylamino)styryl]-3-methylpyridin-1-ium} dibromide (EMA357);
1,1'-(decane-1,10-diyl)*bis*{4-{(*E*)-4-[(2-cyanoethyl)methylamino]styryl}-3-methylpyridin-1-ium} dibromide (EMA359);
1,1'-(decane-1,10-diyl)*bis*{4-[(*E*)-4-(diphenylamino)styryl]-3-methylpyridin-1-ium} dibromide (EMA366);
1,1'-(decane-1,10-diyl)*bis*{3-methyl-4-[(*E*)-4-(pyrrolidin-1-yl)styryl]pyridin-1-ium} dibromide (EMA368);
1,1'-(decane-1,10-diyl)*bis*{3-methyl-4-[(*E*)-4-morpholinostyryl]pyridin-1-ium} dibromide (EMA377);
1,1'-(butane-1,4-diyl)*bis*{4-[(*E*)-4-(diethylamino)styryl]-3-methylpyridin-1-ium} dibromide (PRC-5);
1,1'-(octane-1,8-diyl)*bis*{4-[(*E*)-4-(diethylamino)styryl]-3-methylpyridin-1-ium} dibromide (PRC-8);
1,1'-(undecane-1,11-diyl)*bis*{4-[(*E*)-4-(diethylamino)styryl]-3-methylpyridin-1-ium} dibromide (PRC-14);
1,1'-(dodecane-1,12-diyl)*bis*{4-[(*E*)-4-(diethylamino)styryl]-3-methylpyridin-1-ium} dibromide (PRC-15);
1,1'-(3,6-dioxaoctane-1,8-diyl)*bis*{4-[(*E*)-4-(diethylamino)styryl]-3-methylpyridin-1-ium} diiodide (PRC-20);
1,1'-(nonane-1,9-diyl)*bis*{4-[(*E*)-4-(diethylamino)styryl]-3-methylpyridin-1-ium} dibromide (PRC-25);
1,1'-(3,6,9-trioxaundecane-1,11-diyl)*bis*{4-[(*E*)-4-(diethylamino)styryl]-3-methylpyridin-1-ium} dichloride (PRC-28);
1,1'-[1,4-phenylenebis(methylene)]*bis*{4-[(*E*)-4-(diethylamino)styryl]-3-methylpyridin-1-ium} dibromide (PRC-29);
1,1'-[1,3-phenylenebis(methylene)]*bis*{4-[(*E*)-4-(diethylamino)styryl]-3-methylpyridin-1-ium} dibromide (PRC-31);
1,1'-(decane-1,10-diyl)*bis*{4-[(*E*)-4-(diethylamino)styryl]pyridin-1-ium} dibromide (PRC-39);
1,1'-(decane-1,10-diyl)*bis*{3-bromo-4-[(*E*)-4-(diethylamino)styryl]pyridin-1-ium} dibromide (PRC-42); and
1,1'-(decane-1,10-diyl)*bis*{3-chloro-4-[(*E*)-4-(diethylamino)styryl]pyridin-1-ium} dibromide (PRC-50).

In a more particular embodiment, the compound for use according to the first aspect of the invention is selected from the group consisting of:
1,1'-(decane-1,10-diyl)*bis*{4-[(*E*)-4-(diethylamino)styryl]-3-methylpyridin-1-ium} dibromide (AID-X-2020);
1,1'-(decane-1,10-diyl)*bis*{4-[(*E*)-4-(dimethylamino)styryl]-3-methylpyridin-1-ium} dibromide (EMA357);
1,1'-(decane-1,10-diyl)*bis*{4-{(*E*)-4-[(2-cyanoethyl)methylamino]styryl}-3-methylpyridin-1-ium} dibromide (EMA359);
1,1'-(decane-1,10-diyl)*bis*{3-methyl-4-[(*E*)-4-morpholinostyryl]pyridin-1-ium} dibromide (EMA377);
1,1'-(octane-1,8-diyl)*bis*{4-[(*E*)-4-(diethylamino)styryl]-3-methylpyridin-1-ium} dibromide (PRC-8);
1,1'-(undecane-1,11-diyl)*bis*{4-[(*E*)-4-(diethylamino)styryl]-3-methylpyridin-1-ium} dibromide (PRC-14);
1,1'-(dodecane-1,12-diyl)*bis*{4-[(*E*)-4-(diethylamino)styryl]-3-methylpyridin-1-ium} dibromide (PRC-15);
1,1'-(nonane-1,9-diyl)*bis*{4-[(*E*)-4-(diethylamino)styryl]-3-methylpyridin-1-ium} dibromide (PRC-25);
1,1'-(decane-1,10-diyl)*bis*{4-[(*E*)-4-(diethylamino)styryl]pyridin-1-ium} dibromide (PRC-39);
1,1'-(decane-1,10-diyl)*bis*{3-bromo-4-[(*E*)-4-(diethylamino)styryl]pyridin-1-ium} dibromide (PRC-42); and
1,1'-(decane-1,10-diyl)*bis*{3-chloro-4-[(*E*)-4-(diethylamino)styryl]pyridin-1-ium} dibromide (PRC-50).

In an even more particular embodiment, the compound for use according to the first aspect of the invention is selected from the group consisting of:
1,1'-(decane-1,10-diyl)*bis*{4-[(*E*)-4-(diethylamino)styryl]-3-methylpyridin-1-ium} dibromide (AID-X-2020);
1,1'-(decane-1,10-diyl)*bis*{3-methyl-4-[(*E*)-4-morpholinostyryl]pyridin-1-ium} dibromide (EMA377);
1,1'-(undecane-1,11-diyl)*bis*{4-[(*E*)-4-(diethylamino)styryl]-3-methylpyridin-1-ium} dibromide (PRC-14);
1,1'-(dodecane-1,12-diyl)*bis*{4-[(*E*)-4-(diethylamino)styryl]-3-methylpyridin-1-ium} dibromide (PRC-15); and
1,1'-(nonane-1,9-diyl)*bis*{4-[(*E*)-4-(diethylamino)styryl]-3-methylpyridin-1-ium} dibromide (PRC-25).

**Table 1. Chemical structures of compounds AID-X-2020, EMA357, EMA359, EMA366, EMA368, EMA377, PRC-5, PRC-8, PRC-14, PRC-15, PRC-20, PRC-25, PRC-28, PRC-29, PRC-31, PRC-39, PRC-42, PRC-50, and PRC-69.**

| **Compound** | **Chemical structure** |
|---|---|
| **AID-X-2020** | 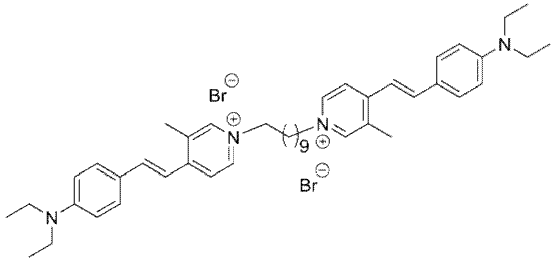 1,1'-(decane-1,10-diyl)*bis*{4-[(*E*)-4-(diethylamino)styryl]-3-methylpyridin-1-ium} dibromide |
| **EMA357** | 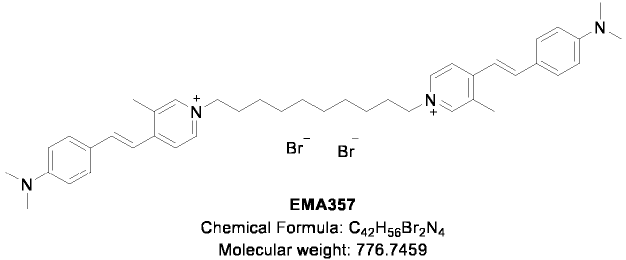 1,1'-(decane-1,10-diyl)*bis*{4-[(*E*)-4-(dimethylamino)styryl]-3-methylpyridin-1-ium} dibromide |
| **EMA359** | 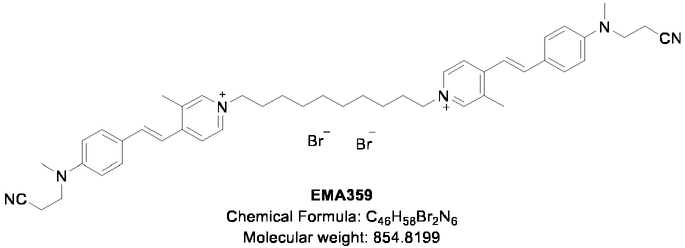 1,1'-(decane-1,10-diyl)*bis*{4-{(*E*)-4-[(2-cyanoethyl)methylamino]styryl}-3-methylpyridin-1-ium} dibromide |
| **EMA366** | 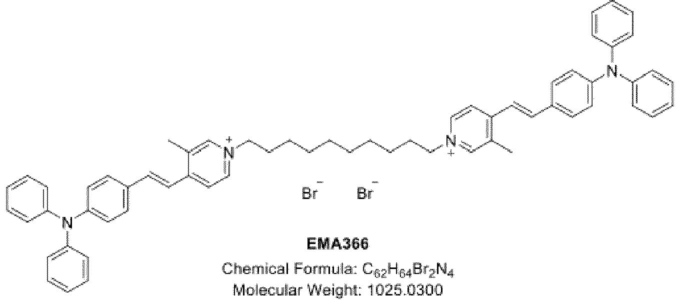 1,1'-(decane-1,10-diyl)*bis*{4-[(*E*)-4-(diphenylamino)styryl]-3-methylpyridin-1-ium} dibromide |
| **EMA368** | 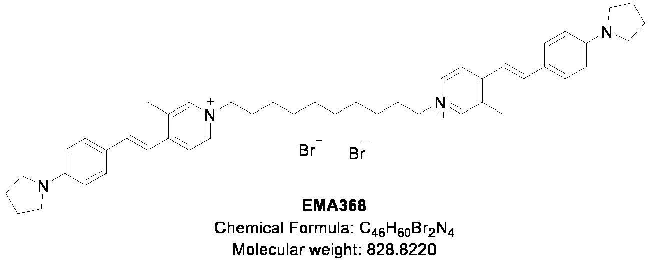 1,1'-(decane-1,10-diyl)*bis*{3-methyl-4-[(*E*)-4-(pyrrolidin-1-yl)styryl]pyridin-1-ium} dibromide |
| **EMA377** | 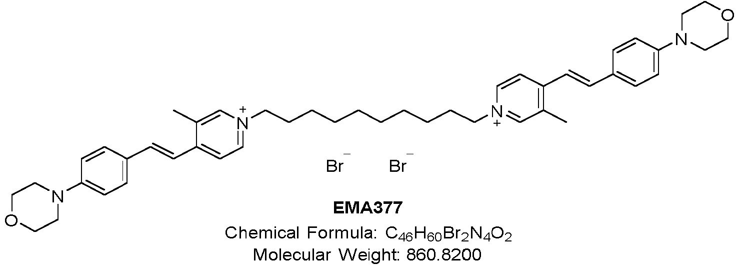 1,1'-(decane-1,10-diyl)*bis*{3-methyl-4-[(*E*)-4-morpholinostyryl]pyridin-1-ium} dibromide |
| **PRC-5** | 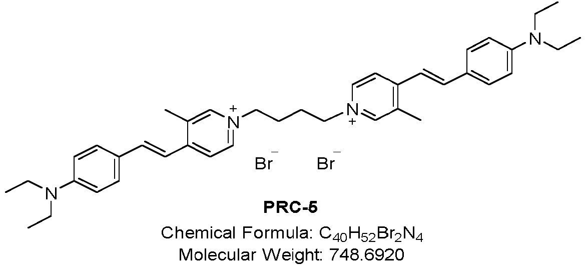 1,1'-(butane-1,4-diyl)*bis*{4-[(*E*)-4-(diethylamino)styryl]-3-methylpyridin-1-ium} dibromide |
| **PRC-8** | 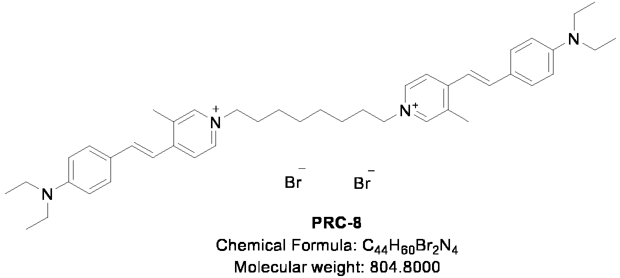 1,1'-(octane-1,8-diyl)*bis*{4-[(*E*)-4-(diethylamino)styryl]-3-methylpyridin-1-ium} dibromide |
| **PRC-14** | 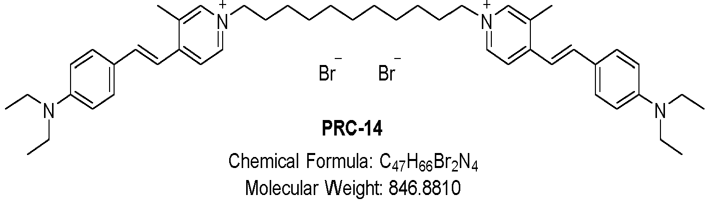 1,1'-(undecane-1,11-diyl)*bis*{4-[(*E*)-4-(diethylamino)styryl]-3-methylpyridin-1-ium} dibromide |
| **PRC-15** | 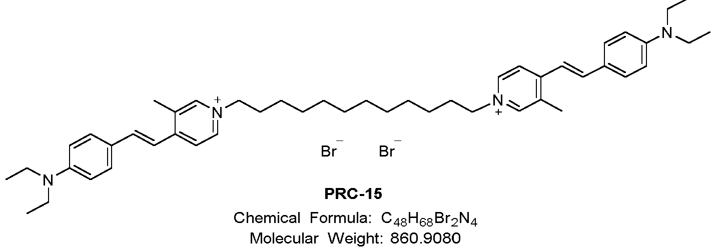 1,1'-(dodecane-1,12-diyl)*bis*{4-[(*E*)-4-(diethylamino)styryl]-3-methylpyridin-1-ium} dibromide |
| **PRC-20** | 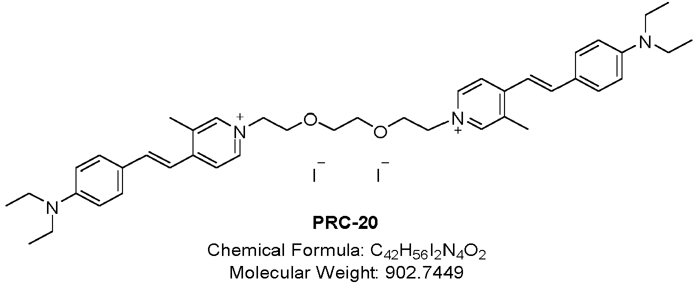 1,1'-(3,6-dioxaoctane-1,8-diyl)*bis*{4-[(*E*)-4-(diethylamino)styryl]-3-methylpyridin-1-ium} diiodide |
| **PRC-25** | 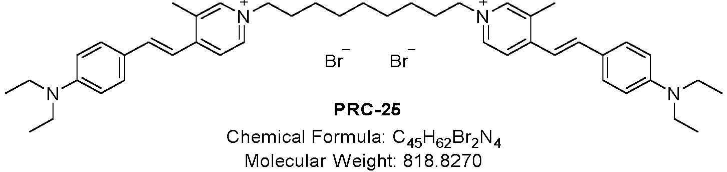 1,1 '-(nonane-1,9-diyl)*bis*{4-[(*E*)-4-(diethylamino)styryl]-3-methylpyridin-1-ium} dibromide |
| **PRC-28** | 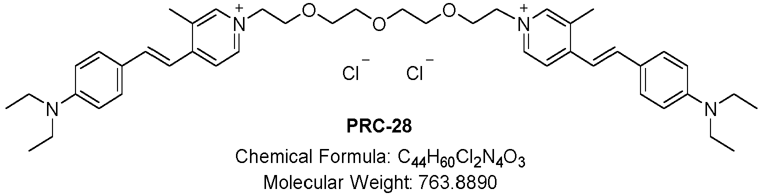 1,1'-(3,6,9-trioxaundecane-1,11-diyl)*bis*{4-[(*E*)-4-(diethylamino)styryl]-3-methylpyridin-1-ium} dichloride |
| **PRC-29** | 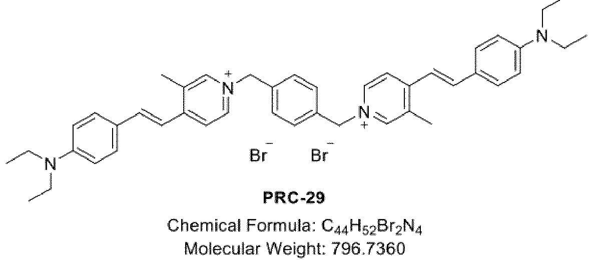 1,1'-[1,4-phenylenebis(methylene)]*bis*{4-[(*E*)-4-(diethylamino)styryl]-3-methylpyridin-1-ium} dibromide |
| **PRC-31** | 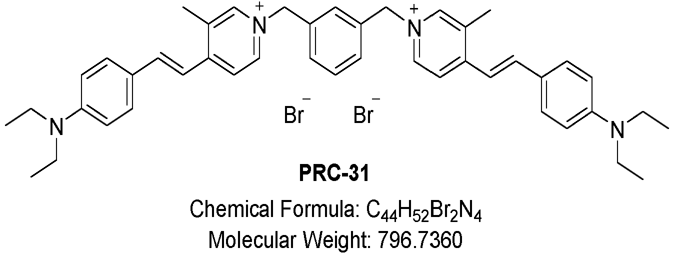 1,1'-[1,3-phenylenebis(methylene)]*bis*{4-[(*E*)-4-(diethylamino)styryl]-3-methylpyridin-1-ium} dibromide |
| **PRC-39** | 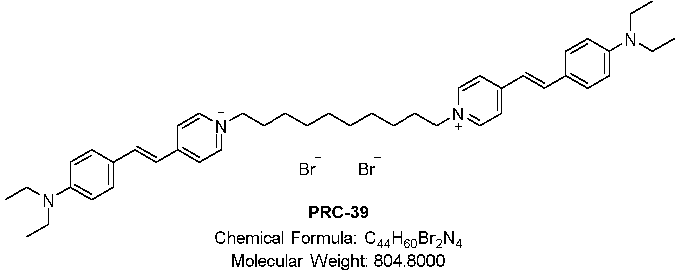 1,1'-(decane-1,10-diyl)*bis*{4-[(*E*)-4-(diethylamino)styryl]pyridin-1-ium} dibromide |
| **PRC-42** | 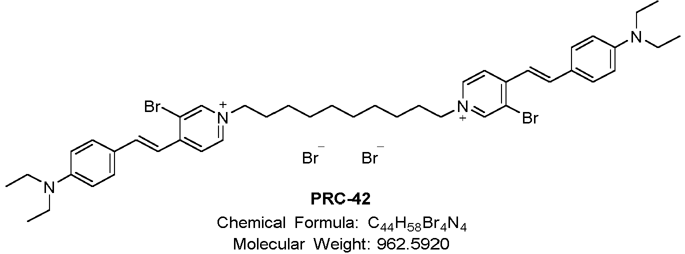 1,1'-(decane-1,10-diyl)*bis*{3-bromo-4-[(*E*)-4-(diethylamino)styryl]pyridin-1-ium} dibromide |
| **PRC-50** | 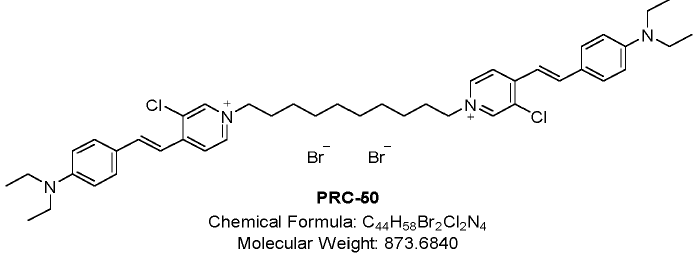 1,1'-(decane-1,10-diyl)*bis*{3-chloro-4-[(*E*)-4-(diethylamino)styryl]pyridin-1-ium} dibromide |
| **PRC-69** | 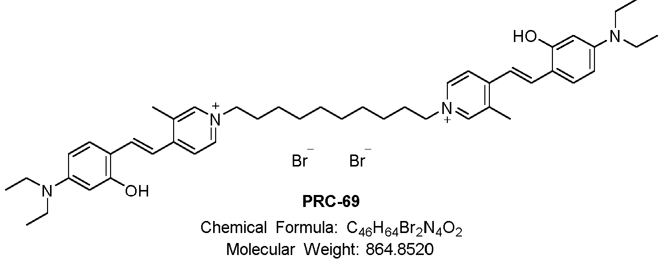 1,1'-(decane-1,10-diyl)*bis*{4-[(*E*)-4-(diethylamino)-2-hydroxystyryl]-3-methylpyridin-1-ium} dibromide |

In a particular embodiment, the compound for use according to the first aspect of the invention is of formula (VI) or a pharmaceutically acceptable salt thereof (also referred herein as AID-X-2020).

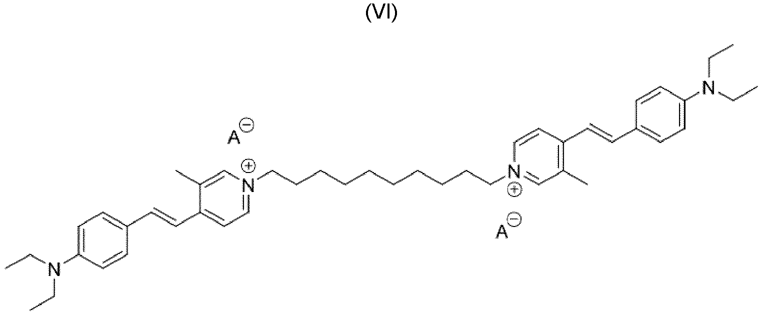

In a more particular embodiment, the compound for use is of formula (VI), wherein the anion (A) is bromide.

As discussed, within the compounds encompassed by formula (I), novel aminostyrylpyridinium compounds are provided (fourth aspect of the invention).

In some embodiments, the compound is according to the fourth aspect of the invention, wherein:
L is a diradical selected from the group consisting of tetramethylene, pentamethylene, hexamethylene, heptamethylene, octamethylene, nonamethylene, decamethylene, undecamethylene, dodecamethylene, 3-oxapentamethylene, 3,6-dioxaoctamethylene, 3,6,9-trioxaundecamethylene, 1,2-phenylene*bis*(methylene), 1,3-phenylene*bis*(methylene), and 1,4-phenylene*bis*(methylene);
R₁, R₂, R₇, and R₈ are radicals independently selected from the group consisting of hydrogen, (C₁-C₂)-alkyl, cyano-(C₁-C₂)-alkyl, and phenyl or alternatively, when taken in combination, R₁-N-R₂ and R₇-N-R₈ are independently selected from the group consisting of pyrrolidine piperidine, azepine, piperazine, 4-methylpiperazine, and morpholine ring;
R₃ and R₆ are hydrogen; and
R₄ and R₅ are radicals independently selected from the group consisting of hydrogen, methyl, chloro, bromo, fluoro, and iodo;
with the proviso that when R₁, R₂, R₃, R₄, R₅, R₆, R₇ and R₈ are hydrogen, L is not 1,4-phenylene*bis*(methylene);
with the proviso that when R₁, R₂, R₇ and R₈ are methyl and R₃, R₄, R₅ and R₆ are hydrogen, L is not tetramethylene, pentamethylene, hexamethylene, decamethylene, dodecamethylene, 3,6-dioxaoctamethylene, 1,2-phenylene*bis*(methylene), 1,3-phenylene*bis*(methylene) or 1,4-phenylene*bis*(methylene);
with the proviso that when R₁, R₂, R₇ and R₈ are ethyl and R₃, R₄, R₅ and R₆ are hydrogen, L is not tetramethylene, 3-oxapentamethylene, 1,2-phenylene*bis*(methylene) or 1,4-phenylene*bis*(methylene);
with the proviso that when R₁, R₂, R₇ and R₈ are phenyl and R₃, R₄, R₅ and R₆ are hydrogen, L is not tetramethylene, pentamethylene, hexamethylene, octamethylene, decamethylene, dodecamethylene, 1,2-phenylene*bis*(methylene), 1,3-phenylene*bis*(methylene) or 1,4-phenylene*bis*(methylene);
with the proviso that when R₁ and R₇ are methyl, R₂ and R₈ are NC-CH₂- and R₃, R₄, R₅ and R₆ are hydrogen, L is not 1,2-phenylene*bis*(methylene) or 1,4-phenylene*bis*(methylene);
with the proviso that when R₁, R₂, R₃, R₆, R₇ and R₈ are methyl and R₄ and R₅ are hydrogen, L is not 1,4-phenylene*bis*(methylene);
with the proviso that when R₁, R₂, R₄, R₅, R₇ and R₈ are methyl and R₃ and R₆ are hydrogen, L is not tetramethylene or decamethylene;
with the proviso that when R₁, R₂, R₇ and R₈ are ethyl, R₃ and R₆ are hydrogen and R₄ and R₅ are methyl, L is not decamethylene;
with the proviso that when R₁-N-R₂ and R₇-N-R₈ are pyrrolidino and R₃, R₄, R₅ and R₆ are hydrogen, L is not 1,4-phenylene*bis*(methylene); and
with the proviso that when R₁-N-R₂ and R₇-N-R₈ are piperidino and R₃, R₄, R₅ and R₆ are hydrogen, L is not 1,2-phenylene*bis*(methylene), 1,3-phenylene*bis*(methylene) or 1,4-phenylene*bis*(methylene).

In a particular embodiment, the compound is according to the fourth aspect, wherein:
L is a diradical selected from the group consisting of tetramethylene, octamethylene, nonamethylene, decamethylene, undecamethylene, dodecamethylene, 3,6-dioxaoctamethylene, 3,6,9-trioxaundecamethylene, 1,3-phenylene*bis*(methylene), and 1,4-phenylene*bis*(methylene);
R₁, R₂, R₇, and R₈ are radicals independently selected from the group consisting of (C₁-C₂)-alkyl, cyano-(C₂)-alkyl, and phenyl or alternatively, when taken in combination, R₁-N-R₂ and R₇-N-R₈ are independently selected from the group consisting of pyrrolidine and morpholine ring;
R₃ and R₆ are hydrogen; and
R₄ and R₅ are radicals independently selected from the group consisting of hydrogen, methyl, chloro, and bromo;
with the proviso that when R₁, R₂, R₃, R₄, R₅, R₆, R₇ and R₈ are hydrogen, L is not 1,4-phenylene*bis*(methylene);
with the proviso that when R₁, R₂, R₇ and R₈ are methyl and R₃, R₄, R₅ and R₆ are hydrogen, L is not tetramethylene, decamethylene, dodecamethylene, 3,6-dioxaoctamethylene, 1,3-phenylene*bis*(methylene) or 1,4-phenylene*bis*(methylene);
with the proviso that when R₁, R₂, R₇ and R₈ are ethyl and R₃, R₄, R₅ and R₆ are hydrogen, L is not tetramethylene or 1,4-phenylene*bis*(methylene);
with the proviso that when R₁, R₂, R₇ and R₈ are phenyl and R₃, R₄, R₅ and R₆ are hydrogen, L is not tetramethylene, octamethylene, decamethylene, dodecamethylene, 1,3-phenylene*bis*(methylene) or 1,4-phenylene*bis*(methylene);
with the proviso that when R₁, R₂, R₃, R₆, R₇ and R₈ are methyl and R₄ and R₅ are hydrogen, L is not 1,4-phenylene*bis*(methylene);
with the proviso that when R₁, R₂, R₄, R₅, R₇ and R₈ are methyl and R₃ and R₆ are hydrogen, L is not tetramethylene or decamethylene;
with the proviso that when R₁, R₂, R₇ and R₈ are ethyl, R₃ and R₆ are hydrogen and R₄ and R₅ are methyl, L is not decamethylene; and
with the proviso that when R₁-N-R₂ and R₇-N-R₈ are pyrrolidino and R₃, R₄, R₅ and R₆ are hydrogen, L is not 1,4-phenylene*bis*(methylene).

In some embodiments, the compound is as described in the fourth aspect of the invention, wherein R₁ = R₇, R₂ = R₈, R₃ = R₆, and/or R₄ = R₃ or alternatively, when used in combination, R₁-N-R₂ = R₇-N-R₈, R₃ = R₆, and/or R₄ = R₅. In particular embodiments, the compound is as described in the fourth aspect of the invention, wherein R₁ = R₇, R₂ = R₈, R₃ = R₆, and R₄ = R₃ or alternatively, when used in combination, R₁-N-R₂ = R₇-N-R₈, R₃ = R₆, and R₄ = R₅. Accordingly, in a particular embodiment, the compound as described in the fourth aspect of the invention is a homodimer.

In some embodiments, the compound is according to the fourth aspect of the invention, wherein
R₁, R₇ (R₁ = R₇) and R₂, R₈ (R₂ = R₈) are radicals independently selected from the group consisting of hydrogen, methyl, ethyl, phenyl, 2-hydroxyethyl, 2-cyanoethyl, and 2-(acetyloxy)ethyl, or alternatively, when taken in combination, R₁-N-R₂ = R₇-N-R₈ is selected from the group consisting of pyrrolidine, piperidine, piperazine and morpholine ring; and/or
R₃, R₆ (R₃ = R₆) are radicals selected from the group consisting of hydrogen, methyl, nitro, amino, acetamido, methanesulfonamido, benzenesulfonamido, and *p*-toluenesulfonamido; and/or
R₄, R₅ (R₄ = R₅) are radicals selected from the group consisting of hydrogen, methyl, chloro, bromo, fluoro, iodo, amino, acetamido, methanesulfonamido, benzenesulfonamido, *p-*toluenesulfonamido, aminomethyl, acetamidomethyl, methanesulfonamidomethyl, benzenesulfon-amidomethyl, and *p*-toluenesulfonamidomethyl.

Alternatively, the aminostyrylpyridinium moieties can be substituted at position 2 of the phenyl ring (R₃ and R₆) with a hydroxy group, a (C₁-C₄)-alkoxy group or a (C₁-C₄)-alkanoyloxy group. In a particular embodiment, the aminostyrylpyridinium moieties is substituted at position 2 of the phenyl ring with a hydroxy group. More particularly, the compound is 1,1'-(decane-1,10-diyl)*bis*{4-[(*E*)-4-(diethylamino)-2-hydroxystyryl]-3-methylpyridin-1-ium} dibromide (PRC-69).

In particular embodiments, the compound is according to the fourth aspect of the invention, wherein
R₁, R₇ (R₁ = R₇) and R₂, R₈ (R₂ = R₈) are radicals independently selected from the group consisting of hydrogen, methyl, ethyl, phenyl, 2-hydroxyethyl, 2-cyanoethyl, and 2-(acetyloxy)ethyl, or alternatively, when taken in combination, R₁-N-R₂ = R₇-N-R₈ is selected from the group consisting of pyrrolidine, piperidine, piperazine and morpholine ring;
R₃, R₆ (R₃ = R₆) are radicals selected from the group consisting of hydrogen, methyl, nitro, amino, acetamido, methanesulfonamido, benzenesulfonamido, and p-toluenesulfonamido; and
R₄, R₅ (R₄ = R₅) are radicals selected from the group consisting of hydrogen, methyl, chloro, bromo, fluoro, iodo, amino, acetamido, methanesulfonamido, benzenesulfonamido, *p-*toluenesulfonamido, aminomethyl, acetamidomethyl, methanesulfonamidomethyl, benzenesulfon-amidomethyl, and *p*-toluenesulfonamidomethyl.

Particularly, the compound according to the fourth aspect of the invention is selected from the group consisting of:
1,1'-(decane-1,10-diyl)*bis*{3-methyl-4-[(*E*)-4-(pyrrolidin-1-yl)styryl]pyridin-1-ium} dibromide (EMA368);
1,1'-(decane-1,10-diyl)*bis*{3-methyl-4-[(*E*)-4-(piperidin-1-yl)styryl]pyridin-1-ium} dibromide;
1,1'-(decane-1,10-diyl)*bis*{3-methyl-4-[(*E*)-4-morpholinostyryl]pyridin-1-ium} dibromide (EMA377);
1,1'-(decane-1,10-diyl)*bis*{3-methyl-4-[(*E*)-4-(piperazin-1-yl)styryl]pyridin-1-ium} dibromide;
1,1'-(decane-1,10-diyl)*bis*{4-[(*E*)-4-(diphenylamino)styryl]-3-methylpyridin-1-ium} dibromide (EMA366);
1,1'-(decane-1,10-diyl)*bis*{4-[(*E*)-4-(dimethylamino)-2-nitrostyryl]-3-methylpyridin-1-ium} dibromide;
1,1'-(decane-1,10-diyl)*bis*{4-{(*E*)-4-[(2-cyanoethyl)methylamino]styryl}-3-methylpyridin-1-ium} dibromide (EMA359);
1,1'-(decane-1,10-diyl)*bis*{4-{(*E*)-4-[bis(2-acetoxyethyl)amino]styryl}-3-methylpyridin-1-ium} dibromide;
1,1'-(decane-1,10-diyl)*bis*{4-{(*E*)-4-[bis(2-hydroxyethyl)amino]styryl}-3-methylpyridin-1-ium} dibromide;
1,1'-(ethane-1,2-diyl)*bis*{4-[(*E*)-4-(diethylamino)styryl]-3-methylpyridin-1-ium} dibromide;
1,1'-(propane-1,3-diyl)*bis*{4-[(*E*)-4-(diethylamino)styryl]-3-methylpyridin-1-ium} dibromide;
1,1'-(butane-1,4-diyl)*bis*{4-[(*E*)-4-(diethylamino)styryl]-3-methylpyridin-1-ium} dibromide (PRC-5);
1,1'-(pentane-1,5-diyl)*bis*{4-[(*E*)-4-(diethylamino)styryl]-3-methylpyridin-1-ium} dibromide;
1,1'-(hexane-1,6-diyl)*bis*{4-[(*E*)-4-(diethylamino)styryl]-3-methylpyridin-1-ium} dibromide;
1,1'-(heptane-1,7-diyl)*bis*{4-[(*E*)-4-(diethylamino)styryl]-3-methylpyridin-1-ium} dibromide;
1,1'-(octane-1,8-diyl)*bis*{4-[(*E*)-4-(diethylamino)styryl]-3-methylpyridin-1-ium} dibromide (PRC-8);
1,1'-(nonane-1,9-diyl)*bis*{4-[(*E*)-4-(diethylamino)styryl]-3-methylpyridin-1-ium} dibromide (PRC-25);
1,1'-(undecane-1,11-diyl)*bis*{4-[(*E*)-4-(diethylamino)styryl]-3-methylpyridin-1-ium} dibromide (PRC-14);
1,1'-(dodecane-1,12-diyl)*bis*{4-[(*E*)-4-(diethylamino)styryl]-3-methylpyridin-1-ium} dibromide (PRC-15);
1,1'-(3-oxapentane-1,5-diyl)*bis*{4-[(*E*)-4-(diethylamino)styryl]-3-methylpyridin-1-ium} dichloride;
1,1'-(3,6-dioxaoctane-1,8-diyl)*bis*{4-[(*E*)-4-(diethylamino)styryl]-3-methylpyridin-1-ium} diiodide (PRC-20);
1,1'-(3,6,9-trioxaundecane-1,11-diyl)*bis*{4-[(*E*)-4-(diethylamino)styryl]-3-methylpyridin-1-ium} dichloride (PRC-28);
1,1'-[1,2-phenylenebis(methylene)]*bis*{4-[(*E*)-4-(diethylamino)styryl]-3-methylpyridin-1-ium} dibromide;
1,1'-[1,3-phenylenebis(methylene)]*bis*{4-[(*E*)-4-(diethylamino)styryl]-3-methylpyridin-1-ium} dibromide (PRC-31);
1,1'-[1,4-phenylenebis(methylene)]*bis*{4-[(*E*)-4-(diethylamino)styryl]-3-methylpyridin-1-ium} dibromide (PRC-29);
1,1'-(decane-1,10-diyl)*bis*{4-[(*E*)-4-(diethylamino)styryl]pyridin-1-ium} dibromide (PRC-39);
1,1'-(decane-1,10-diyl)*bis*{3-bromo-4-[(*E*)-4-(diethylamino)styryl]pyridin-1-ium} dibromide (PRC-42);
1,1'-(decane-1,10-diyl)*bis*{3-chloro-4-[(*E*)-4-(diethylamino)styryl]pyridin-1-ium} dibromide (PRC-50); and 1,1'-(decane-1,10-diyl)*bis*{3-amino-4-[(*E*)-4-(diethylamino)styryl]pyridin-1-ium) dibromide.

In a more particular embodiment, the compound according to the fourth aspect of the invention is selected from the group consisting of:
1,1'-(decane-1,10-diyl)*bis*{3-methyl-4-[(*E*)-4-(pyrrolidin-1-yl)styryl]pyridin-1-ium} dibromide (EMA368);
1,1'-(decane-1,10-diyl)*bis*{3-methyl-4-[(*E*)-4-morpholinostyryl]pyridin-1-ium} dibromide (EMA377);
1,1'-(decane-1,10-diyl)*bis*{4-[(*E*)-4-(diphenylamino)styryl]-3-methylpyridin-1-ium} dibromide (EMA366);
1,1'-(decane-1,10-diyl)*bis*{4-{(*E*)-4-[(2-cyanoethyl)methylamino]styryl}-3-methylpyridin-1-ium} dibromide (EMA359);
1,1'-(butane-1,4-diyl)*bis*{4-[(*E*)-4-(diethylamino)styryl]-3-methylpyridin-1-ium} dibromide (PRC-5);
1,1'-(octane-1,8-diyl)*bis*{4-[(*E*)-4-(diethylamino)styryl]-3-methylpyridin-1-ium} dibromide (PRC-8);
1,1'-(nonane-1,9-diyl)*bis*{4-[(*E*)-4-(diethylamino)styryl]-3-methylpyridin-1-ium} dibromide (PRC-25);
1,1'-(undecane-1,11-diyl)*bis*{4-[(*E*)-4-(diethylamino)styryl]-3-methylpyridin-1-ium} dibromide (PRC-14);
1,1'-(dodecane-1,12-diyl)*bis*{4-[(*E*)-4-(diethylamino)styryl]-3-methylpyridin-1-ium} dibromide (PRC-15);
1,1'-(3,6-dioxaoctane-1,8-diyl)*bis*{4-[(*E*)-4-(diethylamino)styryl]-3-methylpyridin-1-ium} diiodide (PRC-20);
1,1'-(3,6,9-trioxaundecane-1,11-diyl)*bis*{4-[(*E*)-4-(diethylamino)styryl]-3-methylpyridin-1-ium} dichloride (PRC-28);
1,1'-[1,3-phenylenebis(methylene)]*bis*{4-[(*E*)-4-(diethylamino)styryl]-3-methylpyridin-1-ium} dibromide (PRC-31);
1,1'-[1,4-phenylenebis(methylene)]*bis*{4-[(*E*)-4-(diethylamino)styryl]-3-methylpyridin-1-ium} dibromide (PRC-29);
1,1'-(decane-1,10-diyl)*bis*{4-[(*E*)-4-(diethylamino)styryl]pyridin-1-ium} dibromide (PRC-39);
1,1'-(decane-1,10-diyl)*bis*{3-bromo-4-[(*E*)-4-(diethylamino)styryl]pyridin-1-ium} dibromide (PRC-42); and 1,1'-(decane-1,10-diyl)*bis*{3-chloro-4-[(*E*)-4-(diethylamino)styryl]pyridin-1-ium} dibromide (PRC-50).

In an even more particular embodiment, the compound according to the fourth aspect of the invention is selected from the group consisting of:
1,1'-(decane-1,10-diyl)*bis*{4-{(*E*)-4-[(2-cyanoethyl)methylamino]styryl}-3-methylpyridin-1-ium} dibromide (EMA359);
1,1'-(decane-1,10-diyl)*bis*{3-methyl-4-[(*E*)-4-morpholinostyryl]pyridin-1-ium} dibromide (EMA377);
1,1'-(octane-1,8-diyl)*bis*{4-[(*E*)-4-(diethylamino)styryl]-3-methylpyridin-1-ium} dibromide (PRC-8);
1,1'-(undecane-1,11-diyl)*bis*{4-[(*E*)-4-(diethylamino)styryl]-3-methylpyridin-1-ium} dibromide (PRC-14);
1,1'-(dodecane-1,12-diyl)*bis*{4-[(*E*)-4-(diethylamino)styryl]-3-methylpyridin-1-ium} dibromide (PRC-15);
1,1'-(nonane-1,9-diyl)*bis*{4-[(*E*)-4-(diethylamino)styryl]-3-methylpyridin-1-ium} dibromide (PRC-25);
1,1'-(decane-1,10-diyl)*bis*{4-[(*E*)-4-(diethylamino)styryl]pyridin-1-ium} dibromide (PRC-39);
1,1'-(decane-1,10-diyl)*bis*{3-bromo-4-[(*E*)-4-(diethylamino)styryl]pyridin-1-ium} dibromide (PRC-42); and 1,1'-(decane-1,10-diyl)*bis*{3-chloro-4-[(*E*)-4-(diethylamino)styryl]pyridin-1-ium} dibromide (PRC-50).

In an even more particular embodiment, the compound according to the fourth aspect of the invention is selected from the group consisting of:
1,1'-(decane-1,10-diyl)*bis*{3-methyl-4-[(*E*)-4-morpholinostyryl]pyridin-1-ium} dibromide (EMA377);
1,1'-(undecane-1,11-diyl)*bis*{4-[(*E*)-4-(diethylamino)styryl]-3-methylpyridin-1-ium} dibromide (PRC-14);
1,1'-(dodecane-1,12-diyl)*bis*{4-[(*E*)-4-(diethylamino)styryl]-3-methylpyridin-1-ium} dibromide (PRC-15); and
1,1'-(nonane-1,9-diyl)*bis*{4-[(*E*)-4-(diethylamino)styryl]-3-methylpyridin-1-ium} dibromide (PRC-25).

Chemical structures of the above-mentioned compounds are disclosed in Table 1 presented above.

In some embodiments, the invention is also directed to combinations comprising at least one compound described herein and one or more other therapeutic agents. The compound and the other therapeutic agent can be formulated for a separate, sequential, concomitant administration or in admixture in a pharmaceutical composition.

In particular embodiments, the other therapeutic agent is an antimalarial drug. Non-limiting examples of antimalarial drugs are artemether, artesunate, dihydroartemisinin, lumefantrine, mefloquine, tafenoquine, amodiaquine, piperaquine, atovaquone, pyrimethamine, pyronaridine, artemisinin, chloroquine, doxycycline, or quinine.

In a particular embodiment, the composition comprises AID-X-2020 and one or more other therapeutic agents. In another particular embodiment, the composition comprises EMA357 and one or more other therapeutic agents. In another particular embodiment, the composition comprises EMA359 and one or more other therapeutic agents. In another particular embodiment, the composition comprises EMA366 and one or more other therapeutic agents. In another particular embodiment, the composition comprises EMA368 and one or more other therapeutic agents. In another particular embodiment, the composition comprises EMA377 and one or more other therapeutic agents. In another particular embodiment, the composition comprises PRC-5 and one or more other therapeutic agents. In another particular embodiment, the composition comprises PRC8 and one or more other therapeutic agents. In another particular embodiment, the composition comprises PRC-14 and one or more other therapeutic agents. In another particular embodiment, the composition comprises PRC-15 and one or more other therapeutic agents. In another particular embodiment, the composition comprises PRC-20 and one or more other therapeutic agents. In another particular embodiment, the composition comprises PRC-25 and one or more other therapeutic agents. In another particular embodiment, the composition comprises PRC-28 and one or more other therapeutic agents. In another particular embodiment, the composition comprises PRC-29 and one or more other therapeutic agents. In another particular embodiment, the composition comprises PRC-31 and one or more other therapeutic agents. In another particular embodiment, the composition comprises PRC-39 and one or more other therapeutic agents. In another particular embodiment, the composition comprises PRC-42 and one or more other therapeutic agents. In another particular embodiment, the composition comprises PRC-50 and one or more other therapeutic agents. Alternatively, the composition comprises PRC-69 and one or more other therapeutic agents.

### Medical uses of the compounds/compositions

### Use as a medicament

As described previously, the examples of the present invention provide evidence of the antimalarial activity of compounds described herein. Thus, the present invention described for the first time the positive effect of the compounds of the invention in the treatment, prevention and amelioration of a disease, symptoms, complications and/or sequelae thereof.

The invention also encompasses the compounds/compositions described herein for the treatment of a pathological condition or disease susceptible to amelioration by inhibition of protein aggregation comprising administering a therapeutically effective amount of at least one compound/composition described herein.

### Use in the treatment of malaria

The present invention further relates to a compound/composition described herein, for use in the treatment, prevention and/or amelioration of an infectious disease, symptoms, complications and/or sequelae thereof. In a particular embodiment, the present invention relates to a compound/composition described herein, for use in the treatment of an infectious disease.

In a particular embodiment, the infectious disease can be, but it is not limited to malaria. In a particular embodiment, the present invention relates to a compound/composition described herein, for use in the treatment of malaria.

The invention also encompasses a method of treatment, prevention and/or amelioration of an infectious disease (e.g., malaria), symptoms, complications and/or sequelae thereof comprising administering a therapeutically effective amount of a compound or composition of the invention.

The invention also encompasses a method of treatment, prevention and/or amelioration of an infectious disease (e.g., malaria), symptoms, complications and/or sequelae thereof by inhibiting protein aggregation comprising administering a therapeutically effective amount of a compound or composition of the invention.

The invention also encompasses a method of treatment, prevention and/or amelioration of malaria, symptoms, complications and/or sequelae thereof comprising administering a therapeutically effective amount of a compound, salt thereof or composition of the invention. In a particular embodiment, the invention encompasses a method of treatment of malaria comprising administering a therapeutically effective amount of a compound, salt thereof or composition of the invention.

In some embodiments, the administration of the compound/composition of the invention results in the treatment, prevention or amelioration of at least one symptom of malaria selected from the group consisting of: fever, cough chills, shaking, headache, muscle aches, muscle or joint pain, general discomfort, profuse sweating, fatigue, prostration, nausea, vomiting, diarrhea, abdominal pain, rapid breathing, rapid high rate, anemia, acidosis, convulsions, coma, bloody stools, renal failure, pulmonary edema, jaundice and/or low oxygen saturation in blood, particularly an oxygen saturation SaO₂ or SpO₂< 92%.

As known by the skilled person, SaO₂ is an alternative measure of oxygen saturation in blood, that refers to oxygen saturation measured in arteries and the later to oxygen saturation measured by pulse oximetry. SaO₂ measures equivalent to SpO₂ measures can be used indistinctly in this description.

In some embodiments, the administration of the compound/composition of the invention results in at least one outcome (i.e., effect) selected, but not limited to, from the group consisting of:
- reduction of the transmission of the parasite to mosquitoes;
- remission of malaria;
- reduction of the duration of symptoms of malaria;
- reduction of the severity of symptoms of malaria (e.g., reduction of ICU admission);
- reduction of *Plasmodium* (e.g., *Plasmodium falciparum)* load;
- delay of disease progression;
- improvement of blood oxygen saturation; and
- reduction of the duration of low oxygen saturation in blood (i.e., hypoxemia).

In some embodiments, the administration of the compound/composition of the invention to the subject can avoid hospitalization or reduce hospitalization time, avoid ICU admission (i.e., eliminate the need for ICU treatment), delay the need for ICU treatment, reduce (shorten) ICU treatment time, reduce patient mortality rate, increase patient survival rate, reduce patient's chance of death, increase patient's chance of survival, decrease patient's risk of death, reduce the severity of at least one symptom, reduce the severity of at least one complication, reduce the severity of at least one sequela, reduce the duration of at least one symptom, reduce the duration of at least one complication, reduce the duration of at least one sequela, reduce disease transmission, or any combination thereof. Accordingly, the present disclosure provides methods to avoid or reduce hospitalization, reduce (shorten) ICU treatment time, delay the need for ICU treatment, avoid ICU admission (i.e., eliminate the need for ICU treatment), reduce patient mortality rate, increase patient survival rate, reduce patient's chance of death, increase patient's chance of survival, decrease patient's risk of death, reduce the severity of at least one symptom, reduce the severity of at least one complication, reduce the severity of at least one sequela, reduce the duration of at least one symptom, reduce the duration of at least one complication, reduce the duration of at least one sequela, reduce disease transmission, or any combination thereof, in a subject and comprises administering the compound/composition to the subject.

### Use in the treatment of other diseases

As described previously, EXAMPLES 3-5 of the present invention provide evidence of the capacity of the compounds described herein to inhibit the aggregation of proteins (e.g., β-amyloid proteins). Further, the present invention provides evidence of the capacity of the compounds to also disassemble aggregative proteins (EXAMPLE 10). Therefore, evidence herein shows the potential of compounds and compositions of the present invention to have a positive effect in the treatment, prevention and amelioration of a disease, symptoms, complications and/or sequelae thereof, wherein the disease is characterized by protein aggregation.

Therefore, the present invention also relates to the compounds/compositions described herein for use in the treatment, prevention and/or amelioration of disease characterized by protein aggregation, symptoms, complications and/or sequelae thereof. Alternatively, the invention relates to the use of the compounds described herein for the manufacture of a formulation or medicament for treating a disease characterized by protein aggregation. The invention also encompasses a method of treatment of a disease characterized by protein aggregation, comprising administering at least one compound or composition described herein.

Proteinaceous aggregates such as amyloids and prions were first discovered in neurodegenerative diseases and were quickly attributed to a pathological state of the otherwise properly folded protein. Many pathologies are related to a failure of protein folding and the aggregation of partially folded intermediates, including Alzheimer's, Parkinson's and Huntington's diseases, amyotrophic lateral sclerosis, transmissible spongiform encephalopathies like Creutzfeldt-Jacob disease and scrapies, and spinocerebellar ataxia, to name just a few. The cytotoxicity of protein aggregation has been also shown at the root of the evolutionary tree, where susceptibility of bacteria to protein misfolding has been proposed as the target of future antibiotics.

Consequently, in one embodiment, the disease can be, but is not limited to a neurodegenerative disease or a bacterial infection. In a particular embodiment, the disease is a neurodegenerative disease. Particularly, the neurodegenerative disease is a conformational disease.

In a particular embodiment, the neurodegenerative disease can be, but is not limited to, Alzheimer's disease (AD), Parkinson's disease (PD), Huntington's disease (HD), amyotrophic lateral sclerosis (ALS), prion disease (PrD), Gehrig's disease, cystic fibrosis, Gaucher's disease, frontotem-poral dementia (FTD), corticobasal degeneration, progressive supranuclear palsy (PSP), chronic traumatic encephalopathy (CTE), multiple system atrophy (MSA), spinal muscular atrophy (SMA), dementia with Lewy bodies (DLB) and motor neurone diseases (MND).

In a particular embodiment, the prion disease can be, but is not limited to, Creutzfeldt-Jacob disease (CJD), variant Creutzfeldt-Jakob disease (vCJD), scrapie and spinocerebellar ataxia (SCA), Gerstmann-Straussler-Scheinker Syndrome, fatal familial insomnia, or Kuru.

### Products comprising the compounds/compositions

In some embodiments, the composition described herein is in a pharmaceutical form, such as a capsule, a powder, a suspension, a tablet, a topical cream, or an ointment.

The term "pharmaceutical form" is understood in its widest meaning, including any composition that comprises an active ingredient, in this case, a compound of formula (I) or the composition described herein together with at least a pharmaceutically (also referred as nutraceutically or veterinary) acceptable excipient. The term "pharmaceutical form" is not limited to medicaments but includes e.g., pharmaceutical compositions, nutraceutical compositions or veterinary compositions. A pharmaceutical form can adopt different names depending on the product regulatory approval route and also depending on the country.

A nutraceutical composition can also be named e.g., as food supplement or dietary supplement. A nutraceutical composition is understood as a preparation or product intended to supplement the diet, made from compounds usually used in foodstuffs, which provide nutrients or beneficial ingredients that are not usually ingested in the normal diet or may not be consumed in sufficient quantities. Nutraceutical compositions are usually sold "over the counter", i.e., without prescription.

In some embodiments, the composition described herein is formulated as pharmaceutical form in which the compounds described herein is the only active agent or is mixed with one or more other active agents and/or are mixed with pharmaceutically/nutraceutically/veterinary acceptable excipients. Particularly, the additional active agent or agents are other antimalarial agents which are not antagonistic to the compounds described herein forming the composition of the invention. Depending on the formulation, the additional antimalarial agent may be added alone or together with suitable carriers or ingredients.

The term "pharmaceutically/nutraceutical/veterinary acceptable" is art-recognized, and includes excipients, compounds, materials, compositions, carriers, vehicles and/or dosage forms which are, within the scope of sound medical judgment, suitable for use in contact with the tissues of a subject (e.g., human or animal) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation. Suitable carriers, excipients, etc. can be found in standard pharmaceutical/nutraceutical/veterinary texts.

Thus, some embodiments of the invention relate to a pharmaceutical composition, a nutraceutical composition, and a veterinary composition comprising a compound of the invention described herein together with at least a pharmaceutically/nutraceutically/veterinary acceptable excipient as described above. Excipients are selected, without limitation, from the group comprising: fillers/diluents/bulking agents, binders, antiadherents, disintegrants, coatings, anti-caking agents, antioxidants, lubricants, sweeteners, flavors, colors, or tensides.

In each case, the presentation of the composition will be adapted to the type of administration used. Thus, the composition may be presented in the form of solutions or any other form of clinically permissible administration and in a therapeutically effective amount. The pharmaceutical composition can be thus formulated into solid, semisolid or liquid preparations, such as tablets, capsules, powders (such as those derived from lyophilization (freeze-drying) or air-drying), granules, solutions, suppositories, gels or microspheres. In a particular embodiment, the composition is formulated for administration in liquid form or in solid form.

In a particular embodiment, the composition is in solid form such as tablets, lozenges, sweets, chewable tablets, chewing gums, capsules, sachets, powders, granules, coated particles or coated tablets, pills, troches, gastro-resistant tablets and capsules, dispersible strips and films. More particularly, the composition is in form of a capsule, a powder, a tablet, a pill, lozenges, sachets, sticks, or granules. In a more particular embodiment, the composition is in form of a pill, a tablet or a capsule.

In another embodiment, the composition is in liquid form such as oral solutions, drops, suspensions (e.g., oil), emulsions and syrups.

In some embodiments, the composition is in the form of an oily suspension to be administered alone or mixed with a liquid. This form is particularly useful for children and babies. The oily suspension comprises at least one edible oil such as olive oil, maize oil, soybean oil, linseed oil, sunflower oil or rice oil. The oil is present in a quantity of e.g., at least 70 % weight/weight. In a particular embodiment, the oily suspension also comprises at least one excipient which is an emulsifier, stabilizer or anti-caking agent, in an amount of 0.1-15 % w/w. Suitable agents are silicon dioxide, silica gel, colloidal silica, precipitated silica, talc, magnesium silicate, lecithin, pectin, starch, modified starches, konjac gum, xanthan gum, gellan gum, carrageenan, sodium alginate, mono- or diglycerides of fatty acids such as glycerol monostearate or glycerol monooleate and citric acid esters of mono- or diglycerides. Particularly, compositions comprise compounds of formula (I) wherein the counteranions are fatty acids.

Oral tablets are often crushed to help make them easier for children to swallow. Consequently, in another embodiment, the composition is in the form of a syrup, granules, powders or tablets which can be dissolved in water. Particularly, the composition further comprises a flavoring agent.

In a particular embodiment, the compounds/compositions of the invention are formulated for oral administration. Oral administration is preferred for non-complicated malaria (most of the cases in residents of endemic areas) and as prevention (e.g., for travelers to endemic areas).

In other embodiments, e.g., in cases of severe malaria, the compounds/compositions of the invention can be in the form of an injectable for e.g., intravenous or intraperitoneal administration.

The composition of the invention targets also the mosquito stages of the malaria parasite and inhibits the maturation of some of them, thus it can also be directly delivered to mosquitoes to block in the insect the life cycle of *Plasmodium.* This strategy should bypass expensive clinical trials and would therefore lead to a much less expensive formulation.

Therefore, in another embodiment, the composition is administered directly against mosquitoes. Particularly, the composition is administered against female *Anopheles* mosquitoes. In another embodiment, the composition is administered in combination with mosquito attractants. More particularly, the composition is in form of dispensers or sprays, which may be delivered in surfaces such as walls or bednets.

### Process for the preparation of compounds of formula (I)

The present invention also relates to a process for the preparation of compounds of formula (I) which comprises reacting 4-methylpyridines of formula (II) with alkylating reagents of formula (III), wherein X is a common leaving group, such as halide, acyloxy, sulfonate or sulfate, followed by reacting the resulting compound of formula (IV) with a 4-aminobenzaldehyde of formula (V), and, followed, if necessary by standard transformation of functional groups that are present in radicals R₁-R₈. It is to be understood by the skilled in the art that the reagents used in the process of preparation are selected according to the radicals of the compound of the fourth aspect of the invention intended to prepare.

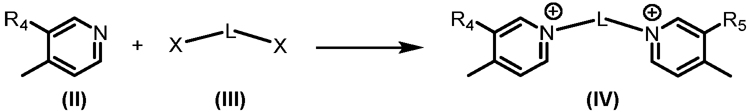

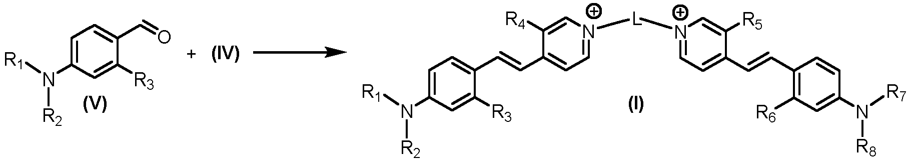

### EXAMPLES

### EXAMPLE 1: Inhibition effect of AID-X-2020 on P. falciparum in vitro

The inventors of the present invention performed a study of the antimalarial effect of AID-X-2020, compared to compounds known to have antimalarial activity and dyes in *in vitro* cultures of *P. falciparum.*

### 1.1 Materials and methods

### 1.1.1 General aspects of synthesis procedures

All reagents and solvents were obtained from commercial suppliers and used without further purification. Automatic flash column chromatography was performed on a CombiFlash Rf 150 (Teledyne Isco) with prepacked RediSep Rf silica gel cartridges. Melting points were determined in open capillary tubes with a MFB 595010M Gallenkamp melting point apparatus. IR spectra were run on a Perkin Elmer Spectrum RX I spectrophotometer. Absorption values are expressed as wave-numbers (cm⁻¹). 400 MHz ¹H NMR spectra and 500 MHz ¹H / 125 MHz ¹³C NMR spectra were recorded on a Varian Mercury 400 and a Bruker Avance Neo 500 MHz spectrometers, respectively, at the Centres Cientifics i Tecnològics of the University of Barcelona (CCiTUB). The chemical shifts are reported in ppm (δ scale) relative to solvent signals (DMSO-d₆ at 2.50 and 39.5 ppm in the¹H and ¹³C NMR spectra, respectively; CD₃OD at 3.31 ppm in the ¹H NMR spectra), and coupling constants are reported in Hertz (Hz). High resolution mass spectra were carried out at the CCiTUB with a LC/MSD TOF Agilent Technologies spectrometer.

### 1.1.2 Synthesis of 1,1'-(decane-1,10-diyl)bis{4-[(E)-4-(diethylamino)styryl]-3-methylpyridin-1-ium} dibromide (AID-X-2020)

A mixture of 1,10-dibromodecane (1.50 g, 5.00 mmol) and 3,4-dimethylpyridine (1.2 mL, 1.14 g, 10.7 mmol) was heated to 120 °C for 3 h. Then, isopropanol (5 mL) was added and the reaction mixture was stirred under reflux for 1 h. The mixture was allowed to cool down to room temperature, the resulting brown residue was washed with ice-cold Et₂O (2 × 40 mL) and the remaining brown sticky oil was dried *in vacuo,* taken up in MeOH (1 mL) and treated with cold Et₂O (2 × 40 mL), drawing off the liquids. After drying the residue *in vacuo,* 1,1'-(decane-1,10-diyl)bis(3,4-dimethylpyridin-1-ium) dibromide (2.48 g, 96%) was obtained as a brown oil that solidified on standing; mp: 69-71 °C; IR (ATR) v: 3443, 3396, 3027, 2988, 2921, 2851, 1635, 1512, 1483, 1471, 1391, 1224, 1143, 1031, 870, 838, 710, 597, 559 cm⁻¹; ¹H NMR (500 MHz, DMSO-d₆) *δ*: 1.20-1.32 (m, 12H), 1.89 (tt, *J = J'* = 7.5 Hz, 4H), 2.40 (s, 6H), 2.52 (s, 6H), 4.50 (t, *J =* 7.5 Hz, 4H), 7.95 (d, *J* = 6.0 Hz, 2H), 8.85 (dd, *J* = 6.0 Hz, *J' =* 1.5 Hz, 2H), 8.96 (br s, 2H); ¹³C NMR (125 MHz, DMSO-d₆) *δ*: 16.3 (2 CH₃), 19.6 (2 CH₃) , 25.4 (2 CH₂), 28.3 (2 CH₂), 28.7 (2 CH₂), 30.5 (2 CH₂), 59.7 (2 CH₂), 127.9 (2 CH), 137.6 (2 C), 141.5 (2 CH), 142.8 (2 CH), 157.6 (2 C); HRMS-ESI+ *m*/*z* calculated for [C₂₄H₃₈N₂]²⁺/2: 177.1512, found 177.1513.

A solution of 1,1'-(decane-1,10-diyl)bis(3,4-dimethylpyridin-1-ium) dibromide (514 mg, 1.00 mmol) and 4-(diethylamino)benzaldehyde (390 mg, 2.20 mmol) in n-butanol (5 mL) was treated with six drops of piperidine and the reaction mixture was stirred under reflux for 4 h, and then concentrated under reduced pressure. The resulting black oily residue was purified by automatic flash column chromatography (CH₂Cl₂ / 7N ammonia solution in MeOH 9:1), to provide 1,1'-(decane-1,10-diyl)bis{4-[(E)-4-(diethylamino)styryl]-3-methylpyridin-1-ium} dibromide (351 mg, 42%) as a red oil that solidified on standing; mp: 173-174 °C; IR (ATR) v: 3399, 2975, 2927, 2853, 1641, 1574, 1520, 1479, 1404, 1351, 1311, 1260, 1219, 1186, 1128, 1076, 1011, 958, 807, 572 cm⁻¹; ¹H NMR (500 MHz, DMSO-d₆) *δ*: 1.13 (t, *J =* 7.0 Hz, 12H), 1.21-1.31 (m, 12H), 1.87 (tt, *J = J' =* 7.5 Hz, 4H), 2.48 (s, 6H), 3.43 (q, *J* = 7.0 Hz, 8H), 4.37 (t, *J =* 7.5 Hz, 4H), 6.74 (d, *J* = 9.0 Hz, 4H), 7.08 (d, *J =* 16.0 Hz, 2H), 7.64 (d, *J* = 9.0 Hz, 4H), 7.88 (d, *J* = 16.0 Hz, 2H), 8.26 (d, *J* = 6.5 Hz, 2H), 8.66 (dd, J *=* 6.5 Hz, *J'* = 1.5 Hz, 2H), 8.73 (br s, 2H); ¹³C NMR (125 MHz, DMSO-d₆) *δ*: 12.5 (4 CH₃), 16.5 (2 CH₃), 25.5 (2 CH₂), 28.4 (2 CH₂), 28.7 (2 CH₂), 30.5 (2 CH₂), 43.9 (4 CH₂), 58.9 (2 CH₂), 111.3 (4 CH), 113.4 (2 CH), 119.8 (2 CH), 122.1 (2 C), 130.8 (4 CH), 132.9 (2 C), 140.5 (2 CH), 142.4 (2 CH), 143.3 (2 CH), 149.5 (2 C), 152.4 (2 C); HRMS-ESI+ m/z calculated for [C₄₆H₆₄N₄]²⁺/2: 336.2560, found: 336.2550.

### 1.1.3 P. falciparum growth inhibition assay

*P. falciparum* parasites of the strain 3D7 MRA-102 (BEI Resources, managed by ATCC) were 5% sorbitol-synchronized in order to obtain a culture enriched in ring stage parasites. After the synchronization process, a new culture at 1.5% parasitemia and 2% hematocrit was established and 150 µL aliquots of it were transferred to 96-well plates.

The characterization of AID-X-2020 in *P. falciparum in vitro* cultures was performed, and other dyes were included as controls: ThT and Congo Red. The required amounts of peptides, antimalarial compounds and dyes were added in each well at different concentrations and in triplicates. A positive growth control of untreated parasites and a negative growth control of parasites treated with a lethal dose of chloroquine (1 µM) were also included.

Parasites were grown for 48 h, a complete replication cycle, in standard culturing conditions (5% O₂, 5% CO₂, and 90% N₂ at 37 °C). After the incubation period, 3 µL of culture from each well were mixed with 197 µL of PBS containing 0.1 µM Syto 11 (Thermo Fisher Scientific Inc), to obtain a final concentration of ca. 1-10 × 10⁶ cells/ml.

Parasitemia was assessed by flow cytometry using a LSRFortessa flow cytometer (BD Biosciences, San Jose, CA, USA) set up with the 4 lasers, 20 parameters standard configuration. The single-cell population was selected on a forward-side scatter scattergram. Syto 11 fluorescence signal was detected by exciting samples at 488 nm and collecting the emission with a 530/30 nm bandpass filter.

Growth inhibition was calculated taking as reference values both the growth rate of the untreated culture and the growth rate of the culture treated with chloroquine. Growth inhibition data was transformed through sigmoidal fitting and used to determine the IC₅₀ values of compounds that actually inhibited parasite growth.

### 1.2 Results

The results obtained indicated that AID-X-2020 has a potent antimalarial activity *in vitro,* with an IC₅₀ of 90 ± 2 nM, comparable and even superior to other compounds previously described to have antimalarial activity (Table 2). Other protein aggregation dyes such as ThT and Congo Red did not exhibit significant antiplasmodial activity.

**Table 2. In vitro antimalarial activity of different compounds.**

| **Compound** | **Chemical family** | **IC₅₀ in 3D7 strain (µM) ± SEM** | **IC₅₀ in K1 strain (µM) ± SEM** | **Therapeutic index *in vitro**** |
|---|---|---|---|---|
| **Compounds with antimalarial activity** | | | | |
| HUP5ANTRA | aminoquinoline | 0.78 ± 1.86 | N/D | 97.7 |
| HUP7ANTRA | aminoquinoline | 7.53 ± 0.84 | N/D | 3.9 |
| HUP10ANTRA | aminoquinoline | 8.73 ± 1.61 | N/D | 9.7 |
| HUP7TH | aminoquinoline | 0.13 ± 0.01 | 0.47 ± 0.36 | 60.0 |
| HUP8TH | aminoquinoline | 0.21 ± 0.01 | 0.39 ± 0.14 | 23.3 |
| HUP9TH | aminoquinoline | 0.16 ± 0.02 | 0.35 ± 0.06 | 30.6 |
| HUPH10TH | aminoquinoline | 0.18 ± 0.05 | 3.50 ± 2.29 | 18.9 |
| HUPNTH | aminoquinoline | 0.31 ± 0.02 | 0.43 ± 0.22 | 11.0 |
| HUPNTCI | aminoquinoline | 0.15 ± 0.01 | 0.52 ± 0.13 | 42.0 |
| DP128 | aminoquinoline | 0.65 ± 0.14 | N/D | 75.4 |
| DONE3TCI | aminoquinoline | 0.08 ± 0.03 | 0.36 ± 0.07 | 157.5 |

| **Compounds previously known as dyes** | | | | |
|---|---|---|---|---|
| AID-X-2020 | bis(styrylpyridinium) salt | 0.09 ± 0.02 | N/D | 23.3 |
| ThT | benzothiazolium salt | 1.57 ± 0.23 | N/D | N/D |
| Congo Red | bis(naphthalenesulfonate) salt | >15 | N/D | N/D |

| | | | | |
|---|---|---|---|---|
| * 50% cytotoxic concentration (CC₅₀) / IC₅₀ in 3D7 strain. | | | | |

**Table 3. Chemical structures of compounds tested for in vitro antimalarial activity.**

| **Compound** | **Chemical structure** |
|---|---|
| **HUP5ANTRA,** n=5 **HUP7ANTRA,** n=7 **HUP10ANTRA,** n=10 | 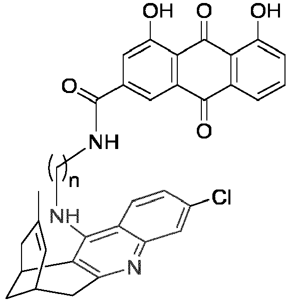 |
| **HUP7TH,** n=7, R¹=Cl, R²=H **HUP8TH,** n=8, R¹=Cl, R²=H **HUP9TH,** n=9, R¹=Cl, R²=H **HUPH10TH,** n=10, R¹=R²=H | 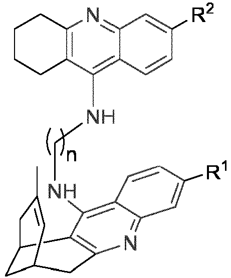 |
| **HUPNTH**, R²=H **HUPNTCI**, R²=Cl | 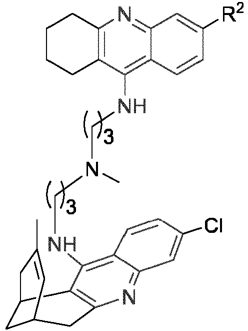 |
| **DP128** | 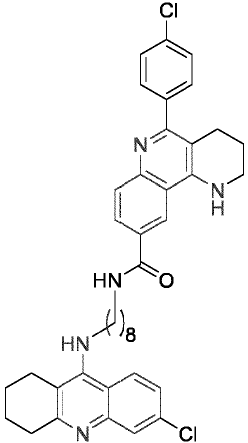 |
| **DONE3TCI** | 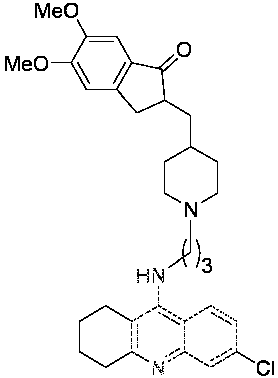 |
| **Congo Red** | 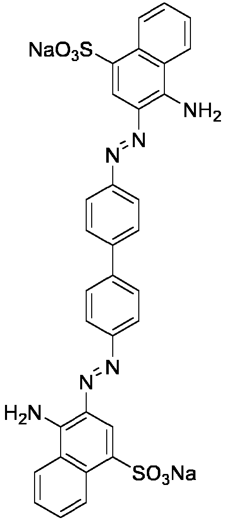 |
| **ThT** | 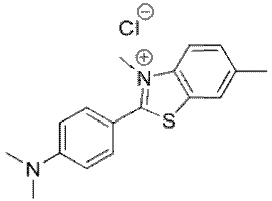 |
| **AID-X-2020** | 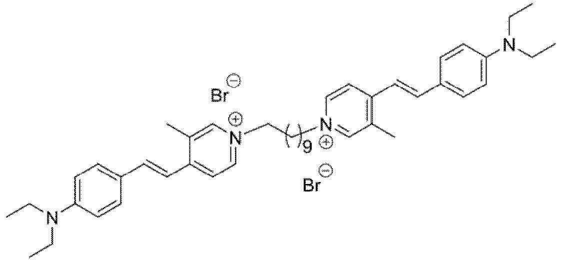 |

AID-X-2020 belongs to a chemical family where no antimalarial drugs have been described so far. Thus, an *in vitro* study on the inhibition effect of AID-X-2020 on each stage of the parasite was performed.

### EXAMPLE 2: Inhibition effect of AID-X-2020 on each stage of the parasite P. falciparum in vitro

The objective of this experiment was the evaluation of the inhibition effect of AID-X-2020 on each stage of the parasite: rings, early trophozoites, mature trophozoites and schizonts.

### 2.1 Materials and methods

For stage of growth inhibition analysis, *P*. *falciparum* cultures were synchronized at ring or trophozoite stages by repeated treatment with 5% sorbitol or 70% Percoll, respectively. Half of each culture remained untreated and the other half was treated with the IC₈₀ of AID-X-2020. At different time points, culture samples were stained with Giemsa and the number of rings, early and mature trophozoites and schizonts was counted by microscopic examination of at least 100 pRBCs for each sample. Pictures were taken with a Nikon Eclipse 50i microscope equipped with a DS-Fi1 camera (Nikon).

### 2.2 Results

When added to ring stages at its IC₈₀, AID-X-2020 arrested the life cycle of the pathogen at trophozoite stage (FIG. 1), whereas when the drug was delivered to cultures containing late *Plasmodium* forms, the parasites were able to complete their intraerythrocytic maturation and egress the pRBC, although their growth inside the new invaded RBCs became arrested at early trophozoite stage.

### EXAMPLE 3: Effect of AID-X-2020 on the in vitro aggregation of Aβ40

In order to determine the mechanism of action of AID-X-2020 on the inhibition of *P*. *falciparum* that was previously proved *in vitro,* the effect of AID-X-2020 on Aβ40 aggregation *in vitro* was first studied. Amyloid-β peptide fragment 1-40 (Aβ40) was used as a model of a highly aggregative peptide to evaluate the potential antiaggregatory effect of AID-X-2020. Firstly, an *in vitro* analysis of Aβ40 aggregation was performed by ThT fluorescence assay. Secondly, to dismiss the possibility that the decrease in ThT fluorescence was due to the presence of AID-X-2020, the Aβ40 samples treated with AID-X-2020 were examined by transmission electron microscopy (TEM).

### 3.1 Materials and methods

### 3.1.1 In vitro analysis of Aβ40 aggregation

For the *in vitro* analysis of Aβ peptide fragment 1-40 (Aβ40) aggregation, 1 mg of Aβ40 (GenScript) was dissolved in 500 µL of 1,1,1,3,3,3-hexafluoro-2-propanol (HFIP, Fluka) under vigorous stirring for 1 h and sonicated for 30 min in a sonication bath. Afterwards, the solution was stirred for 1 h and maintained at 4 °C for 30 min. Aliquots were prepared, HFIP was evaporated under a nitrogen stream for a few seconds and the peptide was stored at -20 °C. Prior to use, aliquots of Aβ40 were resuspended with DMSO, sonicated for 10 min, further diluted to 25 µM in PBS containing different concentrations of test compounds (0.1 µM AID-X-2020, 1 µM AID-X-2020 and 10 µM AID-X-2020), and incubated for 24 h at 37 °C and 1400 rpm. The final sample always contained less than 5% DMSO to avoid interference of this solvent on Aβ40 amyloid fibril formation. Finally, ThT treatment was performed as described above.

25 µM ThT was added to each sample and fluorescence was measured by exciting samples at 440 nm and recording the emission from 465 nm to 600 nm. A blank measurement of each sample was done before adding ThT.

### 3.1.2 Transmission electron microscopy (TEM)

For *in vitro* peptide aggregation analysis, 25 µM Aβ40 solution was prepared as explained above and incubated for 48 h at 37 °C and 1400 rpm in the presence of growing concentrations of AID-X-2020 (0, 0.1, 1 and 10 µM). A carbon-coated copper grid was deposited on top of a 50-µL drop of each solution for 30 min. Then, the excess liquid was removed with filter paper and the grid was placed on top of a water drop for 30 s and finally negatively stained for 2 min with 20 µL of 2% uranyl acetate. Samples were observed using a JEM 1010 transmission electron microscope (JEOL Ltd., Japan). Images were acquired using a CCD Orius camera (Gatan, Inc., USA).

### 3.2 Results

According to ThT fluorescence assays (FIG. 2(A)), AID-X-2020 is a strong inhibitor of the aggregation of Aβ40. Even at 10 nM, well below its IC₅₀ in iP. *falciparum* cultures, AID-X-2020 prevents Aβ40 fibrillogenesis to a large extent. Therefore, inhibition of protein aggregation might be the main mechanism responsible for the antimalarial activity of this compound.

To discard the possibility that the decrease in ThT fluorescence observed in Aβ40 aggregation assays resulted from a steric hindrance imposed to ThT binding of amyloid fibrils by the presence of AID-X-2020, TEM was used to examine Aβ40 samples treated with AID-X-2020 (FIG. 2(B)). TEM images showed that the amyloid fibril aggregates of AID-X-2020-containing samples were smaller and more fragmented than those present in control untreated Aβ40, supporting the role of this compound as a protein aggregation inhibitor.

### EXAMPLE 4: Study of the correlation between the in vitro antimalarial activity of AID-X-2020 and the disruption of protein homeostasis in the treated parasites

To explore if there was a correlation between the observed *in vitro* antimalarial activity of AID-X-2020 and a disruption of protein homeostasis in the treated parasites, dot blots and Western blots of protein extracts of AID-X-2020-treated *P*. *falciparum* cultures were performed, where the presence of ubiquitinated proteins and of amyloid fibrils was examined.

### 4.1 Materials and methods

Cultures of the *P*. *falciparum* 3D7 strain were sorbitol synchronized in ring stages, and after 24 h were treated for 90 min with AID-X-2020 concentrations ranging from 33 nM to 33 µM. After that time, cultures were spun down and pellets were washed once with ice-cold PBS supplemented with EDTA-free protease inhibitor cocktail (PIC, Roche; 1 PIC tablet/10 mL PBS). For anti-ubiquitin Western blots, PBS was also supplemented with 20 mM N-ethylmaleimide. Once washed, parasite pellets were treated with 0.15% saponin at 4 °C for 15 min and washed by centrifugation (10,000× g, 15 min, 4 °C) with appropriately supplemented PBS until no hemoglobin was observed in the supernatant. Protein extracts were quantified with the bicinchoninic acid assay (ThermoFisher Scientific), following the manufacturer's instructions.

For dot blots, 4-µL drops of saponin extract containing 0.5 or 1 mg/mL protein were spotted on a nitrocellulose membrane. Once protein extracts were completely absorbed by the membranes, these were incubated for 3 h in blocking solution: 5% milk powder in TBS-Tween (200 mM tris-base, 1.5 M NaCl, 0.1% Tween-20). The blocked membranes were washed 3 × 5 min with TBS-Tween and incubated overnight at 4 °C with rabbit polyclonal anti-amyloid fibrils OC antibody (Millipore) diluted 1:500 in blocking solution or mouse monoclonal anti-spectrin α/β (Sigma) 1:10,000 in TBS-Tween.

For Western blots, 15 µg of saponin-extracted proteins were incubated for 5 min at 95 °C diluted in Laemmli solution (0.14 M SDS, 0.125 M tris-HCl, pH 6.8, 20% glycerol, 10% 2-mercaptoethanol, 3 mM bromophenol blue) and resolved by SDS-PAGE in 12% bis-tris acrylamide (Bio-Rad) gels run at 80 V until samples entered the resolving gel and at 120 V afterwards. Proteins were transferred from the gel to polyvinylidene difluoride membranes activated with methanol 100%. After transference, membranes were blocked with blocking solution for 1 h at room temperature, washed 3 × 5 min with TBS-Tween and probed overnight at 4 °C with rabbit polyclonal anti-ubiquitin antibody (Cell Signaling Technology) diluted 1:1,000 in blocking solution, or mouse monoclonal anti-spectrin α/β (Sigma) 1:10,000 in TBS-Tween. Then, membranes were washed 5 times with TBS-Tween and incubated for 1 h with either goat anti-rabbit (Upstate) or goat anti-mouse (Amersham Life Science, Inc.) IgG-horseradish peroxidase conjugate antibody diluted 1:10,000 in TBS-Tween. After probing with secondary antibodies, for both dot blot and Western blot membranes, four washes with TBS-Tween and one last wash with TBS were done and peroxidase substrate (ECL Prime Western Blotting Detection Reagent, Amersham Life Science, Inc.) was poured on the membrane and chemiluminescent signal was measured in a LAS 4000 reader (ImageQuant) at different exposure times.

### 4.2 Results

At the concentration of 90 nM, close to its IC₅₀ *in vitro,* AID-X-2020 treatment of P. *falciparum* cultures led to a reduction in ubiquitinated proteins along time (FIG. 3(A)), consistent with an inhibitory effect on protein aggregation. Exposure for only 90 min to >3 µM AID-X-2020 inhibited amyloid fibril formation (FIG. 3(D)) but resulted in a concomitant increase in ubiquitinated proteins (FIG. 3(C)). These results suggested a causal effect between decreasing protein aggregation and a deleterious effect on the parasite ultimately leading to a rapid generalized deregulation of proteostasis.

### EXAMPLE 5: Assay on the effect of the combination of AID-X-2020 and artemisinin

Artemisinin, one of the most potent antimalarials in use, has been described to cause protein damage/unfolding and to inhibit folding of newly synthesized proteins, likely inducing protein aggregation. As shown in the previous examples, AID-X-2020 appears to inhibit protein aggregation, the opposite action of artemisinin. Thus, this study was performed to confirm the capacity of AID-X-2020 to inhibit protein aggregation.

### 5.1 Materials and methods

For assays of AID-X-2020 and artemisinin, serial dilutions of both compounds were prepared at different concentration ratios (1:0, 0:1, 1:1, 1:2, 2:1, 1:5 and 5:1).

To assess the synergistic effect of AID-X-2020 and artemisinin, IC₅₀ values for each individual compound in the mixtures were calculated as described above and plotted in an isobologram ("x" value = AID-X-2020 IC₅₀ and "y" value = artemisinin IC₅₀). Fractional inhibitory concentration (FIC) values were calculated by dividing the IC₅₀ of one of the compounds in the mixture by the IC₅₀ of the same compound in the 1:0 or 0:1 ratio mixture.

### 5.2 Results

When *P. falciparum* cultures were treated with artemisinin and AID-X-2020 combined in different ratios, the resulting ΣFIC values were always higher than 1.5 (FIG. 3(E)), which indicates an antagonistic action of both drugs. Thus, parasite viability was significantly higher than expected if the activities of both drugs were additive, thus reinforcing the hypothesis that AID-X-2020 has protein aggregation inhibitory activity that antagonizes the antimalarial effect of artemisinin, and vice versa.

### EXAMPLE 6: Effect of AID-X-2020 on the pathogen's insect stage (the gametocyte to ookinete transition)

Because *Plasmodium* is known to have protein aggregates in the mosquito stages of *Plasmodium,* the effect of AID-X-2020 was tested on one of the key steps of the pathogen's development in the insect, namely the gametocyte to ookinete transition. Gametocytes are the sole stage of malaria parasites present in the blood circulation capable of transmitting the infection to the mosquito vector following their ingestion by a blood-feeding *Anopheles* female. Transmission-blocking strategies are therefore one of the main approaches being explored to disrupt the life cycle of *Plasmodium,* but active drugs at this critical step are scarce.

### 6.1 Materials and methods

### 6.1.1 Assay of gametocyte to ookinete transition inhibition

Eight days before ookinete production, 200 µL of *Plasmodium berghei* CTRP-GFP (which expresses GFP when reaching ookinete stage) (Vlachou, D. et al., 2004) in cryopreservation solution (RBC pellet:RPMI:30% glycerol in water, 1:1:2) was administered i.p. to a BALB/c mouse. Four days later this mouse was the donor to infect i.p. with 5×10⁷ parasitized red blood cells in 200 µL of PBS three mice that one hour before the infection had been pretreated i.p. with phenylhydrazine (120 µL of a 10 mg/mL solution in PBS).

For ookinete production, up to 1 mL of blood carrying gametocytes was collected from each animal by intracardiac puncture and diluted in 30 mL of ookinete medium: 10.4 g/L of RPMI supplemented with 2% w/v NaHCO₃, 0.05% w/v hypoxanthine, 0.02% w/v xanthurenic acid, 50 U/mL penicillin and 50 µg/mL streptomycin (Invitrogen), 20% heat-inactivated fetal bovine serum (FBS, Invitrogen), 25 mM HEPES, pH 7.4.

The effect of AID-X-2020 and DONE3TCI on gametocyte to ookinete conversion was assessed in three independent replicas by plating 120 µL of each culture mixed with growing concentrations of the compounds in 96-well plates and incubated for 24 h at 21 °C with orbital shaking at 50 rpm. 24 h later, samples were diluted 1:100 in PBS and analyzed in a LSRFortessa^{™} flow cytometer (BD Biosciences) set up with the 5 lasers, 20 parameters standard configuration. The GFP positive cell population was selected and counted using 488 nm laser excitation and a 525/40 nm emission collection filter. BD FACSDiva software (BD biosciences) was used in data collection, and Flowing Software 2.5.1 (Turku Centre for Biotechnology) was used for analysis.

### 6.2 Results

*In vitro* inhibition assays of the gametocyte to ookinete transition in the murine malaria parasite *Plasmodium berghei* indicated that ca. 0.5 µM AID-X-2020 abolished ookinete production in this model (FIG. 4(A)). On the other hand, the protein aggregation inhibitor DONE3TCI, which showed a potent antimalarial activity in *in vitro P. falciparum* cultures, with an IC₅₀ of ca. 80 nM (Table 2), did not have an observable effect on ookinete development up to a concentration of 2 µM (FIG. 4(B)). AID-X-2020 can then be used to arrest the life cycle of malaria parasites in the mosquito host.

### EXAMPLE 7: In vivo toxicity assays of AID-X-2020 and DONE3TCI

### 7.1 Materials and methods

BALB/c female and male mice (Janvier Laboratories) were maintained with unlimited access to food and water under standard environmental conditions (20-24 °C and 12/12 h light/dark cycle). Three 100 µL doses of an AID-X-2020 solution prepared to administer 0.0959, 0.3069 and 0.9822 mg/kg were tested. First, the lowest dose was intravenously injected to one female and one male mice. An oxygen stream of 4% isoflurane was used to anesthetize the mice, which were then maintained during the whole procedure (less than 3 minutes) with 2.5% isoflurane.

After the administration, mice were observed and different parameters related to their behavior (lethargy, motility alterations, seizures, coma, automutilation, aggressiveness, vocalizations, stereotyped movements) and physical conditions (pain, respiratory disturbances, tachycardia or bradycardia, dehydration, hair loss, body weight loss, dermatitis, bad hygiene, pruritus, tearing) were followed. If after 48 h no deleterious effects were observed, the following dose was administered to two other male and female mice. All mice were observed for 14 days after treatment in order to detect long-term side effects.

The animal care and protocol followed here adhered to the specific national and international guidelines stated in the Spanish Royal Decree 53/2013, which is based on the European regulation 2010/63/UE.

### 7.2 Results

*In vivo* toxicity assays in mice indicated that AID-X-2020 and DONE3TCI started inducing adverse effects in female mice at 10 mg/kg. In male mice toxicity started being observed at 17 and 3 mg/kg for AID-X-2020 and DONE3TCI, respectively. 10 mg/kg then is a tentative maximal dose of reference for future preclinical assays.

In human umbilical vein endothelial cell cultures, the AID-X-2020 concentration required for the reduction of cell viability by 50% (CC₅₀) was determined to be 3.4 µM, which resulted in a therapeutic index (CC₅₀/IC₅₀) of 37.8. Despite this relatively high *in vitro* toxicity, *in vivo* toxicity assays indicated that AID-X-2020 starts inducing adverse effects in female and male mice at 10 and 17 mg/kg respectively.

### EXAMPLE 8: Inhibition effect of other compounds of formula (I) on P. falciparum in vitro

Eighteen compounds were synthesized, which are representative of compounds included in formula (I) other than AID-X-2020, and a study of the antimalarial effect of seventeen of these compounds was performed. The antimalarial activity of these compounds was then compared to the antimalarial activity of AID-X-2020 (IC₅₀ = 0.09 µM).

### 8.1 Materials and Methods

### 8.1.1 General aspects of synthesis procedures

Steps and conditions referring to general aspects of the synthesis procedures were performed as described in section 1.1.1 of EXAMPLE 1.

### 8.1.2 Synthesis of 1,1'-(decane-1,10-diyl)bis{4-[(E)-4-(dimethylamino)styryl]-3-methylpyridin-1-ium} dibromide (EMA357)

Compound EMA357 was prepared following the first steps and conditions as described for the synthesis of compound AID-X-2020 (section 1.1.2 of EXAMPLE 1 - 1^{st} paragraph) until 1,1'-(decane-1,10-diyl)*bis*(3,4-dimethylpyridin-1-ium) dibromide was obtained.

A solution of 1,1'-(decane-1,10-diyl)*bis*(3,4-dimethylpyridin-1-ium) dibromide (257 mg, 0.50 mmol) and 4-(dimethylamino)benzaldehyde (149 mg, 1.00 mmol) in *n*-butanol (2.5 mL) was treated with six drops of piperidine and the reaction mixture was stirred under reflux for 16 h, and then concentrated under reduced pressure. The resulting black sticky residue was purified by automatic flash column chromatography (CH₂Cl₂ / 7N ammonia solution in MeOH 9.5:0.5 to 8:2), to provide 1,1'-(decane-1,10-diyl)*bis*{4-[(*E*)-4-(dimethylamino)styryl]-3-methylpyridin-1-ium} dibromide (127 mg, 33%) as a red oil; IR (ATR) v: 3390, 2925, 2852, 1640, 1573, 1525, 1478, 1435, 1364, 1310, 1218, 1185, 1170, 1128, 944, 814 cm⁻¹; ¹H NMR (400 MHz, CD₃OD) *δ*: 1.29-1.43 (m, 12H), 1.97 (m, 4H), 2.55 (s, 6H), 3.06 (s, 12H), 4.40 (t, *J* = 7.6 Hz, 4H), 6.79 (d, *J* = 9.2 Hz, 4H), 7.14 (d, *J* = 16.0 Hz, 2H), 7.64 (d, *J =* 9.2 Hz, 4H), 7.81 (d, *J =* 16.0 Hz, 2H), 8.18 (d, *J =* 6.8 Hz, 2H), 8.47 (br d, *J =* 6.8 Hz, 2H), 8.56 (br s, 2H); HRMS-ESI+ *m*/*z* calculated for [C₄₂H₅₆N₄]²⁺/2: 308.2247, found: 308.2241.

### 8.1.3 Synthesis of 1,1'-(decane-1,10-diyl)bis{4-{(E)-4-[(2-cyanoethyl)(methyl)amino]styryl}-3-methylpyridin-1-ium}dibromide (EMA359)

Compound EMA359 was prepared following the first steps and conditions as described for the synthesis of compound AID-X-2020 (section 1.1.2 of EXAMPLE 1 - 1^{st} paragraph) until 1,1'-(decane-1,10-diyl)*bis*(3,4-dimethylpyridin-1-ium) dibromide was obtained.

A solution of 1,1'-(decane-1,10-diyl)*bis*(3,4-dimethylpyridin-1-ium) dibromide (257 mg, 0.50 mmol) and 3-[(4-formylphenyl)(methyl)amino]propanenitrile (188 mg, 1.00 mmol) in *n*-butanol (2.5 mL) was treated with six drops of piperidine and the reaction mixture was stirred under reflux for 16 h, and then concentrated under reduced pressure. The resulting black oily residue was purified by automatic flash column chromatography (CH₂Cl₂ / 7N ammonia solution in MeOH 9:1), to provide 1,1'-(decane-1,10-diyl)*bis*{4-{(*E*)-4-[(2-cyanoethyl)(methyl)amino]styryl}-3-methylpyridin-1-ium} dibromide (204 mg, 48%) as a red solid; mp: 130-131 °C; IR (ATR) *v*: 3377, 2925, 2854, 1633, 1578, 1523, 1478, 1383, 1311, 1218, 1184, 1117, 959, 808 cm⁻¹; ¹H NMR (400 MHz, CD₃OD) *δ*: 1.31-1.43 (m, 12H), 1.97 (m, 4H), 2.56 (s, 6H), 2.76 (t, *J* = 6.8 Hz, 4H), 3.13 (s, 6H), 3.83 (t, *J =* 6.8 Hz, 4H), 4.41 (t, *J =* 7.6 Hz, 4H), 6.87 (d, *J =* 8.8 Hz, 4H), 7.20 (d, *J* = 16.0 Hz, 2H), 7.68 (d, *J* = 8.8 Hz, 4H), 7.82 (d, *J* = 16.0 Hz, 2H), 8.20 (d, *J* = 6.8 Hz, 2H), 8.50 (br d, *J* = 6.8 Hz, 2H), 8.59 (br s, 2H); HRMS-ESI+ m/z calculated for [C₄₆H₅₈N₆]²⁺/2: 347.2356, found: 347.2348.

### 8.1.4 Synthesis of 1,1'-(decane-1,10-diyl)bis{4-[(E)-4-(diphenylamino)styryl]-3-methylpyridin-1-ium} dibromide (EMA366)

Compound EMA366 was prepared following the first steps and conditions as described for the synthesis of compound AID-X-2020 (section 1.1.2 of EXAMPLE 1 - 1^{st} paragraph) until 1,1'-(decane-1,10-diyl)*bis*(3,4-dimethylpyridin-1-ium) dibromide was obtained.

A solution of 1,1'-(decane-1,10-diyl)*bis*(3,4-dimethylpyridin-1-ium) dibromide (257 mg, 0.50 mmol) and 4-(diphenylamino)benzaldehyde (273 mg, 1.00 mmol) in *n*-butanol (2.5 mL) was treated with six drops of piperidine and the reaction mixture was stirred under reflux for 16 h, and then concentrated under reduced pressure. The resulting black oily residue was purified by automatic flash column chromatography (CH₂Cl₂ / 7N ammonia solution in MeOH 9:1 to 85:15), to provide 1,1'-(decane-1,10-diyl)*bis*{4-[(*E*)-4-(diphenylamino)styryl]-3-methylpyridin-1-ium} dibromide (236 mg, 46%) as a red solid; mp: 163-165 °C; IR (ATR) v: 3352, 3186, 2929, 2854, 1658, 1581, 1510, 1485, 1388, 1318, 1285, 1222, 1192, 1178, 1138, 973, 820, 757, 697, 571 cm⁻¹; ¹H NMR (500 MHz, CD₃OD) *δ*: 1.30-1.43 (m, 12H), 1.98 (tt, *J* = *J' =* 7.5 Hz, 4H), 2.57 (s, 6H), 4.45 (t, *J* = 7.5 Hz, 4H), 7.00 (d, *J* = 8.5 Hz, 4H), 7.11-7.17 (m, 12H), 7.29 (d, *J* = 16.0 Hz, 2H), 7.34 (ddm, *J* = 8.5 Hz, *J'* = 7.5 Hz, 8H), 7.63 (d, *J* = 8.5 Hz, 4H), 7.81 (d, *J =* 16.0 Hz, 2H), 8.24 (d, *J* = 6.5 Hz, 2H), 8.57 (dd, *J* = 6.5 Hz, *J' =* 1.5 Hz, 2H), 8.65 (s, 2H); HRMS-ESI+ m/z calculated for [C₆₂H₆₄N₄]²⁺/2: 432.2560, found: 432.2565.

### 8.1.5 Synthesis of 1,1'-(decane-1,10-diyl)bis{3-methyl-4-[(E)-4-(pyrrolidin-1-yl)styryl]pyridin-1-ium} dibromide (EMA368)

Compound EMA368 was prepared following the first steps and conditions as described for the synthesis of compound AID-X-2020 (section 1.1.2 of EXAMPLE 1 - 1^{st} paragraph) until 1,1'-(decane-1,10-diyl)*bis*(3,4-dimethylpyridin-1-ium) dibromide was obtained.

A solution of 1,1'-(decane-1,10-diyl)*bis*(3,4-dimethylpyridin-1-ium) dibromide (257 mg, 0.50 mmol) and 4-(pyrrolidin-1-yl)benzaldehyde (175 mg, 1.00 mmol) in *n*-butanol (2.5 mL) was treated with six drops of piperidine and the reaction mixture was stirred under reflux for 22 h, and then concentrated under reduced pressure. The resulting red oily residue was purified by automatic flash column chromatography (CH₂Cl₂ / MeOH 10:0 to 9.3:0.7), to provide 1,1'-(decane-1,10-diyl)*bis*{3-methyl-4-[(*E*)-4-(pyrrolidin-1-yl)styryl]pyridin-1-ium} dibromide (233 mg, 56%) as a red solid; mp: 167-168 °C; IR (ATR) v: 3391, 2928, 2850, 1641, 1617, 1571, 1523, 1478, 1387, 1303, 1218, 1177, 1136, 961, 807 cm⁻¹; ¹H NMR (400 MHz, DMSO-d₆) *δ*: 1.21-1.31 (m, 12H), 1.86 (m, 4H), 1.98 (t, *J* = 5.6 Hz, 8H), 2.48 (s, 6H), 3.01 (t *J =* 5.6 Hz, 8H), 4.36 (t, *J* = 7.6 Hz, 4H), 6.62 (d, *J* = 8.8 Hz, 4H), 7.09 (d, *J = 16.0* Hz, 2H), 7.66 (d, *J* = 8.8 Hz, 4H), 7.89 (d, *J* = 16.0 Hz, 2H), 8.26 (d, *J* = 6.8 Hz, 2H), 8.65 (br d, *J* = 6.8 Hz, 2H), 8.72 (br s, 2H); HRMS-ESI+ *m*/*z* calculated for [C₄₆H₆₀N₄]²⁺/2: 334.2404, found: 334.2410.

### 8.1.6 Synthesis of 1,1'-(decane-1,10-diyl)bis{3-methyl-4-[(E)-4-morpholinostyryl]pyridin-1-ium} dibromide (EMA377)

Compound EMA377 was prepared following the first steps and conditions as described for the synthesis of compound AID-X-2020 (section 1.1.2 of EXAMPLE 1 - 1^{st} paragraph) until 1,1'-(decane-1,10-diyl)*bis*(3,4-dimethylpyridin-1-ium) dibromide was obtained.

A solution of 1,1'-(decane-1,10-diyl)*bis*(3,4-dimethylpyridin-1-ium) dibromide (514 mg, 1.00 mmol) and 4-morpholinobenzaldehyde (383 mg, 2.00 mmol) in *n*-butanol (5.0 mL) was treated with six drops of piperidine and the reaction mixture was stirred under reflux for 4 h, and then concentrated under reduced pressure. The resulting black oily residue was purified by automatic flash column chromatography (CH₂Cl₂ / MeOH 10:0 to 9:1), to provide 1,1'-(decane-1,10-diyl)*bis*{3-methyl-4-[(*E*)-4-morpholinostyryl]pyridin-1-ium} dibromide (281 mg, 33%) as a red solid; mp: 153-155 °C; IR (ATR) *v*: 3390, 2925, 2851, 1642, 1580, 1519, 1478, 1447, 1428, 1381, 1358, 1307, 1247, 1222, 1187, 1134, 1110, 1049, 925, 810, 635, 604, 572 cm⁻¹; ¹H NMR (400 MHz, CD₃OD) *δ*: 1.30-1.42 (m, 12H), 1.98 (tt, *J* = *J'* = 7.6 Hz, 4H), 2.57 (s, 6H), superimposed in part with the solvent signal 3.30 (t, *J =* 4.8 Hz, 8H), 3.83 (t, *J =* 4.8 Hz, 8H), 4.43 (t, *J =* 7.6 Hz, 4H), 7.02 (d, *J* = 9.2 Hz, 4H), 7.25 (d, *J =* 16.0 Hz, 2H), 7.69 (d, *J* = 9.2 Hz, 4H), 7.81 (d, *J* = 16.0 Hz, 2H), 8.23 (d, *J* = 6.8 Hz, 2H), 8.54 (dd, *J* = 6.8 Hz, *J'* = 1.6 Hz, 2H), 8.63 (br s, 2H); HRMS-ESI+ m/z calculated for [C₄₆H₆₀N₄O]²⁺/2: 350.2353, found: 350.2361.

### 8.1.7 Synthesis of 1,1'-(butane-1,4-diyl)bis{4-[(E)-4-(diethylamino)styryl]-3-methylpyridin-1-ium} dibromide (PRC-5)

A mixture of 1,4-dibromobutane (0.23 mL, 416 mg, 1.93 mmol) and 3,4-dimethylpyridine (0.49 mL, 467 mg, 4.36 mmol) was heated to 120 °C for 3 h. Then, isopropanol (2 mL) was added and the reaction mixture was stirred under reflux for 1 h. The mixture was allowed to cool down to room temperature the resulting brown residue was washed with ice-cold Et₂O (15 mL) and the remaining beige solid was dried *in vacuo,* taken up in MeOH (5 mL) and treated with cold Et₂O (2 × 15 mL), drawing off the liquids. After drying the residue *in vacuo,* 1,1'-(butane-1,4-diyl)bis(3,4-dimethylpyridin-1-ium) dibromide (757 mg, 88%) was obtained as a beige solid; mp: 109-110 °C; IR (ATR) v: 3431, 3360, 3041, 1641, 1621, 1512, 1472, 1460, 1234, 1149, 1027, 847, 706 cm⁻¹; ¹H NMR (400 MHz, CD₃OD) *δ*: 2.10 (tm, *J =* 7.2 Hz, 4H), 2.48 (s, 6H), 2.59 (s, 6H), 4.62 (br t, *J =* 7.2 Hz, 4H), 7.87 (d, *J* = 6.4 Hz, 2H), 8.71 (dd, *J* = 6.4 Hz, *J'* = 1.6 Hz, 2H), 8.81 (br s, 2H); HRMS-ESI+ *m*/*z* calculated for [C₁₈H₂₆N₂]²⁺/2: 135.1043, found: 135.1051.

A solution of 1,1'-(butane-1,4-diyl)*bis*(3,4-dimethylpyridin-1-ium) dibromide (669 mg, 1.55 mmol) and 4-(diethylamino)benzaldehyde (604 mg, 3.41 mmol) in *n*-butanol (8 mL) was treated with twelve drops of piperidine and the reaction mixture was stirred under reflux for 4 h, and then concentrated under reduced pressure. The resulting oily residue was purified twice by automatic flash column chromatography (CH₂Cl₂ / 7N ammonia solution in MeOH 95:5 to 85:15), to provide 1,1'-(butane-1,4-diyl)*bis*{4-[(*E*)-4-(diethylamino)styryl]-3-methylpyridin-1-ium} dibromide (511 mg, 44%) as a red solid; mp: 275-276 °C; IR (ATR) v: 3406, 2964, 1641, 1577, 1522, 1484, 1467, 1434, 1402, 1372, 1350, 1307, 1262, 1220, 1191, 1132, 1067, 975, 810 cm⁻¹; ¹H NMR (400 MHz, CD₃OD) *δ*: 1.21 (t, *J* = 7.2 Hz, 12H), 2.07 (m, 4H), 2.54 (s, 6H), 3.49 (q, *J* = 7.2 Hz, 8H), 4.48 (m, 4H), 6.76 (d, *J* = 9.2 Hz, 4H), 7.10 (d, *J* = 16.0 Hz, 2H), 7.61 (d, *J* = 9.2 Hz, 4H), 7.81 (d, *J* = 16.0 Hz, 2H), 8.17 (d, *J* = 6.8 Hz, 2H), 8.47 (dd, *J* = 6.8 Hz, *J'* = 1.6 Hz, 2H), 8.56 (br s, 2H); HRMS-ESI+ *m*/*z* calculated for [C₄₀H₅₂N₄]²⁺/2: 294.2091, found: 294.2098.

### 8.1.8 Synthesis of 1,1'-(octane-1,8-diyl)bis{4-[(E)-4-(diethylamino)styryl]-3-methylpyridin-1-ium} dibromide (PRC-8)

A mixture of 1,8-dibromooctane (0.37 mL, 546 mg, 2.00 mmol) and 3,4-dimethylpyridine (0.49 mL, 467 mg, 4.36 mmol) was heated to 120 °C for 4 h. Then, isopropanol (2 mL) was added and the reaction mixture was stirred under reflux for 1 h. The mixture was allowed to cool down to room temperature the resulting brown residue was washed with ice-cold Et₂O (15 mL) and the remaining brown sticky oil was dried *in vacuo,* taken up in MeOH (5 mL) and treated with cold Et₂O (2 × 15 mL), drawing off the liquids. After drying the residue *in vacuo,* 1,1'-(octane-1,8-diyl)bis(3,4-dimethylpyridin-1-ium) dibromide (510 mg, 52%) was obtained as a beige solid; mp: 183-184 °C; IR (ATR) v: 3459, 3026, 2925, 2853, 1639, 1511, 1484, 1460, 1444, 1393, 1337, 1230, 1141, 1020, 951, 839, 711 cm⁻¹; ¹H NMR (400 MHz, CD₃OD) *δ*: 1.41 (m, 8H), 2.00 (tt, *J* = 7.6 Hz, *J'* = 7.2 Hz, 4H), 2.48 (s, 6H), 2.59 (s, 6H), 4.53 (t, *J* = 7.6 Hz, 4H), 7.86 (d, *J* = 6.4 Hz, 2H), 8.68 (dd, *J* = 6.4 Hz, *J'* = 1.6 Hz, 2H), 8.77 (br s, 2H); HRMS-ESI+ *m*/*z* calculated for [C₂₂H₃₄N₂]²⁺/2: 163.1356, found: 163.1358.

A solution of 1,1'-(octane-1,8-diyl)*bis*(3,4-dimethylpyridin-1-ium) dibromide (410 mg, 0.84 mmol) and 4-(diethylamino)benzaldehyde (328 mg, 1.85 mmol) in *n*-butanol (4.5 mL) was treated with six drops of piperidine and the reaction mixture was stirred under reflux for 17 h, and then concentrated under reduced pressure. The resulting black oily residue was purified by automatic flash column chromatography (CH₂Cl₂ / MeOH 95:5 to 85:15), to provide 1,1'-(octane-1,8-diyl)*bis*{4-[(*E*)-4-(diethylamino)styryl]-3-methylpyridin-1-ium} dibromide (230 mg, 34%) as a red solid; mp: 235-236 °C; IR (ATR) *v*: 3393, 2970, 2928, 1641, 1570, 1521, 1478, 1404, 1350, 1310, 1259, 1219, 1185, 1128, 1076, 960, 807, 702 cm⁻¹; ¹H NMR (400 MHz, CD₃OD) *δ*: 1.21 (t, *J =* 7.2 Hz, 12H), 1.41 (m, 8H), 1.87 (tt, *J* = *J'* = 7.2 Hz, 4H), 2.54 (s, 6H), 3.48 (q, *J* = 7.2 Hz, 8H), 4.40 (t, *J* = 7.2 Hz, 4H), 6.76 (d, *J* = 8.8 Hz, 4H), 7.10 (d, *J* = 16.0 Hz, 2H), 7.61 (d, *J* = 8.8 Hz, 4H), 7.79 (d, *J* = 16.0 Hz, 2H), 8.16 (d, *J* = 6.8 Hz, 2H), 8.46 (dd, *J* = 6.8 Hz, *J' =* 1.6 Hz, 2H), 8.55 (br s, 2H); HRMS-ESI+ *m*/*z* calculated for [C₄₄H₆₀N₄]²⁺/2: 322.2404, found: 322.2417.

### 8.1.9 Synthesis of 1,1'-(nonane-1,9-diyl)bis{4-[(E)-4-(diethylamino)styryl]-3-methylpyridin-1-ium} dibromide (PRC-25)

A mixture of 1,9-dibromononane (0.41 mL, 577 mg , 2.02 mmol) and 3,4-dimethylpyridine (0.49 mL, 467 mg, 4.36 mmol) was heated to 120 °C for 3 h. Then, isopropanol (2 mL) was added and the reaction mixture was stirred under reflux for 1 h. The mixture was allowed to cool down to room temperature, the resulting brown residue was washed with ice-cold Et₂O (2 × 20 mL) and the remaining brown sticky oil was dried in vacuo, taken up in MeOH (1 mL) and treated with cold Et₂O (2 × 20 mL), drawing off the liquids. After drying the residue in vacuum, 1,1'-(nonane-1,9-diyl)bis(3,4-dimethylpyridin-1-ium) dibromide (665 mg, 66%) was obtained as a brown oil that solidified on standing; mp: 88-90 °C; IR (ATR) v: 3435, 2933, 2852, 1638, 1505, 1469, 1335, 1228, 1152, 1138, 1023, 834, 754, 729, 707 cm⁻¹; ¹H NMR (400 MHz, DMSO-d₆) *δ*: 1.17-1.31 (m, 10H), 1.88 (tt, *J* = *J' =* 7.2 Hz, 4H), 2.39 (s, 6H), 2.51 (s, 6H), 4.49 (t, *J =* 7.2 Hz, 4H), 7.94 (d, *J* = 6.4 Hz, 2H), 8.84 (br d, *J* = 6.4 Hz, 2H), 8.94 (br s, 2H); HRMS-ESI+ *m*/*z* calculated for [C₂₃H₃₆N₂]²⁺/2: 170.1434, found 170.1435.

A solution of 1,1'-(nonane-1,9-diyl)*bis*(3,4-dimethylpyridin-1-ium) dibromide (250 mg, 0.50 mmol) and 4-(diethylamino)benzaldehyde (195 mg, 1.10 mmol) in n-butanol (2.5 mL) was treated with four drops of piperidine and the reaction mixture was stirred under reflux for 4 h, and then concentrated under reduced pressure. The resulting black oily residue was purified by automatic flash column chromatography (CH₂Cl₂ / MeOH 95:5 to 85:15). Then fractions containing the desired product were evaporated and the residue was dissolved in hot isopropanol (15 mL) and after addition of Et₂O (30 mL) and drawing off the liquids, 1,1'-(nonane-1,9-diyl)*bis*{4-[(*E*)-4-(diethylamino)styryl]-3-methylpyridin-1-ium} dibromide (193 mg, 47%) was obtained as a red solid; mp: 138-142 °C; IR (ATR) v: 3381, 2920, 2852, 1642, 1567, 1519, 1482, 1402, 1347, 1308, 1258, 1217, 1185, 1126, 1070, 954, 807 cm⁻¹; ¹H NMR (400 MHz, DMSO-d₆) *δ*: 1.13 (t, *J* = 7.2 Hz, 12H), 1.22-1.31 (m, 10H), 1.87 (tt, *J = J'* = 7.2 Hz, 4H), 2.48 (s, 6H), 3.43 (q, *J* = 7.2 Hz, 8H), 4.36 (t, *J* = 7.2 Hz, 4H), 6.74 (d, *J =* 9.2 Hz, 4H), 7.08 (d, *J* = 16.0 Hz, 2H), 7.64 (d, *J* = 9.2 Hz, 4H), 7.87 (d, *J* = 16.0 Hz, 2H), 8.26 (d, *J* = 6.8 Hz, 2H), 8.66 (dd, *J* = 6.8 Hz, *J*' = 1.6 Hz, 2H), 8.73 (br s, 2H); HRMS-ESI+ m/z calculated for [C₄₅H₆₂N₄]²⁺/2: 329.2482, found: 329.2485.

### 8.1.10 Synthesis of 1,1'-(undecane-1,11-diyl)his{4-[(E)-4-(diethylamino)styryl]-3-methylpyridin-1-ium} dibromide (PRC-14)

A mixture of 1,11-dibromoundecane (0.47 mL, 627 mg, 2.00 mmol) and 3,4-dimethylpyridine (0.49 mL, 467 mg, 4.36 mmol) was heated to 120 °C for 3 h. Then, isopropanol (2 mL) was added and the reaction mixture was stirred under reflux for 1 h. The mixture was allowed to cool down to room temperature, the resulting brown residue was washed with ice-cold Et₂O (2 × 20 mL) and the remaining brown sticky oil was dried in vacuum, taken up in CH₂Cl₂ (1 mL) and treated with cold Et₂O (2 × 20 mL), drawing off the liquids. After drying the residue in vacuum, 1,1'-(undecane-1,11-diyl)*bis*(3,4-dimethylpyridin-1-ium) dibromide (0.98 g, 93%) was obtained as a brown oil; IR (ATR) v: 3403, 3020, 2924, 2853, 1638, 1510, 1481, 1466, 1389, 1229, 1146, 1026, 843, 707 cm⁻¹; ¹H NMR (400 MHz, CD₃OD) δ: 1.29-1.42 (m, 14H), 1.99 (tt, *J* = *J*' = 7.6 Hz, 4H), 2.48 (s, 6H), 2.59 (s, 6H), 4.52 (t, *J* = 7.6 Hz, 4H), 7.86 (d, *J* = 6.4 Hz, 2H), 8.67 (dd, *J* = 6.4 Hz, *J*' = 1.6 Hz, 2H), 8.76 (br s, 2H); HRMS-ESI+ m/z calculated for [C₂₅H₄₀N₂]²⁺/2: 184.1590, found: 184.1598.

A solution of 1,1'-(undecane-1,11-diyl)*bis*(3,4-dimethylpyridin-1-ium) dibromide (396 mg, 0.75 mmol) and 4-(diethylamino)benzaldehyde (293 mg, 1.65 mmol) in *n*-butanol (3.75 mL) was treated with six drops of piperidine and the reaction mixture was stirred under reflux for 4 h, and then concentrated under reduced pressure, to afford 1,1'-(decane-1,10-diyl)*bis*{4-[(*E*)-4-(diethylamino)styryl]-3-methylpyridin-1-ium} dibromide (180 mg, 28%) as a red solid, which was used in the following step without further purification; mp: 81-82 °C; IR (ATR) v: 3389, 2924, 2847, 1641, 1572, 1522, 1478, 1405, 1351, 1310, 1259, 1217, 1186, 1157, 1133, 1075, 1008, 959, 808, 783, 701 cm⁻¹; ¹H NMR (400 MHz, DMSO-d₆) *δ*: 1.12 (t, *J* = 6.8 Hz, 12H), 1.20-1.31 (m, 14H), 1.86 (tt, *J* = *J*' = 7.2 Hz, 4H), 2.48 (s, 6H), 3.43 (q, *J =* 6.8 Hz, 8H), 4.36 (t, *J = 7.2* Hz, 4H), 6.74 (d, *J =* 9.2 Hz, 4H), 7.08 (d, *J =* 16.0 Hz, 2H), 7.64 (d, *J =* 9.2 Hz, 4H), 7.88 (d, *J =* 16.0 Hz, 2H), 8.26 (d, *J* = 6.8 Hz, 2H), 8.65 (br d, *J* = 6.8 Hz, 2H), 8.73 (br s, 2H); HRMS-ESI+ *m*/*z* calculated for [C₄₇H₆₆N₄]²⁺/2: 343.2638, found: 343.2640.

### 8.1.11 Synthesis of 1,1'-(dodecane-1,12-diyl)bis{4-[(E)-4-(diethylamino)styryl]-3-methylpyridin-1-ium} dibromide (PRC-15)

A mixture of 1,12-dibromododecane (656 mg, 2.00 mmol) and 3,4-dimethylpyridine (0.49 mL, 467 mg, 4.36 mmol) was heated to 120 °C for 3 h. Then, isopropanol (2 mL) was added and the reaction mixture was stirred under reflux for 1 h. The mixture was allowed to cool down to room temperature, the resulting brown residue was washed with ice-cold Et₂O (2 × 20 mL) and the remaining brown sticky oil was dried in vacuum, taken up in CH₂Cl₂ (1 mL) and treated with cold Et₂O (2 × 20 mL), drawing off the liquids. After drying the residue in vacuum, 1,1'-(dodecane-1,12-diyl)bis(3,4-dimethylpyridin-1-ium) dibromide (850 mg, 78%) was obtained as a brown oil that solidified on standing; mp: 82-83 °C; IR (ATR) v: 3438, 3373, 2994, 2923, 2853, 1636, 1516, 1466, 1387, 1332, 1228, 1147, 1027, 856, 837, 721, 709, 619, 592 cm⁻¹; ¹H NMR (400 MHz, CD₃OD) δ: 1.28-1.41 (m, 16H), 1.99 (tt, *J* = *J*' = 7.6 Hz, 4H), 2.48 (s, 6H), 2.59 (s, 6H), 4.52 (t, *J* = 7.6 Hz, 4H), 7.86 (d, *J =* 6.4 Hz, 2H), 8.68 (dd, *J* = 6.4 Hz, *J*' = 1.6 Hz, 2H), 8.77 (br s, 2H); HRMS-ESI+ *m*/*z* calculated for [C₂₆H₄₂N₂]²⁺/2: 191.1669, found: 191.1675.

A solution of 1,1'-(dodecane-1,12-diyl)bis(3,4-dimethylpyridin-1-ium) dibromide (379 mg, 0.70 mmol) and 4-(diethylamino)benzaldehyde (273 mg, 1.54 mmol) in n-butanol (3.5 mL) was treated with six drops of piperidine and the reaction mixture was stirred under reflux for 4.5 h, and then concentrated under reduced pressure. The resulting black oily residue was purified by automatic flash column chromatography (CH₂Cl₂ / MeOH 95:5), to provide 1,1'-(dodecane-1,12-diyl)*bis*{4-[(E)-4-(diethylamino)styryl]-3-methylpyridin-1-ium} dibromide (116 mg, 19%) as a red solid; mp: 112-114 °C; IR (ATR) *v:* 3400, 2971, 2927, 2852, 1641, 1574, 1519, 1477, 1435, 1403, 1348, 1312, 1259, 1219, 1188, 1157, 1126, 1077, 961, 824, 789, 571 cm⁻¹; ¹H NMR (400 MHz, DMSO-d₆) δ: 1.13 (t, *J =* 7.2 Hz, 12H), 1.19-1.32 (m, 16H), 1.88 (tt, *J* = *J*' = 7.2 Hz, 4H), 2.48 (s, 6H), 3.43 (q, *J* = 7.2 Hz, 8H), 4.36 (t, *J =* 7.2 Hz, 4H), 6.74 (d, *J =* 8.8 Hz, 4H), 7.08 (d, *J* = 16.0 Hz, 2H), 7.64 (d, *J =* 8.8 Hz, 4H), 7.88 (d, *J* = 16.0 Hz, 2H), 8.26 (d, *J* = 6.8 Hz, 2H), 8.66 (br d, *J* = 6.8 Hz, 2H), 8.73 (br s, 2H); HRMS-ESI+ m/z calculated for [C₄₈H₆₈N₄]²⁺/2: 350.2717, found: 350.2721.

### 8.1.12 Synthesis of 1,1'-(3,6-dioxaoctane-1,8-diyl)bis{4-[(E)-4-(diethylamino)styryl]-3-methylpyridin-1-ium}diiodide (PRC-20)

A mixture of 1,2-*bis*(2-iodoethoxy)ethane (0.27 mL, 548 mg, 1.48 mmol) and 3,4-dimethylpyridine (0.37 mL, 353 mg, 3.29 mmol) was heated to 120 °C for 3 h. Then, isopropanol (2 mL) was added and the reaction mixture was stirred under reflux for 1 h. The mixture was allowed to cool down to room temperature, the resulting brown residue was washed with ice-cold Et₂O (2 × 20 mL) and the remaining brown sticky oil was dried in vacuum, taken up in MeOH (1 mL) and treated with cold Et₂O (2 × 20 mL), drawing off the liquids. After drying the residue in vacuum, 1,1'-(3,6-dioxaoctane-1,8-diyl)bis(3,4-dimethylpyridin-1-ium) diiodide (620 mg, 72%) was obtained as a brown oil that solidified on standing; mp: 135-136 °C; IR (ATR) v: 3426, 3029, 2983, 2937, 2878, 1636, 1510, 1476, 1450, 1369, 1353, 1327, 1297, 1247, 1230, 1217, 1139, 1102, 1091, 1026, 992, 977, 912, 886, 813, 713, 704, 609, 558 cm⁻¹; ¹H NMR (400 MHz, DMSO-d₆) δ: 2.39 (s, 6H), 2.53 (s, 6H), 3.48 (s, 4H), 3.84 (t, *J* = 5.2 Hz, 4H), 4.64 (t, *J* = 5.2 Hz, 4H), 7.95 (d, *J* = 6.0 Hz, 2H), 8.71 (dd, *J* = 6.0 Hz, *J'* = 1.6 Hz, 2H), 8.81 (br s, 2H); HRMS-ESI+ m/z calculated for [C₂₀H₃₀N₂O₂]²⁺/2: 165.1148, found 165.1147.

A solution of 1,1'-(3,6-dioxaoctane-1,8-diyl)*bis*(3,4-dimethylpyridin-1-ium) diiodide (409 mg, 0.70 mmol) and 4-(diethylamino)benzaldehyde (273 mg, 1.54 mmol) in *n*-butanol (3.5 mL) was treated with six drops of piperidine and the reaction mixture was stirred under reflux for 4.5 h, and then concentrated under reduced pressure. The resulting black oily residue was purified by automatic flash column chromatography (CH₂Cl₂ / MeOH 95:5), to provide 1,1'-(3,6-dioxaoctane-1,8-diyl)*bis*{4-[(*E*)-4-(diethylamino)styryl]-3-methylpyridin-1-ium} diiodide (200 mg, 32%) as a red solid; mp: 71-72 °C; IR (ATR) v: 3417, 2967, 2920, 2859, 1640, 1567, 1519, 1477, 1435, 1404, 1350, 1307, 1258, 1218, 1184, 1154, 1128, 1072, 1009, 812, 700, 603 cm⁻¹; ¹H NMR (400 MHz, DMSO-d₆) *δ*: 1.12 (t, *J* = 7.2 Hz, 12H), 2.44 (s, 6H), 3.43 (q, *J* = 7.2 Hz, 8H), 3.54 (s, 4H), 3.85 (t, *J =* 5.2 Hz, 4H), 4.53 (t, *J =* 5.2 Hz, 4H), 6.72 (d, *J =* 8.8 Hz, 4H), 7.04 (d, *J* = 16.0 Hz, 2H), 7.61 (d, *J =* 8.8 Hz, 4H), 7.83 (d, *J* = 16.0 Hz, 2H), 8.22 (d, *J* = 6.8 Hz, 2H), 8.54 (br d, *J* = 6.8 Hz, 2H), 8.61 (br s, 2H); HRMS-ESI+ *m*/*z* calculated for [C₄₂H₅₆N₄O₂]²⁺/2: 324.2196, found: 324.2197.

### 8.1.13 Synthesis of 1,1'-(3,6,9-trioxaundecane-1,11-diyl)bis{4-[(E)-4-(diethylamino)styryl]-3-methylpyridin-1-ium}dichloride (PRC-28)

A mixture of *bis*[2-(2-chloroethoxy)ethyl] ether (0.26 mL, 307 mg, 1.33 mmol), 3,4-dimethylpyridine (0.49 mL, 467 mg, 4.36 mmol), and Nal (15 mg, 0.10 mmol) was heated to 120 °C for 4 h. Then, isopropanol (1.5 mL) was added and the reaction mixture was stirred under reflux for 1 h. The mixture was allowed to cool down to room temperature, the resulting brown residue was washed with ice-cold Et₂O (2 × 20 mL) and the remaining brown sticky oil was dried in vacuum, taken up in MeOH (1 mL) and treated with cold Et₂O (2 × 20 mL), drawing off the liquids. After drying the residue in vacuum, 1,1'-(3,6,9-trioxaundecane-1,11-diyl)bis(3,4-dimethylpyridin-1-ium) dichloride (467 mg, 79%) was obtained as a brown oil; IR (ATR) v: 3371, 3043, 2923, 2871, 1639, 1510, 1480, 1449, 1233, 1086, 1026, 931, 836, 706 cm⁻¹; ¹H NMR (400 MHz, DMSO-d₆) δ: 2.38 (s, 6H), 2.52 (s, 6H), 3.38 (tm, *J* = 4.8 Hz, 4H), 3.49 (tm, *J* = 4.8 Hz, 4H), 3.87 (t, *J =* 4.8 Hz, 4H), 4.67 (t, *J* = 4.8 Hz, 4H), 7.93 (d, *J* = 6.4 Hz, 2H), 8.75 (dd, *J* = 6.4 Hz, *J*' = 1.6 Hz, 2H), 8.85 (br s, 2H); HRMS-ESI+ m/z calculated for [C₂₂H₃₄N₂O₃]²⁺/2: 187.1279, found 187.1292.

A solution of 1,1'-(3,6,9-trioxaundecane-1,11-diyl)*bis*(3,4-dimethylpyridin-1-ium) dichloride (190 mg, 0.43 mmol) and 4-(diethylamino)benzaldehyde (164 mg, 0.93 mmol) in n-butanol (2.2 mL) was treated with four drops of piperidine and the reaction mixture was stirred under reflux overnight, and then concentrated under reduced pressure. The resulting black oily residue was purified by automatic flash column chromatography (CH₂Cl₂ / MeOH 95:5 to 85:15), to provide 1,1'-(3,6,9-trioxaundecane-1,11-diyl)*bis*{4-[(*E*)-4-(diethylamino)styryl]-3-methylpyridin-1-ium} dichloride (55 mg, 17%) as a red oil; IR (ATR) v: 3364, 2970, 2925, 2859, 1640, 1573, 1522, 1479, 1436, 1406, 1352, 1311, 1260, 1223, 1187, 1156, 1132, 1075, 1011, 814, 700, 607 cm⁻¹; ¹H NMR (400 MHz, DMSO-d₆) *δ*: 1.12 (t, *J =* 7.2 Hz, 12H), 2.44 (s, 6H), superimposed in part 3.42 (tm, *J* = 4.8 Hz, 4H), 3.43 (q, *J = 7.2* Hz, 8H), 3.52 (tm, *J =* 4.8 Hz, 4H), 3.86 (t, *J =* 4.8 Hz, 4H), 4.54 (t, *J* = 4.8 Hz, 4H), 6.73 (d, *J* = 8.8 Hz, 4H), 7.05 (d, *J* = 16.0 Hz, 2H), 7.62 (d, *J* = 8.8 Hz, 4H), 7.85 (d, *J* = 16.0 Hz, 2H), 8.22 (d, *J =* 6.8 Hz, 2H), 8.57 (br d, *J* = 6.8 Hz, 2H), 8.63 (br s, 2H); HRMS-ESI+ *m*/*z* calculated for [C₄₄H₆₀N₄O₃]2⁺/2: 346.2327, found: 346.2328.

### 8.1.14 Synthesis of 1,1'-[1,4-phenylenebis(methylene)]bis{4-[(E)-4-(diethylamino)styryl]-3-methylpyridin-1-ium}dibromide (PRC-29)

A mixture of 1,4-*bis*(bromomethyl)benzene (527 mg, 2.00 mmol) and 3,4-dimethylpyridine (0.49 mL, 467 mg, 4.36 mmol) was heated to 120 °C for 2 h. Then, isopropanol (4 mL) was added and the reaction mixture was stirred under reflux for 1 h. The mixture was allowed to cool down to room temperature, the resulting brown residue was washed with ice-cold Et₂O (2 × 20 mL) and the remaining brown sticky oil was dried in vacuum, taken up in MeOH (1 mL) and treated with cold Et₂O (2 × 20 mL), drawing off the liquids. After drying the residue in vacuum, 1,1'-[1,4-phenylenebis(methylene)]*bis*(3,4-dimethylpyridin-1-ium) dibromide (813 mg, 85%) was obtained as a white solid; mp: >300 °C; IR (ATR) v: 3008, 2984, 2920, 1634, 1507, 1484, 1448, 1364, 1339, 1297, 1248, 1223, 1142, 1039, 1027, 961, 931, 887, 866, 770, 744, 608, 557 cm⁻¹; ¹H NMR (400 MHz, DMSO-d₆) δ: 2.37 (s, 6H), 2.50 (s, 6H), 5.74 (s, 4H), 7.59 (s, 4H), 7.96 (d, *J* = 6.4 Hz, 2H), 8.94 (dd, *J* = 6.4 Hz, *J'* = 1.6 Hz, 2H), 9.03 (br s, 2H); HRMS-ESI+ m/z calculated for [C₂₂H₂₆N₂]²⁺/2: 159.1043, found 159.1032.

A solution of 1,1'-[1,4-phenylenebis(methylene)]bis(3,4-dimethylpyridin-1-ium) dibromide (231 mg, 0.48 mmol) and 4-(diethylamino)benzaldehyde (156 mg, 0.88 mmol) in n-butanol (2 mL) was treated with four drops of piperidine and the reaction mixture was stirred under reflux overnight, and then concentrated under reduced pressure. The resulting black oily residue was purified by automatic flash column chromatography (CH₂Cl₂ / MeOH 95:5). The fractions containing the desired product were evaporated and the resulting oily residue was dissolved in hot isopropanol (15 mL) and treated with Et₂O (40 mL), drawing off the liquid. After drying the residue in vacuum, 1,1'-[1,4-phenylenebis(methylene)]*bis*{4-[(*E*)-4-(diethylamino)styryl]-3-methylpyridin-1-ium} dibromide (320 mg, 84%) was obtained as a red solid; mp: 246-248 °C; IR (ATR) v: 3380, 2969, 2910, 1635, 1567, 1520, 1478, 1446, 1356, 1312, 1260, 1211, 1185, 1151, 1128, 1074, 1013, 970, 813, 701, 645 cm⁻¹; ¹H NMR (500 MHz, DMSO-d₆) *δ*: 1.12 (t, *J* = 7.0 Hz, 12H), 2.46 (s, 6H), 3.43 (q, *J* = 7.0 Hz, 8H), 5.62 (s, 4H), 6.73 (d, *J* = 9.0 Hz, 4H), 7.06 (d, *J* = 16.0 Hz, 2H), 7.58 (s, 4H), 7.63 (d, *J* = 9.0 Hz, 4H), 7.88 (d, *J* = 16.0 Hz, 2H), 8.27 (d, *J* = 6.5 Hz, 2H), 8.76 (dd, *J* = 6.5 Hz, *J*' = 1.5 Hz, 2H), 8.83 (br s, 2H); HRMS-ESI+ m/z calculated for [C₄₄H₅₂N₄]²⁺/2: 318.2091, found: 318.2090.

### 8.1.15 Synthesis of 1,1'-[1,3-phenylenebis(methylene)]bis{4-[(E)-4-(diethylamino)styryl]-3-methylpyridin-1-ium}dibromide (PRC-31)

A mixture of 1,3-*bis*(bromomethyl)benzene (527 mg, 2.00 mmol) and 3,4-dimethylpyridine (0.49 mL, 467 mg, 4.36 mmol) was heated to 120 °C for 2 h. Then, isopropanol (4 mL) was added and the reaction mixture was stirred under reflux for 1 h. The mixture was allowed to cool down to room temperature, the resulting brown residue was washed with ice-cold Et₂O (2 × 20 mL) and the remaining brown sticky oil was dried in vacuum, taken up in MeOH (1 mL) and treated with cold Et₂O (2 × 20 mL), drawing off the liquids. After drying the residue in vacuum, 1,1'-[1,3-phenylenebis(methylene)]*bis*(3,4-dimethylpyridin-1-ium) dibromide (785 mg, 82%) was obtained as a brown solid; mp: 152-153 °C; IR (ATR) v: 3460, 3414, 3006, 2920, 1636, 1509, 1480, 1450, 1387, 1362, 1340, 1225, 1163, 1139, 1020, 856, 842, 740, 705, 692, 652, 613 cm⁻¹; ¹H NMR (500 MHz, DMSO-d₆) *δ*: 2.40 (s, 6H), 2.52 (s, 6H), 5.77 (s, 4H), 7.47-7.55 (m, 3H), 7.80 (t, *J* = 2.0 Hz, 1H), 7.98 (d, *J* = 6.0 Hz, 2H), 8.94 (dd, *J* = 6.0 Hz, *J*' = 1.5 Hz, 2H), 9.10 (br s, 2H); HRMS-ESI+ m/z calculated for [C₂₂H₂₆N₂]²⁺/2: 159.1043, found 159.1042.

A solution of *1*,1'-[1,3-phenylene*bis*(methylene)]bis(3,4-dimethylpyridin-1-ium) dibromide (231 mg, 0.48 mmol) and 4-(diethylamino)benzaldehyde (156 mg, 0.88 mmol) in *n*-butanol (2 mL) was treated with four drops of piperidine and the reaction mixture was stirred under reflux overnight, and then concentrated under reduced pressure. The resulting black oily residue was purified by automatic flash column chromatography (CH₂Cl₂ / MeOH 100:0 to 95:5). The fractions containing the desired product were evaporated and the resulting oily residue was dissolved in hot isopropanol (15 mL) and treated with Et₂O (40 mL), drawing off the liquid. After drying the residue in vacuum, 1,1'-[1,3-phenylenebis(methylene)]bis{4-[(*E*)-4-(diethylamino)styryl]-3-methylpyridin-1-ium} dibromide (20 mg, 5%) was obtained as a red solid; mp: 66-67 °C; IR (ATR) v: 3347, 2969, 2920, 1639, 1572, 1523, 1479, 1438, 1406, 1350, 1310, 1261, 1213, 1187, 1151, 1128, 1076, 1013, 971, 808, 785, 740, 700 cm⁻¹; ¹H NMR (500 MHz, DMSO-d₆) δ: 1.13 (t, *J* = 7.0 Hz, 12H), 2.48 (s, 6H), 3.43 (q, *J* = 7.0 Hz, 8H), 5.64 (s, 4H), 6.74 (d, *J* = 9.0 Hz, 4H), 7.08 (d, *J* = 16.0 Hz, 2H), 7.49-7.51 (m, 3H), 7.60 (br s, 1H), 7.64 (d, *J* = 9.0 Hz, 4H), 7.90 (d, *J* = 16.0 Hz, 2H), 8.29 (d, *J* = 7.0 Hz, 2H), 8.74 (dd, *J* = 7.0 Hz, *J'* = 1.5 Hz, 2H), 8.82 (br s, 2H); HRMS-ESI+ m/z calculated for [C₄₄H₅₂N₄]²⁺/2: 318.2091, found: 318.2090.

### 8.1.16 Synthesis of 1,1'-(decane-1,10-diyl)bis{4-[(E)-4-(diethylamino)styryl]pyridin-1-ium} dibromide (PRC-39)

A mixture of 1,10-dibromodecane (0.44 mL, 587 mg, 1.96 mmol) and 4-methylpyridine (0.43 mL, 412 mg , 4.42 mmol) was heated to 120 °C for 3 h. Then, isopropanol (2 mL) was added and the reaction mixture was stirred under reflux for 1 h. The mixture was allowed to cool down to room temperature, the resulting brown residue was washed with ice-cold Et₂O (2 × 20 mL) and the remaining brown sticky oil was dried in vacuo, taken up in MeOH (1 mL) and treated with cold Et₂O (2 × 20 mL), drawing off the liquids. After drying the residue in vacuum, 1,1'-(decane-1,10-diyl)bis(4-methylpyridin-1-ium) dibromide (751 mg, 79%) was obtained as a orange oil that solidified on standing; mp: 56-57 °C; IR (ATR) v: 3350, 3013, 2920, 2852, 1638, 1516, 1469, 1379, 1219, 1171, 1030, 828, 730, 710 cm⁻¹; ¹H NMR (400 MHz, DMSO-d₆) δ: 1.17-1.31 (m, 12H), 1.87 (tt, *J = J*' = 7.2 Hz, 4H), 2.61 (s, 6H), 4.53 (t, *J* = 7.2 Hz, 4H), 7.99 (d, *J* = 6.4 Hz, 4H), 8.96 (d, *J* = 6.4 Hz, 4H); HRMS-ESI+ m/z calculated for [C₂₂H₃₄N₂]²⁺/2: 163.1356, found 163.1360.

A solution of 1,1'-(decane-1,10-diyl)*bis*(4-methylpyridin-1-ium) dibromide (243 mg, 0.50 mmol) and 4-(diethylamino)benzaldehyde (156 mg, 0.88 mmol) in n-butanol (2. mL) was treated with four drops of piperidine and the reaction mixture was stirred under reflux overnight, and then concentrated under reduced pressure. The resulting black oily residue was purified by automatic flash column chromatography (CH₂Cl₂ / MeOH 100:0 to 95:5). The fractions containing the desired product were evaporated and the resulting oily residue was dissolved in hot isopropanol (15 mL) and treated with Et₂O (2 × 40 mL), drawing off the liquids. After drying the residue in vacuum, 1,1'-(decane-1,10-diyl)*bis*{4-[(E)-4-(diethylamino)styryl]pyridin-1-ium} dibromide (132 mg, 33%) was obtained as a red solid; mp: 157-159 °C; IR (ATR) v: 3404, 2969, 2920, 1646, 1572, 1521, 1471, 1434, 1403, 1348, 1325, 1272, 1192, 1169, 1150, 1011, 827, 704, 572, 561 cm⁻¹; ¹H NMR (400 MHz, CD₃OD) δ: 1.21 (t, *J* = 7.2 Hz, 12H), 1.31-1.42 (m, 12H), 1.96 (tt, *J = J*' = 7.2 Hz, 4H), 3.48 (q, *J =* 7.2 Hz, 8H), 4.40 (t, *J =* 7.2 Hz, 4H), 6.76 (d, *J* = 8.8 Hz, 4H), 7.05 (d, *J* = 16.0 Hz, 2H), 7.59 (d, *J* = 8.8 Hz, 4H), 7.83 (d, *J* = 16.0 Hz, 2H), 7.95 (d, *J* = 6.8 Hz, 4H), 8.55 (d, *J* = 6.8 Hz, 4H); HRMS-ESI+ *m*/*z* calculated for [C₄₄H₆₀N₄]²⁺/2: 322.2404, found: 322.2395.

### 8.1.17 Synthesis of 1,1'-(decane-1,10-diyl)bis{3-bromo-4-[(E)-4-(diethylamino)styryl]pyridin-1-ium} dibromide (PRC-42)

A mixture of 1,10-dibromodecane (0.44 mL, 587 mg, 1.96 mmol) and 3-bromo-4-methylpyridine (0.49 mL, 759 mg, 4.41 mmol) was heated to 120 °C for 3 h. Then, isopropanol (2 mL) was added and the reaction mixture was stirred under reflux for 1 h. The mixture was allowed to cool down to room temperature, the resulting brown residue was washed with ice-cold Et₂O (2 × 20 mL) and the remaining bluish sticky oil was dried in vacuo, taken up in MeOH (1 mL) and treated with cold Et₂O (2 × 20 mL), drawing off the liquids. After drying the residue in vacuum, 1,1'-(decane-1,10-diyl)bis(3-bromo-4-methylpyridin-1-ium) dibromide (605 mg, 48%) was obtained as a brown oil that solidified on standing; mp: 63-64 °C; IR (ATR) v: 3454, 3387, 2988, 2921, 2851, 1632, 1497, 1465, 1375, 1317, 1186, 1174, 1084, 1031, 864, 719, 697, 579 cm⁻¹; ¹H NMR (400 MHz, DMSO-d₆) δ: 1.19-1.32 (m, 12H), 1.90 (tt, *J* = *J*' = 7.2 Hz, 4H), 2.61 (s, 6H), 4.54 (t, *J =* 7.2 Hz, 4H), 8.16 (d, *J =* 6.4 Hz, 2H), 9.04 (dd, *J* = 6.4 Hz, *J*' = 1.6 Hz, 2H), 9.49 (d, *J* = 1.6 Hz, 2H); HRMS-ESI+ m/z calculated for [C₂₂H₃₂Br₂N₂]²⁺/2: 241.0461, found 241.0453.

A solution of 1,1'-(decane-1,10-diyl)*bis*(3-bromo-4-methylpyridin-1-ium) dibromide (322 mg, 0.50 mmol) and 4-(diethylamino)benzaldehyde (195 mg, 1.10 mmol) in n-butanol (2 mL) was treated with four drops of piperidine and the reaction mixture was stirred under reflux overnight, and then concentrated under reduced pressure. The resulting black oily residue was purified by automatic flash column chromatography (CH₂Cl₂ / MeOH 100:0 to 90:10), to provide 1,1'-(decane-1,10-diyl)*bis*{3-bromo-4-[(*E*)-4-(diethylamino)styryl]pyridin-1-ium} dibromide (45 mg, 9%) as a purple solid; mp: 136-138 °C; IR (ATR) v: 3396, 2969, 2924, 2852, 1634, 1567, 1520, 1454, 1406, 1350, 1293, 1269, 1171, 1149, 1075, 1011, 806, 689, 567 cm⁻¹; ¹H NMR (400 MHz, CD₃OD) δ: 1.22 (t, *J* = 7.2 Hz, 12H), 1.31-1.43 (m, 12H), 1.97 (tt, *J = J*' = 7.6 Hz, 4H), 3.51 (q, *J =* 7.2 Hz, 8H), 4.39 (t, *J* = 7.6 Hz, 4H), 6.79 (d, *J =* 9.2 Hz, 4H), 7.24 (d, *J* = 16.0 Hz, 2H), 7.64 (d, *J* = 9.2 Hz, 4H), 7.94 (d, *J* = 16.0 Hz, 2H), 8.23 (d, *J* = 6.8 Hz, 2H), 8.52 (br d, *J* = 6.8 Hz, 2H), 9.02 (d, *J* = 1.2 Hz, 2H); HRMS-ESI+ *m*/*z* calculated for [C₄₄H₅₈Br₂N₄]²⁺/2: 400.1509, found: 400.1512.

### 8.1.18 Synthesis of 1,1'-(decane-1,10-diyl)bis{3-chloro-4-[(E)-4-(diethylamino)styryl]pyridin-1-ium} dibromide (PRC-50)

A mixture of 1,10-dibromodecane (0.44 mL, 587 mg, 1.96 mmol) and 3-chloro-4-methylpyridine (484 mg, 3.79 mmol) was heated to 120 °C for 3 h. Then, isopropanol (2 mL) was added and the reaction mixture was stirred under reflux for 1 h. The mixture was allowed to cool down to room temperature, the resulting brown residue was washed with ice-cold Et₂O (2 × 20 mL) and the remaining bluish sticky oil was dried in vacuo, taken up in MeOH (1 mL) and treated with cold Et₂O (2 × 20 mL), drawing off the liquids. After drying the residue in vacuum, 1,1'-(decane-1,10-diyl)bis(3-chloro-4-methylpyridin-1-ium) dibromide (614 mg, 58%) was obtained as a bluish oil that solidified on standing to a light pink solid; mp: 167-168 °C; IR (ATR) v: 3411, 2945, 2924, 2896, 2859, 1638, 1504, 1468, 1449, 1366, 1311, 1250, 1219, 1200, 1169, 1099, 1025, 1007, 955, 865, 769, 726, 703, 596 cm⁻¹; ¹H NMR (400 MHz, DMSO-d₆) δ: 1.21-1.31 (m, 12H), 1.90 (tt, *J* = *J*' = 7.6 Hz, 4H), 2.61 (s, 6H), 4.54 (t, *J* = 7.6 Hz, 4H), 8.18 (d, *J =* 6.4 Hz, 2H), 9.01 (dd, *J* = 6.4 Hz, *J*' = 1.6 Hz, 2H), 9.43 (d, *J* = 1.6 Hz, 2H); HRMS-ESI+ m/z calculated for [C₂₂H₃₂Cl₂N₂]²⁺/2: 197.0966, found 197.0967.

A solution of 1,1'-(decane-1,10-diyl)bis(3-chloro-4-methylpyridin-1-ium) dibromide (222 mg, 0.40 mmol) and 4-(diethylamino)benzaldehyde (156 mg, 0.88 mmol) in n-butanol (1,6. mL) was treated with four drops of piperidine and the reaction mixture was stirred under reflux overnight, and then concentrated under reduced pressure. The resulting black oily residue was purified by automatic flash column chromatography (CH₂Cl₂ / 7N ammonia solution in MeOH 100:0 to 90:10), to provide 1,1'-(decane-1,10-diyl)bis{3-chloro-4-[(*E*)-4-(diethylamino)styryl]pyridin-1-ium} dibromide (54 mg, 15%) as a purple solid; mp: 124-126 °C; IR (ATR) v: 3396, 2967, 2924, 2852, 1633, 1572, 1520, 1455, 1405, 1349, 1294, 1269, 1176, 1150, 1074, 1037, 1012, 972, 917, 808, 699, 612, 570 cm⁻¹; ¹H NMR (500 MHz, CD₃OD) *δ*: 1.22 (t, *J* = 7.0 Hz, 12H), 1.32-1.42 (m, 12H), 1.97 (tt, *J* = *J*' = 7.5 Hz, 4H), 3.51 (q, *J =* 7.0 Hz, 8H), 4.39 (t, *J* = 7.5 Hz, 4H), 6.79 (d, *J =* 9.0 Hz, 4H), 7.25 (d, *J =* 16.0 Hz, 2H), 7.64 (d, *J* = 9.0 Hz, 4H), 7.96 (d, *J* = 16.0 Hz, 2H), 8.26 (d, *J* = 6.5 Hz, 2H), 8.49 (br d, *J =* 6.5 Hz, 2H), 8.91 (d, *J* = 1.5 Hz, 2H); HRMS-ESI+ *m*/*z* calculated for [C₄₄H₅₈Cl₂N₄]²⁺/2: 356.2014, found: 356.2020.

### 8.1.19 Synthesis of 1,1'-(decane-1,10-diyl)bis{4-[(E)-4-(diethylamino)-2-hydroxystyryl]-3-methylpyridin-1-ium} dibromide (PRC-69)

Compound PRC-69 was prepared following the first steps and conditions as described for the synthesis of compound AID-X-2020 (section 1.1.2 of EXAMPLE 1 - 1^{st} paragraph) until 1,1'-(decane-1,10-diyl)*bis*(3,4-dimethylpyridin-1-ium) dibromide was obtained.

A solution of 1,1'-(decane-1,10-diyl)*bis*(3,4-dimethylpyridin-1-ium) dibromide (307 mg, 0.60 mmol) and 5-(diethylamino)-2-formylphenyl acetate (309 mg, 1.31 mmol) in *n*-butanol (3 mL) was treated with nine drops of DBU and the reaction mixture was stirred under microwave irradiation at 140 °C, 1 bar, 80 W for 15 min. Then, it was concentrated under reduced pressure and the resulting black oily residue was purified by automatic flash column chromatography (CH₂Cl₂ / isopropanol 90:10 to 75:25). The fractions containing the desired product were treated with Cs₂CO₃ in MeOH, until the colour changed from red to blue. The solution was evaporated under reduced pressure and the solid was suspended in CH₂Cl₂. The suspension was filtered, the filtrate was evaporated, and the resulting solid was washed with EtOAc (5 × 10 mL), drawing off the liquids. After drying the residue in vacuum, 1,1'-(decane-1,10-diyl)bis{4-[(E)-4-(diethylamino)-2-hydroxystyryl]-3-methylpyridin-1-ium} dibromide (12 mg, 2%) was obtained as a blue solid; mp: 169-171 °C; IR (ATR) v: 3277, 2924, 2853, 1556, 1512, 1395, 1373, 1350, 1243, 1186, 1104, 1075, 1011, 962, 771, 682 cm⁻¹; ¹H NMR (400 MHz, CD₃OD) *δ*: 1.18 (t, *J =* 7.2 Hz, 12H), 1.23-1.40 (m, 12H), 1.89 (m, 4H), 2.37 (s, 6H), 3.41 (q, *J* = 7.2 Hz, 8H), 4.18 (t, *J =* 7.2 Hz, 4H), 6.01 (d, *J =* 2.0 Hz, 2H), 6.16 (dd, *J* = 9.2 Hz, *J*' = 2.0 Hz, 2H), 6.89 (d, *J =* 15.2 Hz, 2H), 7.45 (dm, *J* = 9.2 Hz, 2H), 7.89 (d, *J =* 7.2 Hz, 2H), 8.05 (d, *J =* 7.2 Hz, 2H), 8.11 (br s, 2H), 8.30 (d, *J* = 15.2 Hz, 2H); HRMS-ESI+ *m*/*z* calculated for [C₄₆H₆₄N₄O₂]²⁺/2: 352.2509, found: 352.2514.

### 8.1.20 P. falciparum growth inhibition assay

The assay on *P. falciparum* growth inhibition for compounds EMA357, EMA359, EMA366, EMA368, EMA377, PRC-5, PRC-8, PRC-14, PRC-15, PRC-20, PRC-25, PRC-28, PRC-29, PRC-31, PRC-39, PRC-42, and PRC-50was performed following the same steps and conditions used in section 1.1.2 of EXAMPLE 1. The results obtained were then compared to the values previously obtained for AID-X-2020 (IC₅₀ = 0.09 µM).

### 8.2 Results

The results obtained indicated that all the compounds exhibit good antimalarial activity, especially EMA357, EMA359, EMA377, PRC-8, PRC-14, PRC-15, PRC-25, PRC-39, PRC-42, and PRC-50, which have a potent antimalarial activity, similar to AID-X-2020. Particularly, PRC-25 shows the highest antimalarial activity among the tested compounds, with an IC₅₀ of 47 nM (Table 4).

**Table 4. In vitro antimalarial activity of EMA357, EMA359, EMA366, EMA368, EMA377, PRC-5, PRC-8, PRC-14, PRC-15, PRC-20, PRC-25, PRC-28, PRC-29, PRC-31, PRC-39, PRC-42, and PRC-50.**

| **Compound** | **IC₅₀ in 3D7 strain (µM)** |
|---|---|
| EMA357 | 0.12 |
| EMA359 | 0.11 |
| EMA366 | 0.52 |
| EMA368 | 0.29 |
| EMA377 | 0.06 |
| PRC-5 | 0.42 |
| PRC-8 | 0.14 |
| PRC-14 | 0.06 |
| PRC-15 | 0.06 |
| PRC-20 | 0.20 |
| PRC-25 | 0.05 |
| PRC-28 | 0.25 |
| PRC-29 | 0.46 |
| PRC-31 | 0.54 |
| PRC-39 | 0.15 |
| PRC-42 | 0.10 |
| PRC-50 | 0.10 |
| AID-X-2020 | 0.09 |

### EXAMPLE 9: AID-X-2020 is also active against chloroquine- and artemisinin-resistant P. falciparum strains

The aim of this experiment was to assess whether AID-X-2020 is active against chloroquine- and artemisinin-resistant *P*. *falciparum* strains, and thus to evaluate the potential of a new antimalarial resistant drug compared to existing antimalarials.

### 9.1 Materials and Methods

### 9.1.1 P. falciparum growth inhibition assays

*P. falciparum* parasites of chloroquine-sensitive (3D7 strain) and chloroquine- and artemisinin-resistant strains were processed as described in section 1.1.3 of EXAMPLE 1.

In particular, the *P*. *falciparum* resistant strains were the following: (1) strain W2 MRA-157 (BEI Resources, managed by ATCC) which is chloroquine-resistant; (2) strains 3D7 harboring the K13 mutations associated to artemisinin resistance, either M579I or R561H (both provided by Dr. David A. Fidock and referenced in Stokes, B.H. et al., 2021); (3) Cam 3.II strain which is chloroquine and sulfadoxine/pyrimethamine-resistant; and (4) Cam 3.II strain modified to carry K13 mutations associated to artemisinin-resistant strains, either R561H or R539T, therefore being multiresistant strains. All Cam 3.II strains (3) and (4) were provided by Dr. David A. Fidock and are referenced in Straimer, J. et al., 2015 and Stokes, B.H. et al., 2021.

Then, the required amount of AID-X-2020 was added to each well at different concentrations (0.0001, 0.001, 0.01, 1 and 10 µM) and triplicates.

The effect of AID-X-2020 of chloroquine- and artemisinin-resistant strains was compared with the activity of AID-X-2020 of *P*. *falciparum* parasites of chloroquine-sensitive 3D7 strain.

### 9.2 Results

Results confirm that AID-X-2020 was also strongly active against the chloroquine-resistant W2 strain (IC₅₀ of 90 ± 1 nM), and several artemisinin-resistant strains with IC₅₀ values ranging from 90 to 160 nM, in comparison to parental 3D7 and Cam3.ll strains (FIG. 5). The results do not show a significant IC₅₀ increase relative to the corresponding parental strains except for M579I (*p* value of 0.02; all other p values > 0.15), in agreement with a mode of action for AID-X-2020 different from that of artemisinin. This result is also consistent with the calculated IC₅₀ of AID-X-2020 in the chloroquine-resistant *P*. *falciparum* W2 strain (FIG. 5(A)), undistinguishable from its activity in the chloroquine-sensitive 3D7 strain.

Results suggest that resistance is slowed down significantly for AID-X-2020, a molecule belonging to a chemical family where no antimalarials have been described so far. Thus, the sensitivity to AID-X-2020 of the chloroquine-resistant W2 strain indicates that the antimalarial mode of action of AID-X-2020 is not related to that of chloroquine. Similarly, data showing a sensitivity of artemisinin-resistant strains to AID-X-2020 similar to that of the corresponding parental non-resistant lines, strongly suggest that the antimalarial modes of action of both drugs are not related. Indeed, an antagonistic action of artemisinin and AID-X-2020 was observed in *P. falciparum* cultures, indicating that their main effects on the pathogen are opposed (EXAMPLE 5). Therefore, in this scenario, it can be concluded that AID-X-2020 is a potential new antimalarial resistant drug compared to the existing antimalarials.

### EXAMPLE 10: AID-X-2020 is also able to disassemble preformed Aβ40 fibrils

EXAMPLE 3 confirmed that AID-X-2020 is a strong inhibitor of the aggregation of Aβ40 in formation, suggesting that inhibition of protein aggregation was the responsible mechanism for the antimalarial activity of this compound. In the present example, inventors further tested the possibility of disaggregation of already formed aggregative peptides.

### 10.1 Materials and Methods

### 10.1.1 In vitro assay of aggregation and disaggregation of aggregative peptides

To test the effect of AID-X-2020 on already formed amyloid fibrils, Aβ40 DMSO solutions were diluted to 25 µM in PBS and incubated as above in order to allow fibril formation. Then, AID-X-2020 was added at different concentrations (0.1 µM AID-X-2020, 1 µM AID-X-2020 and 10 µM AID-X-2020) and the mixture was incubated in the same conditions for another 24 h. The final samples always contained less than 5% DMSO to avoid interference of this solvent on Aβ40 amyloid fibril formation. Finally, ThT treatment and TEM analysis was performed as described in section 3.1 of EXAMPLE 3. The analysis of aggregation inhibition and disaggregation performed with aggregative peptides present in *P. falciparum* proteins were conducted in the same way.

Aggregation inhibition and disaggregation activities were also tested with six aggregative peptides present in *P. falciparum* proteins with SEQ ID NO: 1-6 (NVNIYN, LYWIYY, NFNNIYH, NNFYYNN, LISFIL, LQSNIG) in order to determine the effects on AID-X-2020 directly on *P. falciparum* peptides. AID-X-2020 was added in this case at 0.1 µM and 1 µM.

### 10.2 Results

AID-X-2020 at concentrations > 90 nM was also found to disassemble preformed Aβ40 fibrils (FIG. 6). Both *in vitro* aggregation inhibition and disaggregation assays show the presence of characteristic amorphous aggregates at 0.1 µM AID-X-2020, which might represent an intermediate species between mature Aβ40 fibrils and the disassembled protofibrillar structures found at higher drug concentrations. These aggregation inhibition and disaggregation activities were also observed with the six aggregative peptides present in *P. falciparum* proteins (FIG. 7).

Results confirm the *in vitro* activity of AID-X-2020 as inhibitor of the aggregation of a model amyloi-dogenic peptide like Aβ40 and of aggregative peptides present in *P. falciparum* proteins, as already demonstrated in EXAMPLES 3, 4 and 5. The present example further demonstrates the disaggre-gating activity of AID-X-2020, beyond its capacity to inhibit protein aggregation.

### EXAMPLE 11: Subcellular localization of AID-X-2020 in pRBCs

### 11.1 Materials and Methods

### 11.1.1 Fluorescence microscopy

For AID-X-2020 staining, a *P*. *falciparum* 3D7 culture was incubated in RPMIc for 30 min at 37 °C with 4.5 µM of the compound and 4 µg/ml of Hoechst 33342.

For colocalization studies, 0.5 µM of ER Tracker^{™} Green (BODIPY^{™} FL Glibenclamide, Thermo Fisher Scientific) was included in the solution. Cells were placed in an 8-well LabTek^{™} II chamber slide system (Thermo Fisher Scientific), rinsed with warm PBS and diluted 1:20 for their observation in a Leica TCS SP5 confocal microscope (Leica Camera, Mannheim, Germany) equipped with a 63× objective of 1.4 NA. Hoechst 33342 was excited with a diode laser at 405 nm, ER Tracker Green with the 488 nm line of an argon laser, and AID-X-2020 with a diode-pumped solid-state laser at 561 nm. The corresponding fluorescence emissions were collected in the ranges of, respectively, 415-460, 490-590, and 600-700 nm.

To avoid crosstalk between the different fluorescence signals, sequential line scanning was performed. To quantify Manders' overlap coefficient, images were analyzed using the Just Another Colocalization Plugin (JACoP) in the Fiji software. To avoid fixation artifacts, all the fluorescence microscopy data presented in this work were obtained with live cells.

### 11.1.2 Correlative light and electron microscopy (CLEM)

A 0.5% parasitemia RBC culture was prepared for CLEM by allowing its binding to concanavalin. Briefly, a µ-Dish 35 mm, High, Grid-500 (ibidi GmbH, Gräfelfing, Germany) was coated for 20 min at 37 °C with a 50 mg/ml concanavalin A solution in ddH₂O and wells were rinsed with pre-warmed PBS before parasite seeding. *P. falciparum-*infected RBCs washed twice with PBS were deposited into the dish and incubated for 10 min at 37 °C; afterwards, unbound RBCs were washed away with three PBS rinses. Seeded RBCs were then incubated with 3 µM AID-X-2020, and nuclei were counterstained with 2 µg/ml Hoechst 33342. The preparation was observed with a Zeiss LSM880 confocal microscope (Carl Zeiss, Jena, Germany), with respective λex/em for AID-X-2020 and Hoechst 33342 of 405/415-520 nm and 561/565-600 nm. Images were obtained from areas corresponding to a specific coordinate of the dish-grid by tile scans that were stitched into larger mosaics. A bright field image facilitated the recognition of the grid coordinates from the plate where the cells selected for CLEM were located.

After confocal image acquisition, cells were washed three times with TEM fixation buffer (2% paraformaldehyde and 2.5% glutaraldehyde in PBS) for 5 min each. Then, the fixation buffer was changed to 1% osmium tetroxide and 0.8% potassium ferricyanide in fixation buffer and incubated at 4 °C for 45 min, followed by three 5-min washes with ddH₂O. Then, a dehydration procedure was performed by gradually increasing ethanol concentration: 50% (10 min), 70% (10 min), 80% (10 min), 90% (5 min, 3×), 96% (5 min, 3×), and 100% (5 min, 3×). At this point, the plastic part of the dish was carefully separated from the crystal part containing the samples, which was embedded in Spurr resin by successive incubations with different proportions of resin/ethanol, starting with 1/3 for 1 h, 1/1 for 1 h, 3/1 for 1 h and 1/0 overnight. After the embedding procedure, a BEEM^{®} capsule containing polymerized Spurr resin was filled with a small volume of liquid resin in order to obtain an interphase in which the dish was placed. The BEEM^{®} capsule was incubated at 70 °C for 72 h, and the crystal part of the dish was removed by alternatively immersing samples in liquid nitrogen and boiling water. When the crystal was broken, cells remained attached to the resin, which was further cut in a microtome with a diamond blazer in order to obtain 100 nm-thick resin slides, which were mounted on a carbon-coated copper grid and negatively stained with 2% uranyl acetate for 2 min and washed with ddH₂O for 1 min. Samples were observed in a JEM 1010 transmission electron microscope. Images were processed for CLEM analysis using the CORRELIA plug-in in the Fiji software (version 2.0.0-pre-8).

### 11.2 Results

Confocal fluorescence microscopy colocalization analysis (FIG. 8(A)) and correlative light and electron microscopy data (FIG. 8(B)) confirmed the presence of AID-X-2020 mainly in the parasite's cytosol, particularly in association with endoplasmic reticulum regions.

The cytosolic localization of the protein aggregation inhibitor AID-X-2020 in *Plasmodium* rough ER regions, where proteins are being synthesized, is consistent with the likely role of this drug in disrupting a yet to be described parasite's aggresome.

### EXAMPLE 12: Quantitative analysis of protein aggregation in live Plasmodium cells after treatment with AID-X-2020

The aim of this experiment was to directly prove the level of protein aggregation in live *Plasmodium* cells following AID-X-2020 treatment.

### 12.1 Materials and Methods

12.1.1 Quantitative analysis of protein aggregation in live *P*. *falciparum* cultures To directly probe the level of protein aggregation in live *Plasmodium* cells following AID-X-2020 treatment, a ThT-based method to measure protein aggregation in parasite cultures was developed.

*P. falciparum* cultures enriched in early stages were treated with the IC₁₀ (27 nM) and IC₅₀ (90 nM) of AID-X-2020 or left untreated. After 90 min, 4 h and 30 h, a Percoll purification was done in order to isolate parasitized cells from uninfected RBCs. After Percoll purification, the pellets of late stage parasites and a control non-infected RBC suspension containing the same proportion of cells than the purified cultures were resuspended in 50 µl of lysis buffer (4.5 mg/ml NaCl in water supplemented with EDTA-free protease inhibitor cocktail, PIC, Hoffman-La Roche, Basel, Switzerland; 1 PIC tablet/10 ml water) and incubated overnight, at 4 °C under stirring, with the objective of releasing their inner content. After this time, lysed samples were spun down and the protein content in the supernatant was quantified with the bicinchoninic acid assay (Thermo Fisher Scientific), following the manufacturer's instructions. 30 µg of protein from each supernatant were further diluted with PBS to a final volume of 70 µl and plated on a 96-well black plate in triplicates. ThT fluorescence was measured as described above.

### 12.2 Results

ThT fluorescence of culture extracts, normalized to have equal protein content, exhibited a reduced emission spectrum in samples that had been treated for only 90 min with 90 nM AID-X-2020, the compound's *in vitro* IC₅₀ (FIG. 9). After 4 h of treatment, the decrease in ThT fluorescence was more evident, even for cultures treated with the IC₁₀ of AID-X-2020 (27 nM). This reduced signal could still be clearly detected after 30 h of 90 nM treatment (FIG. 10).

These results indicating a relevant decrease in aggregated protein load in live parasites following AID-X-2020 treatment at physiologically relevant concentrations are supportive of a mode of action of this drug consisting in the inhibition of protein aggregation in the pathogen.

### EXAMPLE 13: Activity of AID-X-2020 on Plasmodium gametocytes

EXAMPLE 6 studied the inhibition of the gametocyte to ookinete transition in order to determine whether AID-X-2020 was able to arrest the life cycle of malaria parasites in the mosquito host.

Contrarily, the objective of the present study was to determine if AID-X-2020 is capable of targeting gametocytes, the sexual phase of the malaria parasites' life cycle and the sole stage of malaria parasites present in the blood circulation capable of transmitting the infection to the mosquito vector, and where drugs active at this critical step are scarce.

### 13.1 Materials and Methods

Cultures of the *P. falciparum NF54-gexp02-Tom* strain (developed and authenticated by Portugaliza, H.P. et al., 2019 and kindly provided by Prof. Alfred Cortés), were maintained in standard conditions in RPMI medium supplemented with 0.5% Albumax II and 2 mM choline, synchronized in ring stages with sorbitol lysis, and diluted to 2% parasitemia. To trigger sexual conversion, choline was removed from the medium and cultures were maintained in the same conditions for 48 h after synchronization (cycle 0). In the next cycle (cycle 1), parasites were treated with 50 mM N-acetylglucosamine (GlcNac) in order to kill asexual parasites, and maintained in RPMI supplemented with 10% human serum. Medium was refreshed daily and GlcNac was kept during 4 days.

To determine the effect of AID-X-2020 and DONE3TCI in early gametocytes, the culture was distributed in triplicates (200 µl/well, 96-well plates) and drugs were added in cycle 1 and maintained for 48 h in the culture. Controls of untreated parasites as well as of parasites treated with a lethal dose of chloroquine were prepared. Gametocytemia was monitored daily by light microscopy until the majority of parasites (~ 90%) could be identified as stage V gametocytes. At that point, Giemsa smears of each well were prepared and mature gametocytes were manually counted (10,000 cells were counted for each replica by two investigators blinded to group assignment). To test the effect of AID-X-2020 and DONE3TCI on mature gametocytes, cultures were grown for 14 days, when the majority of the parasites could be identified as stage V gametocytes. Afterwards, the culture was treated for 48 h with the drugs and the gametocytemia determined as above.

### 13.2 Results

AID-X-2020 efficiently blocked the development of *P. falciparum* early and mature stage V gametocytes *in vitro* with respective IC₅₀ of 95 ± 3 nM and 103 ± 3 nM (FIG. 11), close to that obtained for the asexual blood stages (EXAMPLE 6). For the amyloid pan-inhibitor aminoquinoline DONE3TCI the IC₅₀ values on early and late gametocytes were 285 ± 56 nM and 78 ± 12 nM, respectively.

Therefore, besides its activity against *Plasmodium* asexual blood stages, AID-X-2020 displays marked activity against the sexual phase of the malaria parasites' life cycle, paving the way for the exploration of this drug as a potential multi-stage antiplasmodial therapy.

### EXAMPLE 14: Microscopic observation of clinical samples

### 14.1 Materials and Methods

Clinical malaria samples were provided by the *Centre de Salut Internacional i Malalties Transmissibles Drassanes-Vall d'Hebron.* Five µL of blood from a malaria-infected person were diluted in 100 µL of RPMIc. The solution was stained with 2 µg/mL Hoechst 33342 and 18 µM AID-X-2020 and incubated for 30 min before centrifuging at 5000× g for 30 s and discarding the supernatant. The pellet was then resuspended in 100 µL of PBS and 5 µL of the suspension were placed on a 8 well slide (lbidi) with 200 µL of PBS. The sample was observed in a IX-51 Olympus fluorescence microscope with a 100× objective and a SPRED filter (λex: 586/20 nm, λem: 628/32 nm).

### 14.2 Results

Microscopic observation of AID-X-2020-stained clinical blood samples of a *P. falciparum* infection allowed the identification of the circulating ring forms of the parasite that are detected in malaria diagnosis.

### REFERENCES

WO2011065980A2

Vlachou, D.; Zimmermann, T.; Cantera, R.; Janse, C. J.; Waters, A. P.; Kafatos, F. C. Real-time, in vivo analysis of malaria ookinete locomotion and mosquito midgut invasion. Cell. Microbiol. 2004, 6 (7), 671-685.

Stokes, B.H., Dhingra, S.K., Rubiano, K., Mok, S., Straimer, J., Gnädig, N.F., et al. Plasmodium falciparum K13 mutations in Africa and Asia impact artemisinin resistance and parasite fitness. eLife 2021;10.

Straimer, J., Gnädig, N.F., Witkowski, B., Amaratunga, C., Duru, V., Ramadani, A.P., Dacheux, M., Khim, N., Zhang, L., Lam, S., Gregory, P.D., Urnov, F.D., Mercereau-Puijalon, O., Benoit-Vical, F., Fairhurst, R.M., Ménard, D., Fidock, D.A. K13-propeller mutations confer artemisinin resistance in Plasmodium falciparum clinical isolates. Science 2015, 347:428- 431.

Portugaliza, H.P., Llorà-Batlle, O., Rosanas-Urgell, A., Cortés, A. Reporter lines based on the gexp02 promoter enable early quantification of sexual conversion rates in the malaria parasite Plasmodium falciparum. Sci Rep 2019, 9, 14595.

## Claims

1. A compound of formula (I) 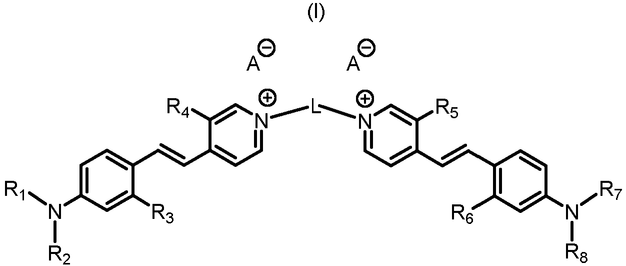 or a pharmaceutically acceptable salt thereof for use as a medicament, wherein:
A is an anion;
L is a diradical selected from the group consisting of 1,2-phenylenebis(methylene), 1,3-phenylenebis(methylene), 1,4-phenylenebis(methylene) and a (C₂-C₁₂)-oligomethylene chain, wherein one to three methylene groups of the (C₂-C₁₂)-oligomethylene chain can be substituted by oxygen atoms;
R₁, R₂, R₇, and R₈ are radicals independently selected from the group consisting of hydrogen, (C₁-C₄)-alkyl, hydroxy-(C₁-C₄)-alkyl, cyano-(C₁-C₄)-alkyl, (C₁-C₄)-alkanoyloxy-(C₁-C₄)-alkyl, phenyl, (C₁-C₄)-alkanoyl, (C₁-C₄)-alkanesulfonyl, benzenesulfonyl, naphthalenesulfonyl, and *p-*toluenesulfonyl, or alternatively, when taken in combination, R₁-N-R₂ and R₇-N-R₈ are independently selected from the group consisting of azetidine, pyrrolidine, piperidine, azepine, piperazine, 4-(C₁-C₂)-alkylpiperazine, and morpholine ring;
R₃ and R₆ are radicals independently selected from the group consisting of hydrogen, (C₁-C₄)-alkyl, nitro, amino, (C₁-C₄)-alkylamino, (C₁-C₄)-alkanamido, (C₁-C₄)-alkanesulfonamido, benzenesulfonamido, naphthalenesulfonamido, and p-toluenesulfonamido; and
R₄ and R₅ are radicals independently selected from the group consisting of hydrogen, (C₁-C₄)-alkyl, halogen, amino, (C₁-C₄)-alkylamino, (C₁-C₄)-alkanamido, (C₁-C₄)-alkanesulfonamido, benzenesulfonamido, naphthalenesulfonamido, p-toluenesulfonamido, amino-(C₁-C₄)-alkyl, (C₁-C₂)-alkylamino-(C₁-C₄)-alkyl, (C₁-C₄)-alkanamido-(C₁-C₄)-alkyl, (C₁-C₄)-alkanesulfonamido-(C₁-C₄)-alkyl, benzenesulfonamido-(C₁-C₄)-alkyl, naphthalenesulfonamido-(C₁-C₄)-alkyl, and p-toluenesulfonamido-(C₁-C₄)-alkyl.

2. The compound for use according to claim 1, wherein the compound is selected from the group consisting of:
1,1'-(decane-1,10-diyl)bis{3-methyl-4-[(*E*)-4-(pyrrolidin-1-yl)styryl]pyridin-1-ium} dibromide;
1,1'-(decane-1,10-diyl)bis{3-methyl-4-[(*E*)-4-(piperidin-1-yl)styryl]pyridin-1-ium} dibromide;
1,1'-(decane-1,10-diyl)bis{3-methyl-4-[(*E*)-4-morpholinostyryl]pyridin-1-ium} dibromide;
1,1'-(decane-1,10-diyl)bis{3-methyl-4-[(*E*)-4-(piperazin-1-yl)styryl]pyridin-1-ium} dibromide;
1,1'-(decane-1,10-diyl)bis{4-[(*E*)-4-(diphenylamino)styryl]-3-methylpyridin-1-ium} dibromide;
1,1'-(decane-1,10-diyl)*bis*{4-[(*E*)-4-(dimethylamino)styryl]-3-methylpyridin-1-ium} dibromide;
1,1'-(decane-1,10-diyl)*bis*{4-[(*E*)-4-(dimethylamino)-2-nitrostyryl]-3-methylpyridin-1-ium} dibromide;
1,1'-(decane-1,10-diyl)*bis*{4-{(*E*)-4-[(2-cyanoethyl)methylamino]styryl}-3-methylpyridin-1-ium} dibromide;
1,1'-(decane-1,10-diyl)*bis*{4-{(*E*)-4-[bis(2-acetoxyethyl)amino]styryl}-3-methylpyridin-1-ium} dibromide;
1,1'-(decane-1,10-diyl)*bis*{4-{(*E*)-4-[bis(2-hydroxyethyl)amino]styryl}-3-methylpyridin-1-ium} dibromide;
1,1'-(ethane-1,2-diyl)*bis*{4-[(*E*)-4-(diethylamino)styryl]-3-methylpyridin-1-ium} dibromide;
1,1'-(propane-1,3-diyl)*bis*{4-[(*E*)-4-(diethylamino)styryl]-3-methylpyridin-1-ium} dibromide;
1,1'-(butane-1,4-diyl)*bis*{4-[(*E*)-4-(diethylamino)styryl]-3-methylpyridin-1-ium} dibromide;
1,1'-(pentane-1,5-diyl)*bis*{4-[(*E*)-4-(diethylamino)styryl]-3-methylpyridin-1-ium} dibromide;
1,1'-(hexane-1,6-diyl)*bis*{4-[(*E*)-4-(diethylamino)styryl]-3-methylpyridin-1-ium} dibromide;
1,1'-(heptane-1,7-diyl)*bis*{4-[(*E*)-4-(diethylamino)styryl]-3-methylpyridin-1-ium} dibromide;
1,1'-(octane-1,8-diyl)*bis*{4-[(*E*)-4-(diethylamino)styryl]-3-methylpyridin-1-ium} dibromide;
1,1'-(nonane-1,9-diyl)*bis*{4-[(*E*)-4-(diethylamino)styryl]-3-methylpyridin-1-ium} dibromide;
1,1'-(decane-1,10-diyl)*bis*{4-[(*E*)-4-(diethylamino)styryl]-3-methylpyridin-1-ium} dibromide;
1,1'-(undecane-1,11-diyl)*bis*{4-[(*E*)-4-(diethylamino)styryl]-3-methylpyridin-1-ium} dibromide;
1,1'-(dodecane-1,12-diyl)*bis*{4-[(*E*)-4-(diethylamino)styryl]-3-methylpyridin-1-ium} dibromide;
1,1'-(3-oxapentane-1,5-diyl)*bis*{4-[(*E*)-4-(diethylamino)styryl]-3-methylpyridin-1-ium} dichloride;
1,1'-(3,6-dioxaoctane-1,8-diyl)*bis*{4-[(*E*)-4-(diethylamino)styryl]-3-methylpyridin-1-ium} diiodide;
1,1'-(3,6,9-trioxaundecane-1,11-diyl)*bis*{4-[(*E*)-4-(diethylamino)styryl]-3-methylpyridin-1-ium} dichloride;
1,1'-[1,2-phenylenebis(methylene)]*bis*{4-[(*E*)-4-(diethylamino)styryl]-3-methylpyridin-1-ium} dibromide;
1,1'-[1,3-phenylenebis(methylene)]*bis*{4-[(*E*)-4-(diethylamino)styryl]-3-methylpyridin-1-ium} dibromide;
1,1'-[1,4-phenylenebis(methylene)]*bis*{4-[(*E*)-4-(diethylamino)styryl]-3-methylpyridin-1-ium} dibromide;
1,1'-(decane-1,10-diyl)*bis*{4-[(*E*)-4-(diethylamino)styryl]pyridin-1-ium} dibromide;
1,1'-(decane-1,10-diyl)*bis*{3-bromo-4-[(*E*)-4-(diethylamino)styryl]pyridin-1-ium} dibromide;
1,1'-(decane-1,10-diyl)*bis*{3-chloro-4-[(*E*)-4-(diethylamino)styryl]pyridin-1-ium} dibromide; and
1,1'-(decane-1,10-diyl)*bis*{3-amino-4-[(*E*)-4-(diethylamino)styryl]pyridin-1-ium} dibromide.

3. The compound for use according to claim 2, wherein the compound is of formula (VI) 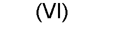 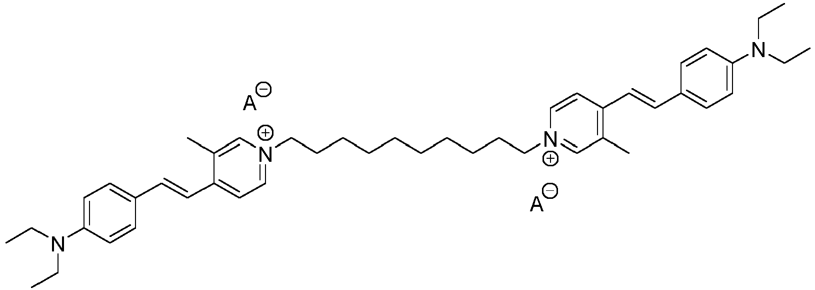 or a pharmaceutically acceptable salt thereof.

4. The compound for use according to claim 3, wherein A is bromide.

5. A compound of formula (I) 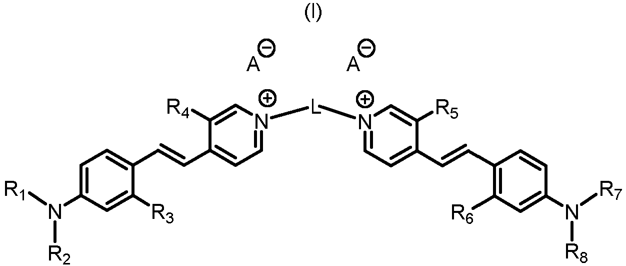 or a pharmaceutically acceptable salt thereof for use in the treatment, prevention or amelioration of malaria, or symptoms, complications and/or sequelae thereof, wherein:
A is an anion;
L is a diradical selected from the group consisting of 1,2-phenylenebis(methylene), 1,3-phenylenebis(methylene), 1,4-phenylenebis(methylene) and a (C₂-C₁₂)-oligomethylene chain, wherein one to three methylene groups of the (C₂-C₁₂)-oligomethylene chain can be substituted by oxygen atoms;
R₁, R₂, R₇, and R₈ are radicals independently selected from the group consisting of hydrogen, (C₁-C₄)-alkyl, hydroxy-(C₁-C₄)-alkyl, cyano-(C₁-C₄)-alkyl, (C₁-C₄)-alkanoyloxy-(C₁-C₄)-alkyl, phenyl, (C₁-C₄)-alkanoyl, (C₁-C₄)-alkanesulfonyl, benzenesulfonyl, naphthalenesulfonyl, and *p-*toluenesulfonyl, or alternatively, when taken in combination, R₁-N-R₂ and R₇-N-R₈ are independently selected from the group consisting of azetidine, pyrrolidine, piperidine, azepine, piperazine, 4-(C₁-C₂)-alkylpiperazine, and morpholine ring;
R₃ and R₆ are radicals independently selected from the group consisting of hydrogen, (C₁-C₄)-alkyl, nitro, amino, (C₁-C₄)-alkylamino, (C₁-C₄)-alkanamido, (C₁-C₄)-alkanesulfonamido, benzenesulfonamido, naphthalenesulfonamido, and *p*-toluenesulfonamido; and
R₄ and R₅ are radicals independently selected from the group consisting of hydrogen, (C₁-C₄)-alkyl, halogen, amino, (C₁-C₄)-alkylamino, (C₁-C₄)-alkanamido, (C₁-C₄)-alkanesulfonamido, benzenesulfonamido, naphthalenesulfonamido, *p*-toluenesulfonamido, amino-(C₁-C₄)-alkyl, (C₁-C₂)-alkylamino-(C₁-C₄)-alkyl, (C₁-C₄)-alkanamido-(C₁-C₄)-alkyl, (C₁-C₄)-alkanesulfonamido-(C₁-C₄)-alkyl, benzenesulfonamido-(C₁-C₄)-alkyl, naphthalenesulfonamido-(C₁-C₄)-alkyl, and p-toluenesulfonamido-(C₁-C₄)-alkyl.

6. A pharmaceutical composition comprising an effective amount of a compound of formula (I) 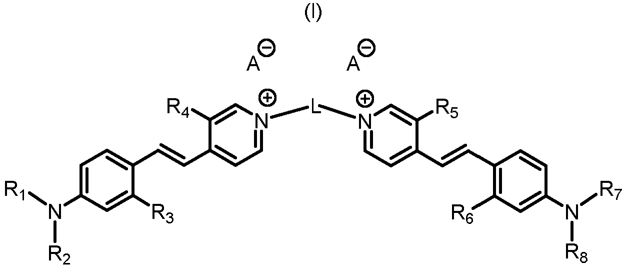
or a pharmaceutically acceptable salt thereof, wherein:
A is an anion;
L is a diradical selected from the group consisting of 1,2-phenylenebis(methylene), 1,3-phenylenebis(methylene), 1,4-phenylenebis(methylene) and a (C₂-C₁₂)-oligomethylene chain, wherein one to three methylene groups of the (C₂-C₁₂)-oligomethylene chain can be substituted by oxygen atoms;
R₁, R₂, R₇, and R₈ are radicals independently selected from the group consisting of hydrogen, (C₁-C₄)-alkyl, hydroxy-(C₁-C₄)-alkyl, cyano-(C₁-C₄)-alkyl, (C₁-C₄)-alkanoyloxy-(C₁-C₄)-alkyl, phenyl, (C₁-C₄)-alkanoyl, (C₁-C₄)-alkanesulfonyl, benzenesulfonyl, naphthalenesulfonyl, and *p-*toluenesulfonyl, or alternatively, when taken in combination, R₁-N-R₂ and R₇-N-R₈ are independently selected from the group consisting of azetidine, pyrrolidine, piperidine, azepine, piperazine, 4-(C₁-C₂)-alkylpiperazine, and morpholine ring;
R₃ and R₆ are radicals independently selected from the group consisting of hydrogen, (C₁-C₄)-alkyl, nitro, amino, (C₁-C₄)-alkylamino, (C₁-C₄)-alkanamido, (C₁-C₄)-alkanesulfonamido, benzenesulfonamido, naphthalenesulfonamido, and *p*-toluenesulfonamido; and
R₄ and R₅ are radicals independently selected from the group consisting of hydrogen, (C₁-C₄)-alkyl, halogen, amino, (C₁-C₄)-alkylamino, (C₁-C₄)-alkanamido, (C₁-C₄)-alkanesulfonamido, benzenesulfonamido, naphthalenesulfonamido, *p*-toluenesulfonamido, amino-(C₁-C₄)-alkyl, (C₁-C₂)-alkylamino-(C₁-C₄)-alkyl, (C₁-C₄)-alkanamido-(C₁-C₄)-alkyl, (C₁-C₄)-alkanesulfonamido-(C₁-C₄)-alkyl, benzenesulfonamido-(C₁-C₄)-alkyl, naphthalenesulfonamido-(C₁-C₄)-alkyl, and *p-*toluenesulfonamido-(C₁-C₄)-alkyl,
together with appropriate amounts of pharmaceutically acceptable excipients or carriers.

7. A compound of formula (I) 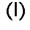 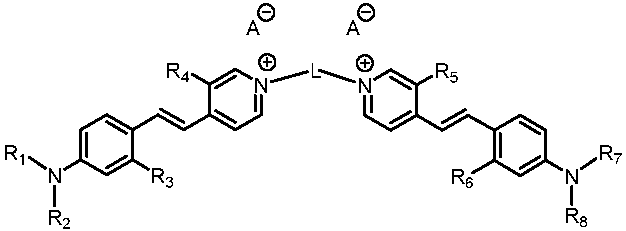 or a pharmaceutically acceptable salt thereof, wherein:
A is an anion;
L is a diradical selected from the group consisting of ethylene, trimethylene, tetramethylene, pentamethylene, hexamethylene, heptamethylene, octamethylene, nonamethylene, decamethylene, undecamethylene, dodecamethylene, 3-oxapentamethylene, 3,6-dioxaoctamethylene, 3,6,9-trioxaundecamethylene, 1,2-phenylene*bis*(methylene), 1,3-phenylenebis(methylene), and 1,4-phenylene*bis*(methylene);
R₁, R₂, R₇, and R₈ are radicals independently selected from the group consisting of hydrogen, (C₁-C₄)-alkyl, hydroxy-(C₁-C₄)-alkyl, cyano-(C₁-C₄)-alkyl, (C₁-C₄)-alkanoyloxy-(C₁-C₄)-alkyl, phenyl, (C₁-C₄)-alkanoyl, (C₁-C₄)-alkanesulfonyl, benzenesulfonyl, naphthalenesulfonyl, and *p-*toluenesulfonyl or alternatively, when taken in combination, R₁-N-R₂ and R₇-N-R₈ are independently selected from the group consisting of azetidine, pyrrolidine, piperidine, azepine, piperazine, 4-(C₁-C₂)-alkylpiperazine, and morpholine ring;
R₃ and R₆ are radicals independently selected from the group consisting of hydrogen, (C₁-C₄)-alkyl, nitro, amino, (C₁-C₄)-alkylamino, (C₁-C₄)-alkanamido, (C₁-C₄)-alkanesulfonamido, benzenesulfonamido, naphthalenesulfonamido, and p-toluenesulfonamido; and
R₄ and R₅ are radicals independently selected from the group consisting of hydrogen, (C₁-C₄)-alkyl, halogen, amino, (C₁-C₄)-alkylamino, (C₁-C₄)-alkanamido, (C₁-C₄)-alkanesulfonamido, benzenesulfonamido, naphthalenesulfonamido, *p*-toluenesulfonamido, amino-(C₁-C₄)-alkyl, (C₁-C₂)-alkylamino-(C₁-C₄)-alkyl, (C₁-C₄)-alkanamido-(C₁-C₄)-alkyl, (C₁-C₄)-alkanesulfonamido-(C₁-C₄)-alkyl, benzenesulfonamido-(C₁-C₄)-alkyl, naphthalenesulfonamido-(C₁-C₄)-alkyl, and *p-*toluenesulfonamido-(C₁-C₄)-alkyl;
with the proviso that when R₁, R₂, R₃, R₄, R₅, R₆, R₇ and R₈ are hydrogen, L is not 1,4-phenylene*bis*(methylene);
with the proviso that when R₁, R₂, R₇ and R₈ are methyl and R₃, R₄, R₅ and R₆ are hydrogen, L is not ethylene, trimethylene, tetramethylene, pentamethylene, hexamethylene, decamethylene, dodecamethylene, 3,6-dioxaoctamethylene, 1,2-phenylene*bis*(methylene), 1,3-phenylene*bis*(methylene) or 1,4-phenylenebis(methylene);
with the proviso that when R₁, R₂, R₇ and R₈ are ethyl and R₃, R₄, R₅ and R₆ are hydrogen, L is not trimethylene, tetramethylene, 3-oxapentamethylene, 1,2-phenylene*bis*(methylene) or 1,4-phenylene*bis*(methylene);
with the proviso that when R₁, R₂, R₇ and R₈ are phenyl and R₃, R₄, R₅ and R₆ are hydrogen, L is not trimethylene, tetramethylene, pentamethylene, hexamethylene, octamethylene, decamethylene, dodecamethylene, 1,2-phenylenebis(methylene), 1,3-phenylenebis(methylene) or 1,4-phenylene*bis*(methylene);
with the proviso that when R₁, R₂, R₇ and R₈ are HO-CH₂-CH₂- and R₃, R₄, R₅ and R₆ are hydrogen, L is not tetramethylene;
with the proviso that when R₁ and R₇ are ethyl, R₂ and R₈ are HO-CH₂-CH₂- and R₃, R₄, R₅ and R₆ are hydrogen, L is not ethylene, trimethylene, tetramethylene, pentamethylene, hexamethylene or heptamethylene;
with the proviso that when R₁ and R₇ are methyl, R₂ and R₈ are NC-CH₂- and R₃, R₄, R₅ and R₆ are hydrogen, L is not 1,2-phenylene*bis*(methylene) or 1,4-phenylenebis(methylene),
with the proviso that when R₁ and R₇ are acetyl, R₂, R₃, R₄, R₅ R₆ and R₈ are hydrogen, L is not 1,4-phenylene*bis*(methylene);
with the proviso that when R₁, R₂, R₃, R₆, R₇ and R₈ are methyl and R₄ and R₅ are hydrogen, L is not trimethylene or 1,4-phenylene*bis*(methylene);
with the proviso that when R₁, R₂, R₄, R₅, R₇ and R₈ are methyl and R₃ and R₆ are hydrogen, L is not trimethylene, tetramethylene or decamethylene;
with the proviso that when R₁, R₂, R₇ and R₈ are ethyl, R₃ and R₆ are hydrogen and R₄ and R₅ are methyl, L is not decamethylene;
with the proviso that when R₁-N-R₂ and R₇-N-R₈ are pyrrolidino and R₃, R₄, R₅ and R₆ are hydrogen, L is not trimethylene or 1,4-phenylene*bis*(methylene);
with the proviso that when R₁-N-R₂ and R₇-N-R₈ are piperidino and R₃, R₄, R₅ and R₆ are hydrogen, L is not trimethylene, 1,2-phenylenebis(methylene), 1,3-phenylene*bis*(methylene) or 1,4-phenylene*bis*(methylene); and
with the proviso that when R₁-N-R₂ and R₇-N-R₈ are 4-methylpiperazino and R₃, R₄, R₅ and R₆ are hydrogen, L is not trimethylene.

8. The compound according to claim 7, wherein R₁ = R₇, R₂ = R₈, R₃ = R₆, and R₄ = R₅ or alternatively, when used in combination, R₁-N-R₂ = R₇-N-R₈, R₃ = R₆, and R₄ = R₅.

9. The compound according to claim 8, wherein:
R₁, R₇ and R₂, R₈ are radicals independently selected from the group consisting of hydrogen, methyl, ethyl, phenyl, 2-hydroxyethyl, 2-cyanoethyl, and 2-(acetyloxy)ethyl, or alternatively, when taken in combination, R₁-N-R₂ = R₇-N-R₈ is selected from the group consisting of pyrrolidine, piperidine, piperazine and morpholine ring;
R₃, R₆ are radicals selected from the group consisting of hydrogen, methyl, nitro, amino, acetamido, methanesulfonamido, benzenesulfonamido, and *p*-toluenesulfonamido; and
R₄, R₅ are radicals selected from the group consisting of hydrogen, methyl, chloro, bromo, fluoro, iodo, amino, acetamido, methanesulfonamido, benzenesulfonamido, *p*-toluenesulfonamido, aminomethyl, acetamidomethyl, methanesulfonamidomethyl, benzenesulfonamidomethyl, and *p-*toluenesulfonamidomethyl.

10. The compound according to claim 9, wherein the compound is selected from the group consisting of:
1,1'-(decane-1,10-diyl)*bis*{3-methyl-4-[(*E*)-4-(pyrrolidin-1-yl)styryl]pyridin-1-ium} dibromide;
1,1'-(decane-1,10-diyl)*bis*{3-methyl-4-[(*E*)-4-(piperidin-1-yl)styryl]pyridin-1-ium} dibromide;
1,1'-(decane-1,10-diyl)*bis*{3-methyl-4-[(*E*)-4-morpholinostyryl]pyridin-1-ium} dibromide;
1,1'-(decane-1,10-diyl)*bis*{3-methyl-4-[(*E*)-4-(piperazin-1-yl)styryl]pyridin-1-ium} dibromide;
1,1'-(decane-1,10-diyl)*bis*{4-[(*E*)-4-(diphenylamino)styryl]-3-methylpyridin-1-ium} dibromide;
1,1'-(decane-1,10-diyl)*bis*{4-[(*E*)-4-(dimethylamino)-2-nitrostyryl]-3-methylpyridin-1-ium} dibromide;
1,1'-(decane-1,10-diyl)*bis*{4-{(*E*)-4-[(2-cyanoethyl)methylamino]styryl}-3-methylpyridin-1-ium} dibromide;
1,1'-(decane-1,10-diyl)b*i*s{4-{(*E*)-4-[bis(2-acetoxyethyl)amino]styryl}-3-methylpyridin-1-ium} dibromide;
1,1'-(decane-1,10-diyl)*bis*{4-{(*E*)-4-[bis(2-hydroxyethyl)amino]styryl}-3-methylpyridin-1-ium} dibromide;
1,1'-(ethane-1,2-diyl)*bis*{4-[(*E*)-4-(diethylamino)styryl]-3-methylpyridin-1-ium} dibromide;
1,1'-(propane-1,3-diyl)*bis*{4-[(*E*)-4-(diethylamino)styryl]-3-methylpyridin-1-ium} dibromide;
1,1'-(butane-1,4-diyl)*bis*{4-[(*E*)-4-(diethylamino)styryl]-3-methylpyridin-1-ium} dibromide;
1,1'-(pentane-1,5-diyl)*bis*{4-[(*E*)-4-(diethylamino)styryl]-3-methylpyridin-1-ium} dibromide;
1,1'-(hexane-1,6-diyl)*bis*{4-[(*E*)-4-(diethylamino)styryl]-3-methylpyridin-1-ium} dibromide;
1,1'-(heptane-1,7-diyl)*bis*{4-[(*E*)-4-(diethylamino)styryl]-3-methylpyridin-1-ium} dibromide;
1,1'-(octane-1,8-diyl)*bis*{4-[(*E*)-4-(diethylamino)styryl]-3-methylpyridin-1-ium} dibromide;
1,1'-(nonane-1,9-diyl)*bis*{4-[*(*E)-4-(diethylamino)styryl]-3-methylpyridin-1-ium} dibromide;
1,1'-(undecane-1,11-diyl)*bis*{4-[(*E*)-4-(diethylamino)styryl]-3-methylpyridin-1-ium} dibromide;
1,1'-(dodecane-1,12-diyl)*bis*{4-[(*E*)-4-(diethylamino)styryl]-3-methylpyridin-1-ium} dibromide;
1,1'-(3-oxapentane-1,5-diyl)*bis*{4-[(*E*)-4-(diethylamino)styryl]-3-methylpyridin-1-ium} dichloride;
1,1'-(3,6-dioxaoctane-1,8-diyl)*bis*{4-[(*E*)-4-(diethylamino)styryl]-3-methylpyridin-1-ium} diiodide;
1,1'-(3,6,9-trioxaundecane-1,11-diyl)*bis*{4-[(*E*)-4-(diethylamino)styryl]-3-methylpyridin-1-ium} dichloride;
1,1'-[1,2-phenylenebis(methylene)]*bis*{4-[(*E*)-4-(diethylamino)styryl]-3-methylpyridin-1-ium} dibromide;
1,1'-[1,3-phenylenebis(methylene)]*bis*{4-[(*E*)-4-(diethylamino)styryl]-3-methylpyridin-1-ium} dibromide;
1,1'-[1,4-phenylenebis(methylene)]*bis*{4-[(*E*)-4-(diethylamino)styryl]-3-methylpyridin-1-ium} dibromide;
1,1'-(decane-1,10-diyl)*bis*{4-[(*E*)-4-(diethylamino)styryl]pyridin-1-ium} dibromide;
1,1'-(decane-1,10-diyl)*bis*{3-bromo-4-[(*E*)-4-(diethylamino)styryl]pyridin-1-ium} dibromide;
1,1'-(decane-1,10-diyl)*bis*{3-chloro-4-[(*E*)-4-(diethylamino)styryl]pyridin-1-ium} dibromide; and
1,1'-(decane-1,10-diyl)*bis*{3-amino-4-[(*E*)-4-(diethylamino)styryl]pyridin-1-ium} dibromide.

## Patentansprüche

1. Eine Verbindung der Formel (I) 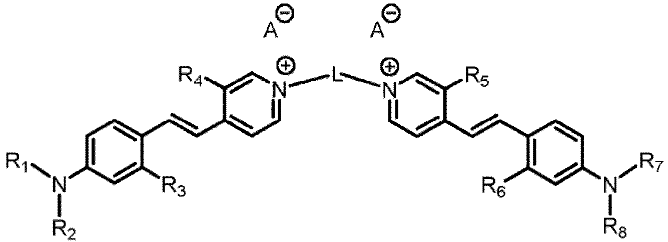 oder ein pharmazeutisch akzeptables Salz davon zur Verwendung als Medikament, wobei:
A ein Anion ist;
L ein Diradikal ausgewählt aus der Gruppe bestehend aus 1,2-Phenylenbis(methylen), 1,3-Phenylenbis(methylen), 1,4-Phenylenbis(methylen) und einer (C₂-C₁₂)-Oligomethylen-Kette ist, wobei ein bis drei Methylengruppen der (C₂-C₁₂)-Oligomethylen-Kette durch Sauerstoffatome substituiert sein können;
R₁, R₂, R₇ und R₈ Reste sind, die unabhängig voneinander ausgewählt sind aus der Gruppe bestehend aus Wasserstoff, (C₁-C₄)-Alkyl, Hydroxy-(C₁-C₄)-alkyl, Cyano-(C₁-C₄)-alkyl, (C₁-C₄)-Alkanoyloxy-(C₁-C₄)-alkyl, Phenyl, (C₁-C₄)-Alkanoyl, (C₁-C₄)-Alkansulfonyl, Benzolsulfonyl, Naphthalinsulfonyl und *p*-Toluolsulfonyl, oder, ersatzweise, wenn sie in Kombination genommen werden, R₁-N-R₂ und R₇-N-R₈ unabhängig voneinander ausgewählt sind aus der Gruppe bestehend aus Azetidin-, Pyrrolidin-, Piperidin-, Azepin-, Piperazin-, 4-(C₁-C₂)-Alkylpiperazin- und Morpholin-Ring;
R₃ und R₆ Reste sind, die unabhängig voneinander ausgewählt sind aus der Gruppe bestehend aus Wasserstoff, (C₁-C₄)-Alkyl, Nitro, Amino, (C₁-C₄)-Alkylamino, (C₁-C₄)-Alkanamido, (C₁-C₄)-Alkansulfonamido, Benzolsulfonamido, Naphthalinsulfonamido und p-Toluolsulfonamido; und
R₄ und R₅ Reste sind, die unabhängig voneinander ausgewählt sind aus der Gruppe bestehend aus Wasserstoff, (C₁-C₄)-Alkyl, Halogen, Amino, (C₁-C₄)-Alkylamino, (C₁-C₄)-Alkanamido, (C₁-C₄)-Alkansulfonamido, Benzolsulfonamido, Naphthalinsulfonamido, p-Toluolsulfonamido, Amino-(C₁-C₄)-Alkyl, (C₁-C₄)-Alkylamino-(C₁-C₄)-alkyl, (C₁-C₄)-Alkanamido-(C₁-C₄)-alkyl, (C₁-C₄)-Alkansulfonamido-(C₁-C₄)-Alkyl, Benzolsulfonamido-(C₁-C₄)-Alkyl, Naphthalinsulfonamido-(C₁-C₄)-Alkyl und p-Toluolsulfonamido-(C₁-C₄)-Alkyl.

2. Die Verbindung zur Verwendung nach Anspruch 1, wobei die Verbindung ausgewählt ist aus der Gruppe bestehend aus:
1,1'-(Decan-1,10-diyl)bis{3-methyl-4-[(E)-4-(pyrrolidin-1-yl)styryl]pyridin-1-ium}dibromid;
1,1'-(Decan-1,10-diyl)bis{3-methyl-4-[(E)-4-(piperidin-1-yl)styryl]pyridin-1-ium}dibromid;
1,1'-(Decan-1,10-diyl)bis{3-methyl-4-[(E)-4-morpholinostyryl]pyridin-1-ium}dibromid;
1,1'-(Decan-1,10-diyl)bis{3-methyl-4-[(E)-4-(piperazin-1-yl)styryl]pyridin-1-ium}dibromid;
1,1'-(Decan-1,10-diyl)bis{4-[(E)-4-(diphenylamino)styryl]-3-methylpyridin-1-ium}dibromid;
1,1'-(Decan-1,10-diyl)bis{4-[(E)-4-(dimethylamino)styryl]-3-methylpyridin-1-ium}dibromid;
1,1'-(Decan-1,10-diyl)bis{4-[(E)-4-(dimethylamino)-2-nitrostyryl]-3-methylpyridin-1-ium}dibromid;
1,1'-(Decan-1,10-diyl)bis{4-{(E)-4-[(2-cyanoethyl)methylamino]styryl}-3-methylpyridin-1-ium}dibromid;
1,1'-(Decan-1,10-diyl)bis{4-{(E)-4-[bis(2-acetoxyethyl)amino]styryl}-3-methylpyridin-1-ium}dibromid;
1,1'-(Decan-1,10-diyl)bis{4-{(E)-4-[bis(2-hydroxyethyl)amino]styryl}-3-methylpyridin-1-ium}amid;
1,1'-(Ethan-1,2-diyl)bis{4-[(E)-4-(diethylamino)styryl]-3-methylpyridin-1-ium}dibromid;
1,1'-(Propan-1,3-diyl)bis{4-[(E)-4-(diethylamino)styryl]-3-methylpyridin-1-ium}dibromid;
1,1'-(Butan-1,4-diyl)bis{4-[(E)-4-(diethylamino)styryl]-3-methylpyridin-1-ium}dibromid;
1,1'-(Pentan-1,5-diyl)bis{4-[(E)-4-(diethylamino)styryl]-3-methylpyridin-1-ium}dibromid;
1,1'-(Hexan-1,6-diyl)bis{4-[(E)-4-(diethylamino)styryl]-3-methylpyridin-1-ium}dibromid;
1,1'-(Heptan-1,7-diyl)bis{4-[(E)-4-(diethylamino)styryl]-3-methylpyridin-1-ium}dibromid;
1,1'-(Octan-1,8-diyl)bis{4-[(E)-4-(diethylamino)styryl]-3-methylpyridin-1-ium}dibromid;
1,1'-(Nonan-1,9-diyl)bis{4-[(E)-4-(diethylamino)styryl]-3-methylpyridin-1-ium}dibromid;
1,1'-(Decan-1,10-diyl)bis{4-[(E)-4-(diethylamino)styryl]-3-methylpyridin-1-ium}dibromid;
1,1'-(Undecan-1,11-diyl)bis{4-[(E)-4-(diethylamino)styryl]-3-methylpyridin-1-ium}dibromid;
1,1'-(Dodecan-1,12-diyl)bis{4-[(E)-4-(diethylamino)styryl]-3-methylpyridin-1-ium}dibromid
1,1'-(3-Oxapentan-1,5-diyl)bis{4-[(E)-4-(diethylamino)styryl]-3-methylpyridin-1-ium}dichlorid;
1,1'-(3,6-Dioxaoctan-1,8-diyl)bis{4-[(E)-4-(diethylamino)styryl]-3-methylpyridin-1-ium}diiodid;
1,1'-(3,6,9-Trioxaundecan-1,11-diyl)bis{4-[(E)-4-(diethylamino)styryl]-3-methylpyridin-1-ium}dichlorid;
1,1'-[1,2-Phenylenbis(methylen)]bis{4-[(E)-4-(diethylamino)styryl]-3-methylpyridin-1-ium}dibromid;
1,1'-[1,3-Phenylenbis(methylen)]bis{4-[(E)-4-(diethylamino)styryl]-3-methylpyridin-1-ium}dibromid;
1,1'-[1,4-Phenylenbis(methylen)]bis{4-[(E)-4-(diethylamino)styryl]-3-methylpyridin-1-ium}dibromid;
1,1'-(Decan-1,10-diyl)bis{4-[(E)-4-(diethylamino)styryl]pyridin-1-ium}dibromid;
1,1'-(Decan-1,10-diyl)bis{3-brom-4-[(E)-4-(diethylamino)styryl]pyridin-1-ium}dibromid;
1,1'-(Decan-1,10-diyl)bis{3-chlor-4-[(E)-4-(diethylamino)styryl] pyridin-1-ium}dibromid; und
1,1'-(Decan-1,10-diyl)bis{3-amino-4-[(E)-4-(diethylamino)styryl]pyridin-1-ium}dibromid.

3. Die Verbindung zur Verwendung nach Anspruch 2, wobei die Verbindung die Formel (VI) hat 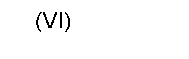 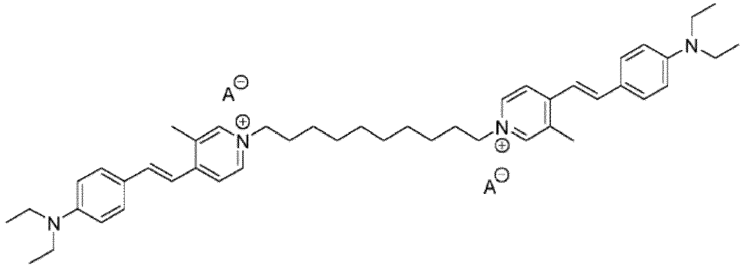 oder ein pharmazeutisch akzeptables Salz davon ist.

4. Die Verbindung zur Verwendung nach Anspruch 3, wobei A Bromid ist.

5. Eine Verbindung der Formel (I) 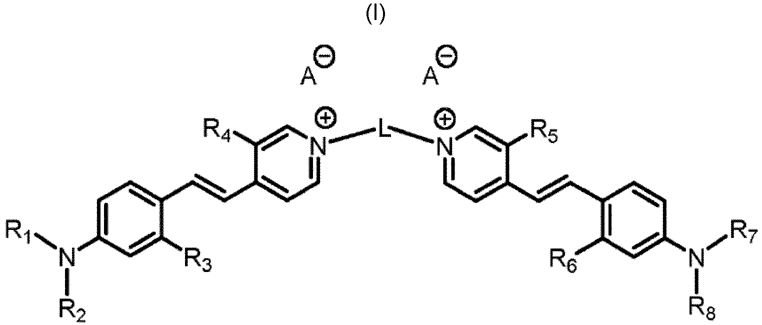 oder ein pharmazeutisch akzeptables Salz davon zur Verwendung bei der Behandlung, Prävention oder Verbesserung von Malaria oder Symptomen, Komplikationen und/oder Folgeerscheinungen davon, wobei:
A ein Anion ist;
L ein Diradikal ausgewählt aus der Gruppe bestehend aus 1,2-Phenylenbis(methylen), 1,3-Phenylenbis(methylen), 1,4-Phenylenbis(methylen) und einer (C₂-C₁₂)-Oligomethylen-Kette ist, wobei ein bis drei Methylengruppen der (C₂-C₁₂)-Oligomethylen-Kette durch Sauerstoffatome substituiert sein können;
R₁, R₂, R₇ und R₈ Reste sind, die unabhängig voneinander ausgewählt sind aus der Gruppe bestehend aus Wasserstoff, (C₁-C₄)-Alkyl, Hydroxy-(C₁-C₄)-Alkyl, Cyano-(C₁-C₄)-Alkyl, (C₁-C₄)-Alkanoyloxy-(C₁-C₄)-Alkyl, Phenyl, (C₁-C₄)-Alkanoyl, (C₁-C₄)-Alkansulfonyl, Benzolsulfonyl, Naphthalinsulfonyl und p-Toluolsulfonyl, oder ersatzweise, wenn sie in Kombination genommen werden, R₁-N-R₂ und R₇-N-R₈ unabhängig voneinander ausgewählt sind aus der Gruppe bestehend aus Azetidin-, Pyrrolidin-, Piperidin-, Azepin-, Piperazin-, 4-(C₁-C₂)-Alkylpiperazin- und Morpholin-Ring;
R₃ und R₆ Reste sind, die unabhängig voneinander ausgewählt sind aus der Gruppe bestehend aus Wasserstoff, (C₁-C₄)-Alkyl, Nitro, Amino, (C₁-C₄)-Alkylamino, (C₁-C₄)-Alkanamido, (C₁-C₄)-Alkansulfonamido, Benzolsulfonamido, Naphthalinsulfonamido und p-Toluolsulfonamido; und
R₄ und R₅ Reste sind, die unabhängig voneinander ausgewählt sind aus der Gruppe bestehend aus Wasserstoff, (C₁-C₄)-Alkyl, Halogen, Amino, (C₁-C₄)-Alkylamino, (C₁-C₄)-Alkanamido, (C₁-C₄)-Alkansulfonamido, Benzolsulfonamido, Naphthalinsulfonamido, p-Toluolsulfonamido, Amino-(C₁-C₄)-Alkyl, (C₁-C₂)-Alkylamino-(C₁-C₄)-Alkyl, (C₁-C₄)-Alkanamido-(C₁-C₄)-Alkyl, (C₁-C₄)-Alkansulfonamido-(C₁-C₄)-Alkyl, Benzolsulfonamido-(C₁-C₄)-Alkyl, Naphthalinsulfonamido- (C₁-C₄)-Alkyl und p-Toluolsulfonamido- (C₁-C₄)-Alkyl.

6. Eine pharmazeutische Zusammensetzung umfassend eine wirksame Menge einer Verbindung der Formel (I) 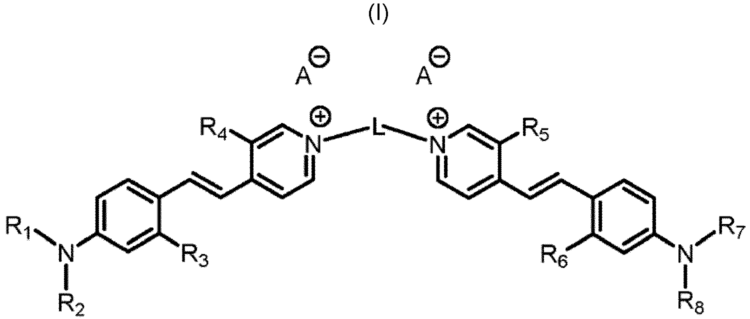
oder ein pharmazeutisch akzeptables Salz davon, wobei:
A ein Anion ist;
L ein Diradikal ausgewählt aus der Gruppe bestehend aus 1,2-Phenylenbis(methylen), 1,3-Phenylenbis(methylen), 1,4-Phenylenbis(methylen) und einer (C₂-C₁₂)-Oligomethylen-Kette ist, wobei ein bis drei Methylengruppen der (C₂-C₁₂)-Oligomethylen-Kette durch Sauerstoffatome substituiert sein können;
R₁, R₂, R₇ und R₈ Reste sind, die unabhängig voneinander ausgewählt sind aus der Gruppe bestehend aus Wasserstoff, (C₁-C₄)-Alkyl, Hydroxy-(C₁-C₄)-alkyl, Cyano-(C₁-C₄)-alkyl, (C₁-C₄)-Alkanoyloxy-(C₁-C₄)-alkyl, Phenyl, (C₁-C₄)-Alkanoyl, (C₁-C₄)-Alkansulfonyl, Benzolsulfonyl, Naphthalinsulfonyl und p-Toluolsulfonyl, oder ersatzweise, wenn sie in Kombination genommen werden, R₁-N-R₂ und R₇-N-R₈ unabhängig voneinander ausgewählt sind aus der Gruppe bestehend aus Azetidin-, Pyrrolidin-, Piperidin-, Azepin-, Piperazin-, 4-(C₁-C₂)-Alkylpiperazin- und Morpholin-Ring;
R₃ und R₆ Reste sind, die unabhängig voneinander ausgewählt sind aus der Gruppe bestehend aus Wasserstoff, (C₁-C₄)-Alkyl, Nitro, Amino, (C₁-C₄)-Alkylamino, (C₁-C₄)-Alkanamido, (C₁-C₄)-Alkansulfonamido, Benzolsulfonamido, Naphthalinsulfonamido und p-Toluolsulfonamido; und
R₄ und R₅ Reste sind, die unabhängig voneinander ausgewählt sind aus der Gruppe bestehend aus Wasserstoff, (C₁-C₄)-Alkyl, Halogen, Amino, (C₁-C₄)-Alkylamino, (C₁-C₄)-Alkanamido, (C₁-C₄)-Alkansulfonamido, Benzolsulfonamido, Naphthalinsulfonamido, p-Toluolsulfonamido, Amino-(C₁-C₄)-Alkyl, (C₁-C₂)-Alkylamino-(C₁-C₄)-Alkyl, (C₁-C₄)-Alkanamido-(C₁-C₄)-Alkyl, (C₁-C₄)-Alkansulfonamido-(C₁-C₄)-Alkyl, Benzolsulfonamido-(C₁-C₄)-Alkyl, Naphthalinsulfonamido-(C₁-C₄)-Alkyl und p-Toluolsulfonamido-(C₁-C₄)-Alkyl,
zusammen mit geeigneten Mengen an pharmazeutisch akzeptablen Hilfsstoffen oder Trägersubstanzen.

7. Eine Verbindung der Formel (I) 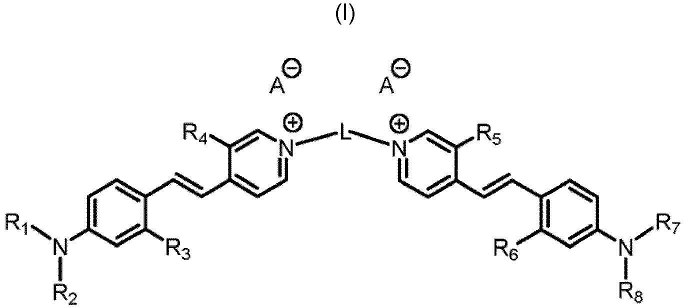 oder ein pharmazeutisch akzeptables Salz davon, wobei:
A ein Anion ist;
L ein Diradikal ist, das aus der Gruppe ausgewählt ist, die aus Ethylen, Trimethylen, Tetramethylen, Pentamethylen, Hexamethylen, Heptamethylen, Octamethylen, Nonamethylen, Decamethylen, Undecamethylen, Dodecamethylen, 3-Oxapentamethylen, 3,6-Dioxaoctamethylen, 3,6,9-Trioxaundecamethylen, 1,2-Phenylenbis(methylen), 1,3-Phenylenbis(methylen) und 1,4-Phenylenbis(methylen) besteht;
R₁, R₂, R₇ und R₈ Reste sind, die unabhängig voneinander ausgewählt sind aus der Gruppe bestehend aus Wasserstoff, (C₁-C₄)-Alkyl, Hydroxy-(C₁-C₄)-alkyl, Cyano-(C₁-C₄)-alkyl, (C₁-C₄)-Alkanoyloxy-(C₁-C₄)-alkyl, Phenyl, (C₁-C₄)-Alkanoyl, (C₁-C₄)-Alkansulfonyl, Benzolsulfonyl, Naphthalinsulfonyl und *p*-Toluolsulfonyl oder, ersatzweise, wenn sie in Kombination genommen werden, R₁-N-R₂ und R₇-N-R₈ unabhängig voneinander ausgewählt sind aus der Gruppe bestehend aus Azetidin-, Pyrrolidin-, Piperidin-, Azepin-, Piperazin-, 4-(C₁-C₂)-Alkylpiperazin- und Morpholin-Ring;
R₃ und R₆ Reste sind, die unabhängig voneinander ausgewählt sind aus der Gruppe bestehend aus Wasserstoff, (C₁-C₄)-Alkyl, Nitro, Amino, (C₁-C₄)-Alkylamino, (C₁-C₄)-Alkanamido, (C₁-C₄)-Alkansulfonamido, Benzolsulfonamido, Naphthalinsulfonamido und p-Toluolsulfonamido; und
R₄ und R₅ Reste sind, die unabhängig voneinander ausgewählt sind aus der Gruppe bestehend aus Wasserstoff, (C₁-C₄)-Alkyl, Halogen, Amino, (C₁-C₄)-Alkylamino, (C₁-C₄)-Alkanamido, (C₁-C₄)-Alkansulfonamido, Benzolsulfonamido, Naphthalinsulfonamido, p-Toluolsulfonamido, Amino-(C₁-C₄)-Alkyl, (C₁-C₂)-Alkylamino-(C₁-C₄)-Alkyl, (C₁-C₄)-Alkanamido-(C₁-C₄)-Alkyl, (C₁-C₄)-Alkansulfonamido-(C₁-C₄)-Alkyl, Benzolsulfonamido-(C₁-C₄)-Alkyl, Naphthalinsulfonamido-(C₁-C₄)-Alkyl und *p-*Toluolsulfonamido-(C₁-C₄)-Alkyl;
mit der Maßgabe, dass, wenn R₁, R₂, R₃, R₄, R₅, R₆, R₇ und R₈ Wasserstoff sind, L nicht 1,4-Phenylenbis(methylen) ist;
mit der Maßgabe, dass, wenn R₁, R₂, R₇ und R₈ Methyl sind und R₃, R₄, R₅ und R₆ Wasserstoff sind, L nicht Ethylen, Trimethylen, Tetramethylen, Pentamethylen, Hexamethylen, Decamethylen, Dodecamethylen, 3,6-Dioxaoctamethylen, 1,2-Phenylenbis(methylen), 1,3-Phenylenbis(methylen) oder 1,4-Phenylenbis(methylen) ist;
mit der Maßgabe, dass, wenn R₁, R₂, R₇ und R₈ Ethyl sind und R₃, R₄, R₅ und R₆ Wasserstoff sind, Lis nicht Trimethylen, Tetramethylen, 3-Oxapentamethylen, 1,2-Phenylenbis(methylen) oder 1,4-Phenylenbis(methylen) ist;
mit der Maßgabe, dass, wenn R₁, R₂, R₇ und R₈ Phenyl sind und R₃, R₄, R₅ und R₆ Wasserstoff sind, L nicht Trimethylen, Tetramethylen, Pentamethylen, Hexamethylen, Octamethylen, Decamethylen, Dodecamethylen, 1,2-Phenylenbis(methylen), 1,3-Phenylenbis(methylen) oder 1,4-Phenylenbis(methylen) ist;
mit der Maßgabe, dass, wenn R₁, R₂, R₇ und R₈ HO-CH₂-CH₂- sind und R₃, R₄, R₅ und R₆ Wasserstoff sind, L nicht Tetramethylen ist;
mit der Maßgabe, dass, wenn R₁ und R₇ Ethyl sind, R₂ und R₈ HO-CH₂-CH₂- sind und R₃, R₄, R₅ und R₆ Wasserstoff sind, L nicht Ethylen, Trimethylen, Tetramethylen, Pentamethylen, Hexamethylen oder Heptamethylen ist;
mit der Maßgabe, dass, wenn R₁ und R₇ Methyl sind, R₂ und R₈ NC-CH₂- sind und R₃, R₄, R₅ und R₆ Wasserstoff sind, L nicht 1,2-Phenylenbis(methylen) oder 1,4-Phenylenbis(methylen) ist,
mit der Maßgabe, dass, wenn R₁ und R₇ Acetyl sind, R₂, R₃, R₄, R₅, R₆ und R₈ Wasserstoff sind, L nicht 1,4-Phenylenbis(methylen) ist;
mit der Maßgabe, dass, wenn R₁, R₂, R₃, R₆, R₇ und R₈ Methyl sind und R₄ und R₅ Wasserstoff sind, L nicht Trimethylen oder 1,4-Phenylenbis(methylen) ist;
mit der Maßgabe, dass, wenn R₁, R₂, R₃, R₆, R₇ und R₈ Methyl sind und R₃ und R₆ Wasserstoff sind, L nicht Trimethylen, Tetramethylen oder Decamethylen ist;
mit der Maßgabe, dass, wenn R₁, R₂, R₇ und R₈ Ethyl sind, R₃ und R₆ Wasserstoff sind und R₄ und R₅ Methyl sind, L nicht Decamethylen ist;
mit der Maßgabe, dass, wenn R₁-N-R₂ und R₇-N-R₈ Pyrrolidino sind und R₃, R₄, R₅ und R₆ Wasserstoff sind, L nicht Trimethylen oder 1,4-Phenylenbis(methylen) ist;
mit der Maßgabe, dass, wenn R₁-N-R₂ und R₇-N-R₈ Piperidino sind und R₃, R₄, R ₅ und R₆ Wasserstoff sind, L nicht Trimethylen, 1,2-Phenylenbis(methylen), 1,3-Phenylenbis(methylen) oder 1,4-Phenylenbis(methylen) ist; und
mit der Maßgabe, dass, wenn R₁-N-R₂ und R₇-N-R₈ 4-Methylpiperazino sind und R₃, R₄, R₅ und R₆ Wasserstoff sind, L nicht Trimethylen ist.

8. Die Verbindung nach Anspruch 7, wobei R₁ = R₇, R₂ = R₈, R₃ = R₆ und R ₄ = R₅ ist oder ersatzweise, wenn sie in Kombination verwendet werden, R₁-N-R₂ = R₇-N-R₈, R₃ = R₆ und R₄ = R₅ ist.

9. Die Verbindung nach Anspruch 8, wobei:
R₁, R₇ und R₂, R₈ Reste sind, die unabhängig ausgewählt sind aus der Gruppe bestehend aus Wasserstoff, Methyl, Ethyl, Phenyl, 2-Hydroxyethyl, 2-Cyanoethyl und 2-(Acetyloxy)ethyl, oder ersatzweise, wenn sie in Kombination genommen werden, R₁-N-R₂ = R₇-N-R₈ ausgewählt ist aus der Gruppe bestehend aus Pyrrolidin-, Piperidin-, Piperazin- und Morpholin-Ring;
R₃, R₆ Reste sind, die aus der Gruppe ausgewählt sind, die aus Wasserstoff, Methyl, Nitro, Amino, Acetamido, Methansulfonamido, Benzolsulfonamido und p-Toluolsulfonamido besteht; und
R₄, R₅ Reste sind, die aus der Gruppe ausgewählt sind, die aus Wasserstoff, Methyl, Chlor, Brom, Fluor, lod, Amino, Acetamido, Methansulfonamido, Benzolsulfonamido, p-Toluolsulfonamido, Aminomethyl, Acetamidomethyl, Methansulfonamidomethyl, Benzolsulfonamidomethyl und p-Toluolsulfonamidomethyl besteht.

10. Die Verbindung nach Anspruch 9, wobei die Verbindung ausgewählt ist aus der Gruppe bestehend aus:
1,1'-(Decan-1,10-diyl)bis{3-methyl-4-[(E)-4-(pyrrolidin-1-yl)styryl]pyridin-1-ium}dibromid;
1,1'-(Decan-1,10-diyl)bis{3-methyl-4-[(E)-4-(piperidin-1-yl)styryl]pyridin-1-ium}dibromid;
1,1'-(Decan-1,10-diyl)bis{3-methyl-4-[(E)-4-morpholinostyryl]pyridin-1-ium}dibromid;
1,1'-(Decan-1,10-diyl)bis{3-methyl-4-[(E)-4-(piperazin-1-yl)styryl]pyridin-1-ium}dibromid;
1,1'-(Decan-1,10-diyl)bis{4-[(E)-4-(diphenylamino)styryl]-3-methylpyridin-1-ium}dibromid;
1,1'-(Decan-1,10-diyl)bis{4-[(E)-4-(dimethylamino)-2-nitrostyryl]-3-methylpyridin-1-ium}dibromid;
1,1'-(Decan-1,10-diyl)bis{4-{(E)-4-[(2-cyanoethyl)methylamino]styryl}-3-methylpyridin-1-ium}dibromid;
1,1'-(Decan-1,10-diyl)bis{4-{(E)-4-[bis(2-acetoxyethyl)amino]styryl}-3-methylpyridin-1-ium}dibromid;
1,1'-(Decan-1,10-diyl)bis{4-{(E)-4-[bis(2-hydroxyethyl)amino]styryl}-3-methylpyridin-1-ium}dibromid;
1,1'-(Ethan-1,2-diyl)bis{4-[(E)-4-(diethylamino)styryl]-3-methylpyridin-1-ium}dibromid;
1,1'-(Propan-1,3-diyl)bis{4-[(E)-4-(diethylamino)styryl]-3-methylpyridin-1-ium}dibromid;
1,1'-(Butan-1,4-diyl)bis{4-[(E)-4-(diethylamino)styryl]-3-methylpyridin-1-ium}dibromid;
1,1'-(Pentan-1,5-diyl)bis{4-[(E)-4-(diethylamino)styryl]-3-methylpyridin-1-ium}dibromid;
1,1'-(Hexan-1,6-diyl)bis{4-[(E)-4-(diethylamino)styryl]-3-methylpyridin-1-ium}dibromid;
1,1'-(Heptan-1,7-diyl)bis{4-[(E)-4-(diethylamino)styryl]-3-methylpyridin-1-ium}dibromid;
1,1'-(Octan-1,8-diyl)bis{4-[(E)-4-(diethylamino)styryl]-3-methylpyridin-1-ium}dibromid;
1,1'-(Nonan-1,9-diyl)bis{4-[(E)-4-(diethylamino)styryl]-3-methylpyridin-1-ium}dibromid;
1,1'-(Undecan-1,11-diyl)bis{4-[(E)-4-(diethylamino)styryl]-3-methylpyridin-1-ium}dibromid;
1,1'-(Dodecan-1,12-diyl)bis{4-[(E)-4-(diethylamino)styryl]-3-methylpyridin-1-ium}dibromid;
1,1'-(3-Oxapentan-1,5-diyl)bis{4-[(E)-4-(diethylamino)styryl]-3-methylpyridin-1-ium}dichlorid;
1,1'-(3,6-Dioxaoctan-1,8-diyl)bis{4-[(E)-4-(diethylamino)styryl]-3-methylpyridin-1-ium}diiodid;
1,1'-(3,6,9-Trioxaundecan-1,11-diyl)bis{4-[(E)-4-(diethylamino)styryl]-3-methylpyridin-1-ium}dichlorid;
1,1'-[1,2-Phenylenbis(methylen)]bis{4-[(E)-4-(diethylamino)styryl]-3-methylpyridin-1-ium}dibromid;
1,1'-[1,3-Phenylenbis(methylen)]bis{4-[(E)-4-(diethylamino)styryl]-3-methylpyridin-1-ium}dibromid;
1,1'-[1,4-Phenylenbis(methylen)]bis{4-[(E)-4-(diethylamino)styryl]-3-methylpyridin-1-ium}dibromid;
1,1'-(Decan-1,10-diyl)bis{4-[(E)-4-(diethylamino)styryl]pyridin-1-ium}dibromid;
1,1'-(Decan-1,10-diyl)bis{3-brom-4-[(E)-4-(diethylamino)styryl]pyridin-1-ium}dibromid;
1,1'-(Decan-1,10-diyl)bis{3-chlor-4-[(E)-4-(diethylamino)styryl]pyridin-1-ium}dibromid; und
1,1'-(Decan-1,10-diyl)bis{3-amino-4-[(E)-4-(diethylamino)styryl]pyridin-1-ium}dibromid.

## Revendications

1. Un composé de formule (I) 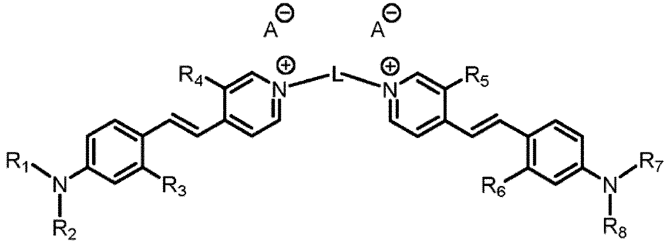 ou un sel pharmaceutiquement acceptable de celui-ci pour son utilisation en tant que médicament, dans lequel :
A est un anion ;
L est un diradical choisi dans le groupe constitué par le 1,2-phénylènebis(méthylène), le 1,3-phénylènebis(méthylène), le 1,4-phénylènebis(méthylène) et une chaîne d'oligométhylène en (C₂-C₁₂), dans lequel d'un à trois groupes méthylène de la chaîne d'oligométhylène en (C₂-C₁₂) peuvent être substitués par des atomes d'oxygène ;
R₁, R₂, R₇ et R₈ sont des radicaux choisis indépendamment dans le groupe constitué par l'hydrogène, un alkyle en (C₁-C₄), un hydroxyalkyle en (C₁-C₄), un cyanoalkyle en (C₁-C₄), un alcanoyloxy-alkyle en (C₁-C₄), le phényle, un alcanoyle en (C₁-C₄), un alcanesulfonyle en (C₁-C₄), le benzènesulfonyle, le naphtalènesulfonyle et le p-toluènesulfonyle, ou, en variante, lorsqu'ils sont pris en combinaison, R₁-N-R₂ et R₇-N-R₈ sont choisis indépendamment dans le groupe constitué par le cycle azétidine, pyrrolidine, pipéridine, azépine, pipérazine, 4-alkylpipérazine en (C₁-C₂) et morpholine ;
R₃ et R₆ sont des radicaux choisis indépendamment dans le groupe constitué par l'hydrogène, alkyle en (C₁-C₄), nitro, amino, alkylamino en (C₁-C₄), alcanamido en (C₁-C₄), alcanesulfonamido en (C₁-C₄), benzène-sulfonamido, naphtalènesulfonamido et p-toluènesulfonamido ; et
R₄ et R₅ sont des radicaux choisis indépendamment dans le groupe constitué par l'hydrogène, alkyle en (C₁-C₄), halogène, amino, alkylamino en (C₁-C₄), alcanamido en (C₁-C₄), alcanesulfonamido en (C₁-C₄), benzènesulfonamido, naphtalènesulfonamido, p-toluènesulfonamido, amino-alkyle en (C₁-C₄), alkylamino en (C₁-C₄)-alkyle en (C₁-C₄), alcanamido en (C₁-C₄)-alkyle en (C₁-C₄), alcanesulfonamido en (C₁-C₄)-alkyle en (C₁-C₄), benzènesulfonamido-alkyle en (C₁-C₄), naphtalènesulfonamido-alkyle en (C₁-C₄) et un p-toluènesulfonamido-alkyle en (C₁-C₄).

2. Le composé pour son utilisation selon la revendication 1, dans lequel le composé est choisi dans le groupe constitué par :
1,1'-(décane-1,10-diyl)bis{3-méthyl-4-[(E)-4-(pyrrolidin-1-yl)styryl]pyridin-1-ium}dibromure ;
1,1'-(décane-1,10-diyl)bis{3-méthyl-4-[(E)-4-(pipéridin-1-yl)styryl]pyridin-1-ium}dibromure ;
1,1'-(décane-1,10-diyl)bis{3-méthyl-4-[(E)-4-morpholinostyryl]pyridin-1-ium}dibromure ;
1,1'-(décane-1,10-diyl)bis{3-méthyl-4-[(E)-4-(pipérazin-1-yl)styryl]pyridin-1-ium}dibromure ;
1,1'-(décane-1,10-diyl)bis{4-[(E)-4-(diphénylamino)styryl]-3-méthylpyridin-1-ium}dibromure ;
1,1'-(décane-1,10-diyl)bis{4-[(E)-4-(diméthylamino)styryl]-3-méthylpyridin-1-ium}dibromure ;
1,1'-(décane-1,10-diyl)bis{4-[(E)-4-(diméthylamino)-2-nitrostyryl]-3-méthylpyridin-1-ium}dibromure ;
1,1'-(décane-1,10-diyl)bis{4-{(E)-4-[(2-cyanoéthyl)méthylamino]styryl}-3-méthylpyridin-1-ium}dibromure ;
1,1'-(décane-1,10-diyl)bis{4-{(E)-4-[bis(2-acétoxyéthyl)amino]styryl}-3-méthylpyridin-1-ium}dibromure ;
1,1'-(décane-1,10-diyl)bis{4-{(E)-4-[bis(2-hydroxyéthyl)amino]styryl}-3-méthylpyridin-1-ium}mide ;
1,1'-(éthane-1,2-diyl)bis{4-[(E)-4-(diéthylamino)styryl]-3-méthylpyridin-1-ium}dibromure ;
1,1'-(propane-1,3-diyl)bis{4-[(E)-4-(diéthylamino)styryl]-3-méthylpyridin-1-ium}dibromure ;
1,1'-(butane-1,4-diyl)bis{4-[(E)-4-(diéthylamino)styryl]-3-méthylpyridin-1-ium}dibromure ;
1,1'-(pentane-1,5-diyl)bis{4-[(E)-4-(diéthylamino)styryl]-3-méthylpyridin-1-ium}dibromure ;
1,1'-(hexane-1,6-diyl)bis{4-[(E)-4-(diéthylamino)styryl]-3-méthylpyridin-1-ium}dibromure ;
1,1'-(heptane-1,7-diyl)bis{4-[(E)-4-(diéthylamino)styryl]-3-méthylpyridin-1-ium}dibromure ;
1,1'-(octane-1,8-diyl)bis{4-[(E)-4-(diéthylamino)styryl]-3-méthylpyridin-1-ium}dibromure ;
1,1'-(nonane-1,9-diyl)bis{4-[(E)-4-(diéthylamino)styryl]-3-méthylpyridin-1-ium}dibromure ;
1,1'-(décane-1,10-diyl)bis{4-[(E)-4-(diéthylamino)styryl]-3-méthylpyridin-1-ium}dibromure ;
1,1'-(undécane-1,11-diyl)bis{4-[(E)-4-(diéthylamino)styryl]-3-méthylpyridin-1-ium}dibromure ;
1,1'-(dodécane-1,12-diyl)bis{4-[(E)-4-(diéthylamino)styryl]-3-méthylpyridin-1-ium}dibromure
1,1'-(3-oxapentane-1,5-diyl)bis{4-[(E)-4-(diéthylamino)styryl]-3-méthylpyridin-1-ium}dichlorure ;
1,1'-(3,6-dioxaoctane-1,8-diyl)bis{4-[(E)-4-(diéthylamino)styryl]-3-méthylpyridin-1-ium}diiodure ;
1,1'-(3,6,9-trioxaundécane-1,11-diyl)bis{4-[(E)-4-(diéthylamino)styryl]-3-méthylpyridin-1-ium}dichlorure ;
1,1'-[1,2-phénylènebis(méthylène)]bis{4-[(E)-4-(diéthylamino)styryl]-3-méthylpyridin-1-ium}dibromure ;
1,1'-[1,3-phénylènebis(méthylène)]bis{4-[(E)-4-(diéthylamino)styryl]-3-méthylpyridin-1-ium}dibromure ;
1,1'-[1,4-phénylènebis(méthylène)]bis{4-[(E)-4-(diéthylamino)styryl]-3-méthylpyridin-1-ium}dibromure ;
1,1'-(décane-1,10-diyl)bis{4-[(E)-4-(diéthylamino)styryl]pyridin-1-ium}dibromure ;
1,1'-(décane-1,10-diyl)bis{3-bromo-4-[(E)-4-(diéthylamino)styryl]pyridin-1-ium}dibromure ;
1,1'-(décane-1,10-diyl)bis{3-chloro-4-[(E)-4-(diéthylamino)styryl]pyridin-1-ium}dibromure ;
et
1,1'-(décane-1,10-diyl)bis{3-amino-4-[(E)-4-(diéthylamino)styryl]pyridin-1-ium}dibromure.

3. Le composé pour son utilisation selon la revendication 2, dans lequel le composé est de formule (VI) 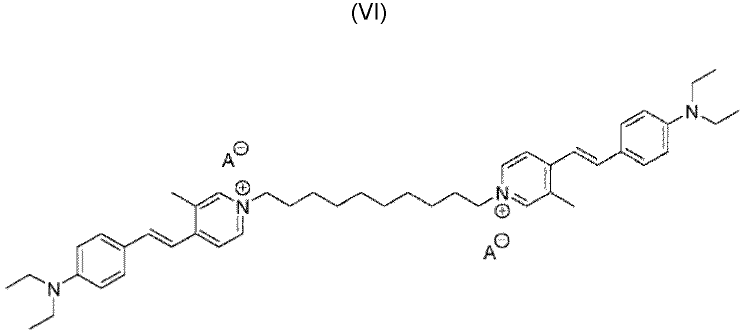 ou un sel pharmaceutiquement acceptable de celui-ci.

4. Le composé pour son utilisation selon la revendication 3, dans lequel A est le bromure.

5. Un composé de formule (I) 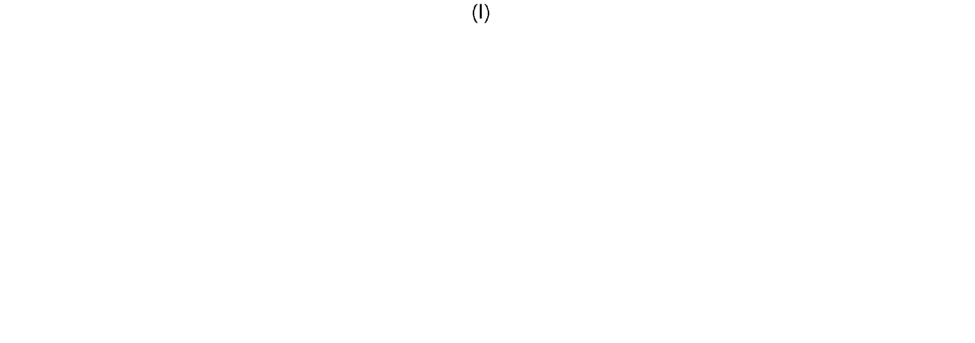 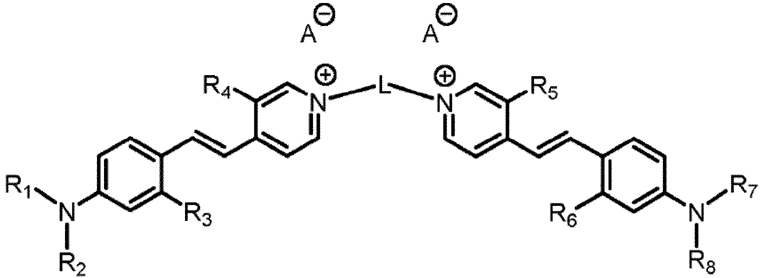 ou un sel pharmaceutiquement acceptable de celui-ci pour son utilisation dans le traitement, la prévention ou l'amélioration de la malaria, ou des symptômes, complications et/ou séquelles de celle-ci, dans lequel :
A est un anion ;
L est un diradical choisi dans le groupe constitué par le 1,2-phénylènebis(méthylène), le 1,3-phénylènebis(méthylène), le 1,4-phénylènebis(méthylène) et une chaîne d'oligométhylène en (C₂-C₁₂), dans lequel d'un à trois groupes méthylène de la chaîne d'oligométhylène en (C₂-C₁₂) peuvent être substitués par des atomes d'oxygène ;
R₁, R₂, R₇ et R₈ sont des radicaux choisis indépendamment dans le groupe constitué par l'hydrogène, alkyle en (C₁-C₄), hydroxyalkyle en (C₁-C₄), cyanoalkyle en (C₁-C₄), alcanoyloxyalkyle en (C₁-C₄), phényle, alcanoyle en (C₁-C₄), alcanesulfonyle en (C₁-C₄), benzènesulfonyle, naphtalènesulfonyle et p-toluènesulfonyle, ou, en variante, lorsqu'ils sont pris en combinaison, R₁-N-R₂ et R₇-N-R₈ sont choisis indépendamment dans le groupe constitué par les cycles azétidine, pyrrolidine, pipéridine, azépine, pipérazine, 4-alkylpipérazine en (C₁-C₂) et morpholine ;
R₃ et R₆ sont des radicaux choisis indépendamment dans le groupe constitué par l'hydrogène, alkyle en (C₁-C₄), nitro, amino, alkylamino en (C₁-C₄), alcanamido en (C₁-C₄), alcanesulfonamido en (C₁-C₄), benzène-sulfonamido, naphtalènesulfonamido et p-toluènesulfonamido ; et
R₄ et R₅ sont des radicaux choisis indépendamment dans le groupe constitué par l'hydrogène, un alkyle en (C₁-C₄), un halogène, amino, alkylamino en (C₁-C₄), alcanamido en C₁-C₄, alcanesulfonamido en (C₁-C₄), benzènesulfonamido, naphtalènesulfonamido, p-toluènesulfonamido, amino-alkyle en (C₁-C₄), un alkylamino en (C₁-C₂)-alkyle en (C₁-C₄), alcanamido en (C₁-C₄)-alkyle en (C₁-C₄), alcanesulfonamido en (C₁-C₄)-alkyle en (C₁-C₄), benzènesulfonamido-alkyle en (C₁-C₄), naphtalènesulfonamido-alkyle en (C₁-C₄) et p-toluènesulfonamido-alkyle en (C₁-C₄).

6. Une composition pharmaceutique comprenant une quantité efficace d'un composé de formule (I) 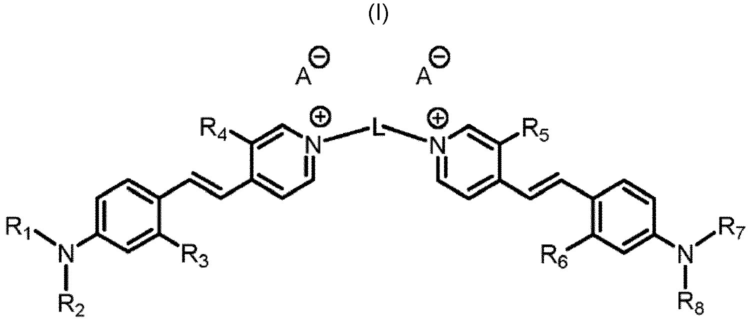
ou un sel pharmaceutiquement acceptable de celui-ci, dans laquelle
A est un anion ;
L est un diradical choisi dans le groupe constitué par le 1,2-phénylènebis(méthylène), le 1,3-phénylènebis(méthylène), le 1,4-phénylènebis(méthylène) et une chaîne d'oligométhylène en (C₂-C₁₂), dans lequel d'un à trois groupes méthylène de la chaîne d'oligométhylène en (C₂-C₁₂) peuvent être substitués par des atomes d'oxygène ;
R₁, R₂, R₇ et R₈ sont des radicaux choisis indépendamment dans le groupe constitué par l'hydrogène, un alkyle en (C₁-C₄), un hydroxyalkyle en (C₁-C₄), un cyanoalkyle en (C₁-C₄), un alcanoyloxyalkyle en (C₁-C₄), phényle, alcanoyle en (C₁-C₄), alcanesulfonyle en (C₁-C₄), benzènesulfonyle, naphtalènesulfonyle et un p-toluènesulfonyle, ou, en variante, lorsqu'ils sont pris en combinaison, R₁-N-R₂ et R₇-N-R₈ sont choisis indépendamment dans le groupe constitué par les cycles azétidine, pyrrolidine, pipéridine, azépine, pipérazine, 4-alkylpipérazine en (C₁-C₂) et morpholine ;
R₃ et R₆ sont des radicaux choisis indépendamment dans le groupe constitué par l'hydrogène, alkyle en (C₁-C₄), nitro, amino, alkylamino en (C₁-C₄), alcanamido en (C₁-C₄), alcanesulfonamido en (C₁-C₄), benzène-sulfonamido, naphtalènesulfonamido et p-toluènesulfonamido ; et
R₄ et R₅ sont des radicaux choisis indépendamment dans le groupe constitué par l'hydrogène, alkyle en (C₁-C₄), halogène, amino, alkylamino en (C₁-C₄), alcanamido en (C₁-C₄), alcanesulfonamido en (C₁-C₄), benzènesulfonamido, naphtalènesulfonamido, p-toluènesulfonamido, aminoalkyle en (C₁-C₄), alkylamino en (C₁-C₂)-alkyle en (C₁-C₄), alcanamido en (C₁-C₄)-alkyle en (C₁-C₄), alcanesulfonamido en (C₁-C₄)-alkyle en (C₁-C₄), benzènesulfonamido-alkyle en (C₁-C₄), naphtalènesulfonamido-alkyle en (C₁-C₄) et un p-toluènesulfonamido-alkyle en (C₁-C₄),
avec des quantités appropriées d'excipients ou de véhicules pharmaceutiquement acceptables.

7. Un composé de formule (I) 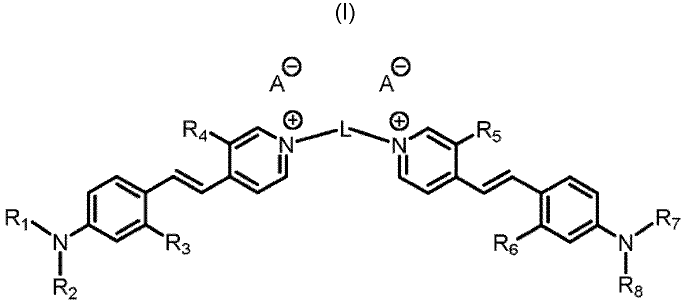 ou un sel pharmaceutiquement acceptable de celui-ci, dans lequel
A est un anion ;
L est un diradical choisi dans le groupe constitué par l'éthylène, le triméthylène, le tétraméthylène, le pentaméthylène, l'hexaméthylène, l'heptaméthylène, l'octaméthylène, le nonaméthylène, le décaméthylène, l'undécaméthylène, le dodécaméthylène, le 3-oxapentaméthylène, le 3,6-dioxaoctaméthylène, le 3,6,9-trioxaundécaméthylène, le 1,2-phénylènebis(méthylène), le 1,3-phénylènebis(méthylène) et le 1,4-phénylènebis(méthylène) ;
R₁, R₂, R₇ et R₈ sont des radicaux choisis indépendamment dans le groupe constitué par l'hydrogène, alkyle en (C₁-C₄), hydroxyalkyle en (C₁-C₄), cyanoalkyle en (C₁-C₄), alcanoyloxyalkyle en (C₁-C₄), phényle, alcanoyle en (C₁-C₄), alcanesulfonyle en (C₁-C₄), benzènesulfonyle, naphtalènesulfonyle et p-toluènesulfonyle ou, en variante, lorsqu'ils sont pris en combinaison, R₁-N-R₂ et R₇-N-R₈ sont choisis indépendamment dans le groupe constitué par les cycles azétidine, pyrrolidine, pipéridine, azépine, pipérazine, 4-alkylpipérazine en (C₁-C₂) et morpholine ;
R₃ et R₆ sont des radicaux choisis indépendamment dans le groupe constitué par l'hydrogène, alkyle en (C₁-C₄), nitro, amino, alkylamino en (C₁-C₄), alcanamido en (C₁-C₄), alcanesulfonamido en (C₁-C₄), benzène-sulfonamido, naphtalènesulfonamido et p-toluènesulfonamido ; et
R₄ et R₅ sont des radicaux choisis indépendamment dans le groupe constitué par l'hydrogène, un alkyle en (C₁-C₄), halogène, amino, alkylamino en (C₁-C₄), alcanamido en (C₁-C₄), alcanesulfonamido en (C₁-C₄), benzènesulfonamido, naphtalènesulfonamido, p-toluènesulfonamido, aminoalkyle en (C₁-C₄), alkylamino en (C₁-C₂)-alkyle en (C₁-C₄), alcanamido en (C₁-C₄)-alkyle en (C₁-C₄), alcanesulfonamido en (C₁-C₄)-alkyle en (C₁-C₄), benzènesulfonamido-alkyle en (C₁-C₄), naphtalènesulfonamido-alkyle en (C₁-C₄) et p-toluènesulfonamido-alkyle en (C₁-C₄) ;
à condition que lorsque R₁, R₂, R₃, R₄, R₅, R₆, R₇ et R₈ sont l'hydrogène, L n'est pas le 1,4-phénylènebis(méthylène) ;
à condition que lorsque R₁, R₂, R₇ et R₈ sont le méthyle et R₃, R₄, R₅ et R₆ sont l'hydrogène, L n'est pas l'éthylène, triméthylène, tétraméthylène, pentaméthylène, hexaméthylène, décaméthylène, dodécaméthylène, 3,6-dioxaoctaméthylène, 1,2-phénylènebis(méthylène), 1,3-phénylènebis(méthylène) ou le 1,4-phénylènebis(méthylène) ;
à condition que lorsque R₁, R₂, R₇ et R₈ sont éthyle et R₃, R₄, R₅ et R₆ sont l'hydrogène, Lis n'est pas le triméthylène, le tétraméthylène, le 3-oxapentaméthylène, le 1,2-phénylènebis(méthylène) ou le 1,4-phénylènebis(méthylène) ;
à condition que lorsque R₁, R₂, R₇ et R₈ sont le phényle et R₃, R₄, R₅ et R₆ sont l'hydrogène, L n'est pas le triméthylène, le tétraméthylène, le pentaméthylène, l'hexaméthylène, l'octaméthylène, le décaméthylène, le dodécaméthylène, le 1,2-phénylènebis(méthylène), le 1,3-phénylènebis(méthylène) ou le 1,4-phénylènebis(méthylène) ;
à condition que lorsque R₁, R₂, R₇ et R₈ sont HO-CH₂-CH₂- et R₃, R₄, R₅ et R₆ sont l'hydrogène, L n'est pas le tétraméthylène ;
à condition que lorsque R₁ et R₇ sont l'éthyle, R₂ et R₈ sont HO-CH₂-CH₂- et R₃, R₄, R₅ et R₆ sont l'hydrogène, L n'est pas l'éthylène, le triméthylène, le tétraméthylène, le pentaméthylène, l'hexaméthylène ou l'heptaméthylène ;
à condition que lorsque R₁ et R₇ sont le méthyle, R₂ et R₈ sont NC-CH₂- et R₃, R₄, R₅ et R₆ sont l'hydrogène, L n'est pas le 1,2-phénylènebis(méthylène) ou le 1,4-phénylènebis(méthylène),
à condition que lorsque R₁ et R₇ sont l'acétyle, R₂, R₃, R₄, R₅, R₆ et R₈ sont l'hydrogène, L n'est pas le 1,4-phénylènebis(méthylène) ;
à condition que lorsque R₁, R₂, R₃, R₆, R₇ et R₈ sont le méthyle et R₄ et R₅ sont l'hydrogène, L n'est pas le triméthylène ou le 1,4-phénylènebis(méthylène) ;
à condition que lorsque R₁, R₂, R₃, R₆, R₇ et R₈ sont le méthyle et R₃ et R₆ sont l'hydrogène, L n'est pas le triméthylène, le tétraméthylène ou le décaméthylène ;
à condition que lorsque R₁, R₂, R₇ et R₈ sont l'éthyle, R₃ et R₆ sont l'hydrogène et R₄ et R₅ sont le méthyle, L n'est pas le décaméthylène ;
à condition que lorsque R₁-N-R₂ et R₇-N-R₈ sont le pyrrolidino et R₃, R₄, R₅ et R₆ sont l'hydrogène, L n'est pas le triméthylène ou le 1,4-phénylènebis(méthylène) ;
à condition que lorsque R₁-N-R₂ et R₇-N-R₈ sont le pipéridino et R₃, R₄, R₅ et R₆ sont l'hydrogène, L n'est pas le triméthylène, le 1,2-phénylènebis(méthylène), le 1,3-phénylènebis(méthylène) ou le 1,4-phénylènebis(méthylène) ; et
à condition que lorsque R₁-N-R₂ et R₇-N-R₈ sont le 4-méthylpipérazino et R₃, R₄, R₅ et R₆ sont de l'hydrogène, L n'est pas le triméthylène.

8. Le composé selon la revendication 7, dans lequel = R₇, R₂ = R₈, R₃ = R₆ et R₄ = R₅ ou, en variante, lorsqu'ils sont utilisés en combinaison, R₁-N-R₂ = R₇-N-R₈, R₃ = R₆ et R₄ = R₅.

9. Le composé selon la revendication 8, dans lequel :
R₁, R₇ et R₂, R₈ sont des radicaux choisis indépendamment dans le groupe constitué par l'hydrogène, le méthyle, l'éthyle, le phényle, le 2-hydroxyéthyle, le 2-cyanoéthyle et le 2-(acétyloxy)éthyle, ou, en variante, lorsqu'ils sont pris en combinaison, R₄-N-R₂ = R₇-N-R₈ est choisi dans le groupe constitué par le cycle pyrrolidine, pipéridine, pipérazine et morpholine ;
R₃, R₆ sont des radicaux choisis dans le groupe constitué par l'hydrogène, le méthyle, le nitro, amino, acétamido, méthanesulfonamido, benzènesulfonamido et p-toluènesulfonamido ; et
R₄, R₅ sont des radicaux choisis dans le groupe constitué par l'hydrogène, le méthyle, le chloro, le bromo, le fluoro, le iodo, l'amino, l'acétamido, le méthanesulfonamido, le benzènesulfonamido, le p-toluènesulfonamido, l'aminométhyle, l'acétamidométhyle, le méthanesulfonamidométhyle, le benzènesulfonamidométhyle et le p-toluènesulfonamidométhyle.

10. Le composé selon la revendication 9, dans lequel le composé est choisi dans le groupe constitué par :
1,1'-(décane-1,10-diyl)bis{3-méthyl-4-[(E)-4-(pyrrolidin-1-yl)styryl]pyridin-1-ium}dibromure ;
1,1'-(décane-1,10-diyl)bis{3-méthyl-4-[(E)-4-(pipéridin-1-yl)styryl]pyridin-1-ium}dibromure ;
1,1'-(décane-1, 10-diyl)bis{3-méthyl-4-[(E)-4-morpholinostyryl]pyridin-1-ium}dibromure ;
1,1'-(décane-1,10-diyl)bis{3-méthyl-4-[(E)-4-(pipérazin-1-yl)styryl]pyridin-1-ium}dibromure ;
1,1'-(décane-1,10-diyl)bis{4-[(E)-4-(diphénylamino)styryl]-3-méthylpyridin-1-ium}dibromure ;
1,1'-(décane-1,10-diyl)bis{4-[(E)-4-(diméthylamino)-2-nitrostyryl]-3-méthylpyridin-1-ium}dibromure ;
1,1'-(décane-1,10-diyl)bis{4-{(E)-4-[(2-cyanoéthyl)méthylamino]styryl}-3-méthylpyridin-1-ium}dibromure ;
1,1'-(décane-1,10-diyl)bis{4-{(E)-4-[bis(2-acétoxyéthyl)amino]styryl}-3-méthylpyridin-1-ium}dibromure ;
1,1'-(décane-1,10-diyl)bis{4-{(E)-4-[bis(2-hydroxyéthyl)amino]styryl}-3-méthylpyridin-1-ium}dibromure ;
1,1'-(éthane-1,2-diyl)bis{4-[(E)-4-(diéthylamino)styryl]-3-méthylpyridin-1-ium}dibromure ;
1,1'-(propane-1,3-diyl)bis{4-[(E)-4-(diéthylamino)styryl]-3-méthylpyridin-1-ium}dibromure ;
1,1'-(butane-1,4-diyl)bis{4-[(E)-4-(diéthylamino)styryl]-3-méthylpyridin-1-ium}dibromure ;
1,1'-(pentane-1,5-diyl)bis{4-[(E)-4-(diéthylamino)styryl]-3-méthylpyridin-1-ium}dibromure ;
1,1'-(hexane-1,6-diyl)bis{4-[(E)-4-(diéthylamino)styryl]-3-méthylpyridin-1-ium}dibromure ;
1,1'-(heptane-1,7-diyl)bis{4-[(E)-4-(diéthylamino)styryl]-3-méthylpyridin-1-ium}dibromure ;
1,1'-(octane-1,8-diyl)bis{4-[(E)-4-(diéthylamino)styryl]-3-méthylpyridin-1-ium}dibromure ;
1,1'-(nonane-1,9-diyl)bis{4-[(E)-4-(diéthylamino)styryl]-3-méthylpyridin-1-ium}dibromure ;
1,1'-(undécane-1,11-diyl)bis{4-[(E)-4-(diéthylamino)styryl]-3-méthylpyridin-1-ium}dibromure ;
1,1'-(dodécane-1,12-diyl)bis{4-[(E)-4-(diéthylamino)styryl]-3-méthylpyridin-1-ium}dibromure ;
1,1'-(3-oxapentane-1,5-diyl)bis{4-[(E)-4-(diéthylamino)styryl]-3-méthylpyridin-1-ium}dichlorure ;
1,1'-(3,6-dioxaoctane-1,8-diyl)bis{4-[(E)-4-(diéthylamino)styryl]-3-méthylpyridin-1-ium}diiodure ;
1,1'-(3,6,9-trioxaundécane-1,11-diyl)bis{4-[(E)-4-(diéthylamino)styryl]-3-méthylpyridin-1-ium}dichlorure ;
1,1'-[1,2-phénylènebis(méthylène)]bis{4-[(E)-4-(diéthylamino)styryl]-3-méthylpyridin-1-ium}dibromide ;
1,1'-[1,3-phénylènebis(méthylène)]bis{4-[(E)-4-(diéthylamino)styryl]-3-méthylpyridin-1-ium}dibromure ;
1,1'-[1,4-phénylènebis(méthylène)]bis{4-[(E)-4-(diéthylamino)styryl]-3-méthylpyridin-1-ium}dibromure ;
1,1'-(décane-1, 10-diyl)bis{4-[(E)-4-(diéthylamino)styryl]pyridin-1-ium}dibromure ;
1,1'-(décane-1,10-diyl)bis{3-bromo-4-[(E)-4-(diéthylamino)styryl]pyridin-1-ium}dibromure ;
1,1'-(décane-1,10-diyl)bis{3-chloro-4-[(E)-4-(diéthylamino)styryl]pyridin-1-ium}dibromure ;
et
1,1'-(décane-1,10-diyl)bis{3-amino-4-[(E)-4-(diéthylamino)styryl]pyridin-1-ium}dibromure.
